# EUROPÄISCHE PATENTSCHRIFT

(11) **EP 1 513 942 B1**
(45) Veröffentlichungstag und Bekanntmachung des Hinweises auf die Patenterteilung: **13.09.2006**
(21) Anmeldenummer: 03738005.2
(22) Anmeldetag: 06.06.2003
(51) Int. Cl.: C12P 19/14, C12P 19/12, C07H 3/06, A23L 1/09, A61K 31/702

(54) **GALACTOSYL-ISOMALT, VERFAHREN ZU SEINER HERSTELLUNG UND VERWENDUNG**
GALACTOSYL ISOMALT, METHOD FOR PRODUCTION AND USE THEREOF
GALACTOSYL-ISOMALTE, SON PROCEDE DE PREPARATION ET SON UTILISATION

(30) Priorität: 07.06.2002 DE 10225242
(43) Veröffentlichungstag der Anmeldung: 16.03.2005
(73) Patentinhaber: Südzucker Aktiengesellschaft Mannheim/Ochsenfurt, 68165 Mannheim (DE)
(72) Erfinder: HAJI BEGLI, Alireza, 67305 Ramsen (DE); KLINGEBERG, Michael, 67269 Grünstadt (DE); KUNZ, Markwart, 67550 Worms (DE); MATTES, Ralf, 70619 Stuttgart (DE); SCHRÖDER, Sven, 20259 Hamburg (DE); THIEM, Joachim, 22391 Hamburg (DE); VOGEL, Manfred, 67271 Neuleiningen (DE)
(74) Vertreter: Schrell, Andreas
(86) Internationale Anmeldenummer: PCT/EP2003/005999
(87) Internationale Veröffentlichungsnummer: WO 2003/104473

(56) Entgegenhaltungen:
- EP-A- 0 859 006
- US-A- 5 578 339
- GU Q-M: "ENZYME-MEDIATED REACTIONS OF OLIGOSACCHARIDES AND POLYSACCHARIDES" JOURNAL OF ENVIRONMENTAL POLYMER DEGRADATION, XX, XX, Bd. 7, Nr. 1, 1999, Seiten 1-7, XP000992950 ISSN: 1064-7546
- MAHONEY R R: "GALACTOSYL-OLIGOSACCHARIDE FORMATION DURING LACTOSE HYDROLYSIS: A REVIEW" FOOD CHEMISTRY, ELSEVIER SCIENCE PUBLISHERS LTD, GB, Bd. 63, Nr. 2, Oktober 1998 (1998-10), Seiten 147-154, XP001156621 ISSN: 0308-8146
- FARKAS E ET AL: "REGIOSELECTIVE SYNTHESIS OF GALACTOSYLATED TRI- AND TETRASACCHARIDES BY USE OF BETA-GALACTOSIDASE FROM BACILLUS CIRCULANS" SYNTHESIS, GEORG THIEME VERLAG. STUTTGART, DE, Nr. 5, 2003, Seiten 699-706, XP001156627 ISSN: 0039-7881

## Beschreibung

Die vorliegende Erfindung betrifft β-galactosylierte Saccharide oder Saccharidalkohole, insbesondere Zusammensetzungen oder Gemische enthaltend Galactosyl-Isomalt und Zusammensetzungen und Gemische enthaltend Galactosyl-Isomaltulose, Verfahren zu deren Herstellung und die erhaltenen Produkte und Zwischenprodukte sowie deren Verwendung in Nahrungs-, Lebens-, Genuss- und Tierfuttermitteln sowie für oder als Arzneimittel.

Nahrungs-, Genuss- und Futtermittel dienen in erster Linie der Ernährung und dem Wohlbefinden des menschlichen und tierischen Konsumenten. Neben diesen beiden Aspekten wird von Nahrungs- und Genussmitteln zunehmend auch eine gesundheitsfördernde Funktion erwartet. Lebens- und Genussmittel sollen einerseits die Gesundheit erhalten und fördern, andererseits schädliche Einflüsse abwehren und gegebenenfalls prophylaktisch gegen Krankheiten wirken. Bestimmungsgemäß entfalten derartige gesundheitsfördernde Nahrungs- und Genussmittel ihre Wirkung vorwiegend im Verdauungstrakt. Im vorderen Verdauungstrakt werden die aufgenommenen Nährstoffe aufgeschlossen und teilweise resorbiert. Nicht verdauliche Kohlenhydrate gelangen in den Dickdarm und stehen dort der mikrobiellen Darmflora zur Verfügung.

Kurzkettige Fettsäuren wie Buttersäure (Butyrat), werden fermentativ von saccharolytischen Bakterien des Dickdarms insbesondere aus nicht verdauten Kohlenhydraten gebildet. Buttersäure stellt im Kolon die dominierende Energiequelle für die Epithelzellen dar, beeinflusst die zelluläre Proliferation und Differenzierung und spielt eine zentrale Rolle als Wachstumsfaktor für ein gesundes Darmepithel und bei der Aufrechterhaltung der mukosalen Barriere im Kolon. Kurzkettige Fettsäuren wie Buttersäure und deren Salze (Butyrat) tragen zur Entgiftung möglicher mutagener Stoffwechselprodukte im Dickdarm bei und wirken dem oxidativen Stress entgegen, beispielsweise über die Induktion der Genexpression protektiver Proteine wie der intestinalen Glutathion-S-Transferase oder der Hemmung der Omithindecarboxylase. Glutathion (GSH) ist ein Cystein-haltiges Tripeptid und die häufigste Thiol-Verbindung in Säugerzellen. GSH ist ein Substrat für die Enzyme Glutathion-S-Transferase und die GSH-Peroxidase, die die Entgiftung xenobiotischer Verbindungen und Reaktionen zur Hemmung reaktiver Sauerstoff-Moleküle und anderer freier Radikale katalysieren. Als Substrat der Glutathion-S-transferase (GST) geht GSH durch reversible Oxidation in das entsprechende Disulfid GSSG über. Glutathion wirkt als Antioxidans und stellt dadurch insbesondere ein Puffersystem für den Redox-Zustand der Zelle dar. Die GSTs bilden eines der wichtigsten Entgiftungssysteme der Zellen, insbesondere auch während der Phase II der Zellteilung. Die Entgiftung erfolgt durch Übertragung von Glutathion auf elektrophile Komponenten, die beispielsweise während der Verstoffwechselung von Kanzerogenen entstehen. Durch den GST-katalysierten nucleophilen Angriff von Glutathion auf elektrophile Substrate wird deren Reaktivität bezüglich zellulärer Makromoleküle stark vermindert. GSTs können so die Wirksamkeit einer Reihe chemischer Kanzerogene stark vermindern. GSTs spielen daher eine wichtige physiologische Rolle beim Schutz vor oxidativem Stress und vor den damit einhergehenden Erkrankungen, insbesondere vor Krebserkrankungen. Verbindungen wie polycyclische aromatische Kohlenwasserstoffe, Phenol-Antioxidanzien, reaktive Sauerstoff-Moleküle, Isothiocyanate, trivalente Arsenverbindungen, Barbiturate und synthetische Glucocorticoide können die GST-Aktivitäten induzieren, wobei die GST-Enzyme codierenden Gene aktiviert werden (Hayes und Pulford, 1995). Die GST-Induktion erfolgt hauptsächlich über verschiedene Transkriptionsmechanismen. Die Regulationsbereiche GST-codierender Gene enthalten Elemente, woran die vorgenannten Stoffe binden und die Gentranskription induzieren können. Auch Nahrungsbestandteile, beispielsweise phytochemische Substanzen, können GST-Aktivitäten induzieren, wobei insbesondere GST-Formen der π-Klasse im Darmbereich induziert werden. Die GST-Induktion im Intestinaltrakt durch Nahrungsbestandteile wird daher als Mechanismus zur Verhütung von Darmkrebserkrankungen angesehen (Peters und Roelofs, Cancer Res., 52 (1992), 1886-1890). Von besonderer Bedeutung für die GST-Induktion sind schwer oder nicht verdauliche Nahrungsmittelbestandteile, das heißt Nahrungsfasern oder Ballaststoffe, die gegen die Verdauung durch menschliche Enzyme resistent sind, aber im Dickdarm fermentiert werden. Dazu gehören einige Kohlenhydrate, wie Pektin, "Guar Gum" (Guarkernmehl) und resistente Stärke, die erst im Intestinaltrakt durch die Bakterienflora des Dickdarms zu kurzkettigen Fettsäuren, insbesondere Essigsäure, Propionsäure und Buttersäure, fermentiert werden (Bartram et al., Cancer Res., 53 (1993), 3283-3288).

Des Weiteren wirken kurzkettige Fettsäuren wie Buttersäure kontrollierend auf die Induktion spezifischer Gene und die Modifizierung von Proteinen der Zellzyklusregulation, antibakterieller Peptide und Signalkaskaden. Hohe Buttersäurekonzentrationen im Dickdarm, insbesondere in hinteren Dickdarmbereichen unterstützen ein gesundes Darmmilieu und ein gesundes Darmepithel, verbessern Symptome von ulzerativen Entzündungen des Kolons und sind protektiv in der Colonkarzinogenese, das heißt sie gelten als das Dickdarmkrebsrisiko reduzierende Substanzen.

Es ist daher wünschenswert, die auf die menschliche oder tierische Gesundheit positiv wirkende Darmflora zu fördern und darüber hinaus eine Produktion von großen Mengen an Buttersäure, insbesondere auch in hinteren Dickdarmabschnitten zu erreichen. Dies kann durch die Zufuhr geeigneter Substrate, zur Verbesserung der Lebensbedingungen für die gesundheitsförderliche Darmflora und von Substraten für die mikrobielle Bildung von Buttersäure, insbesondere auch in hinteren Dickdarmbereichen, erreicht werden. Substanzen oder Substanzgemische, die als Bestandteile von Nahrungs- oder Genussmitteln selektiv das Wachstum und/oder die Aktivität spezifischer gesundheitsfördernder Darmbakterien fördern, insbesondere Bifidobakterien und Lactobacillen, werden als Präbiotika bezeichnet. Präbiotika fördern das Wachstum und/oder die Aktivität gesundheitsfördernder Darmbakterien und sind in der Regel durch Enzyme des Gastrointestinaltraktes nicht verdaubare Kohlenhydrate.

Der tatsächliche Anteil von verdauungsresistenten und fermentierbaren Nahrungsfasern oder Ballaststoffen in der Nahrung hängt von vielen Faktoren ab, beispielsweise der Art des Lebensmittels und seiner Zubereitungsart. Die meisten Nahrungs-, Futter oder Genussmittel sind arm an Ballaststoffen. Gemüse, bestimmte Obstsorten, Nüsse, Samen und vor allem unraffinierte Getreideprodukte sind hingegen reich an Ballaststoffen. Ein Weg, die durch die Lebensmittel-Verarbeitung entstehenden Ballaststoff-Defizite beziehungsweise eine ballaststoffarme Ernährung auszugleichen und insbesondere Krebserkrankungen und Infektionskrankheiten über die Nahrungszufuhr vorzubeugen, besteht in der Anreicherung von Lebensmitteln mit idealer Weise unverdaulichen aber gut fermentierbaren Ballaststoffen. Viele der bislang zur Anreicherung von Lebensmitteln verwendeten Ballaststoffe weisen allerdings eine Reihe entscheidender Nachteile auf und erfüllen nicht die in sie gesetzten Erwartungen bezüglich der Verhinderung und/oder Therapie von Krebserkrankungen, insbesondere des Dickdarms, und von Infektionskrankheiten. In Langzeit-Studien unter anderen in denen des U.S. National Cancer Institute und der University of Arizona wurde festgestellt, dass eine mehrjährige Ernährung mit ballaststoffreicher Kost, beispielsweise mit Müsli-Produkten, offensichtlich keinen Einfluss auf die Häufigkeit von Dickdarmkrebs hatte. Bei diesen Untersuchungen wurden jedoch nur solche Ballaststoffe einbezogen, die nicht im Dickdarmbereich fermentiert werden können.

Nicht alle als Präbiotika bekannte Saccharide dienen der Lieferung von nützlichen Fermentierungsprodukten der darmbesiedelnden Mikroflora, insbesondere kurzkettige Fettsäuren wie der vorteilhaften Buttersäure, bevorzugt als Butyrat, in hintere Darmabschnitte. Bekannte Präbiotika wie Fructooligosaccharide, die den Dickdarm erreichen, werden dort recht schnell und vollständig fermentiert. Die hierbei gebildeten kurzkettigen Fettsäuren werden schnell und nahezu vollständig von den intestinalen Epithelzellen am Ort ihrer Entstehung resorbiert. Für eine Bereitstellung dieser Fermentierungsprodukte in hinteren Darmabschnitten ist es jedoch notwendig, dass Saccharide langsamer fermentiert werden, damit genug Substrat auch in hintere Darmbereiche gelangt und zur mikrobiellen Fermentation zur Verfügung steht. Die sehr schnelle Fermentation bekannter Präbiotika beinhaltet nachteiligerweise auch ein höheres Risiko für laxative Effekte und andere gastrointestinale Unpässlichkeiten. Weiterhin zeichnen sich bekannte Präbiotika wie Inulin und Oligofructose nachteilig auch dadurch aus, dass bei ihrem Abbau durch die intestinale Mikroflora überwiegend andere kurzkettige Fettsäuren, insbesondere Essigsäure, gebildet werden, und dass sie die vorteilhafte Buttersäure nur in sehr geringem Umfang liefern und damit nur gering butyrogen sind und somit keine Substrate für die Buttersäurebildung in hinteren Dickdarmbereichen darstellen.

Bekannte Präbiotika wie Fructooligosaccharide zeichnen sich nachteilig auch dadurch aus, dass ihre technologische Verarbeitbarkeit bei der Lebensmittelherstellung zum Teil zu wünschen übrig lässt. Ihre mangelnde Wasserlöslichkeit zum Beispiel bei längerkettigen Kohlenhydraten wie resistenter Stärke, ihre geringe Säurestabilität und ihre Reaktivität als teilweise reduzierende Oligosaccharide tragen zu ihrer beschränkten Anwendbarkeit bei. Dies gilt insbesondere bei der Anwendung in solchen Produkten, die einen niedrigen pH-Wert aufweisen.

Häufig wird beispielsweise auch Weizenkleie als Zusatz zu ballaststoffarmer Kost eingesetzt. Wie Untersuchungen an Ratten bezüglich der Tumorinzidenz im Colon zeigten, sind jedoch Weizenkleie-Anwendungen kaum zur Krebsprävention geeignet. Weizenkleie wird, ähnlich wie Cellulose, kaum von der Dickdarmflora fermentiert. Vielmehr besitzen Weizenkleie und auch andere Getreidefasern meist einen hohen Anteil des Kleberproteins Gluten und dessen toxischen Bestandteilen, die zu schweren Veränderungen der Dünndarmschleimhaut führen. Die Schädigung des Resorptionsepithels führt zu einem Verlust an Verdauungsenzymen und zu schwersten morphologischen sowie funktionellen Störungen (Malabsorption mit gestörter Resorption aller Nährstoffe einschließlich Mineralien, Vitaminen etc., Zöliakie).

Demgegenüber erfüllen β-galactosylierte Oligosaccharide in hohem Maße die vorgenannten, an einen idealen Ballaststoff gestellten Forderungen und vermitteln daher positiven Effekte auf die Verdauungsorgane und auf den Zustand des Immunsystems. Sie hätten daher ein breites Anwendungsspektrum in der Nahrungsmittelindustrie, insbesondere im Bereich der Gesundheitskost und der Diätkost. Die tatsächliche Anwendung hängt jedoch stark von der Verfügbarkeit größerer Mengen dieser Galactose-haltigen Oligosaccharide ab. Die Synthese β-galactosylierter Oligosaccharide mittels bekannter chemischer Methoden wurde innerhalb des letzten Jahrzehnts entwickelt. Allerdings handelt es sich bei den bekannten β-Galactosylierungsverfahren um mehrstufige Ansätze, die beispielsweise das Einführen von Schutzgruppen und deren Abspaltung in einem späteren Verfahrensschritt erfordern. Diese Verfahren sind daher nur wenig für die Herstellung β-galactosylierter Oligosaccharide in großem industriellen Maßstab geeignet. Um diese Schwierigkeiten zu überwinden, wurden Synthesestrategien entwickelt, worin biologische Enzyme als Katalysatoren wirken. Allerdings sind beispielsweise bekannte alternative Ansätze, welche die Verwendung von Glykosyltransferasen vorsehen, ebenfalls nicht in großem Maßstab durchführbar, da zur Zeit die Verfügbarkeit dieser Transferasen und ihrer Co-Faktoren stark begrenzt ist. Außerdem sind auch die für diese Ansätze notwendigen Nucleosid-Zucker als Galactosyl-Donorstrukturen ebenfalls nicht in größeren Mengen verfügbar.

Der vorliegenden Erfindung liegt das technische Problem zugrunde, Mittel, die zur Vorbeugung von Erkrankungen, insbesondere Dickdarmkrebs, geeignet sind und nicht die Nachteile der im Stand der Technik bekannten Ballaststoffe aufweisen, sowie verbesserte und wirtschaftlichere Verfahren für die Herstellung dieser Mittel bereitzustellen.

Die vorliegende Aufgabe wird gelöst durch die Bereitstellung eines Verfahrens zur Herstellung einer Zusammensetzung, die galactosylierte hydrierte Isomaltulose und/oder galactosylierte nicht-hydrierte Isomaltulose enthält, insbesondere daraus besteht, wobei
a) mindestens ein Galactosyl-Donor gelöst in einer wässrigen Lösung mit
b) mindestens einer β-Galactosidase und mit
c) einer wässrigen Lösung eines Gemisches, das hydrierte Isomaltulose, insbesondere Isomalt, und/oder nicht-hydrierte Isomaltulose enthält, insbesondere daraus besteht,
in Kontakt gebracht wird und
ein Gemisch enthaltend galactosylierte hydrierte Isomaltulose, insbesondere enthaltend Galactosyl-Isomalt, im Folgenden Galactosyl-Isomalt-Zusammensetzung (A) und/oder enthaltend galactosylierte nicht-hydrierte Isomaltulose, das heißt Galactosyl-Isomaltulose-Zusammensetzung erhalten wird.

Die Erfinder fanden überraschend, dass Glykosid-Hydrolasen, das heißt Glykosidasen wie die β-Galactosidase, welche transglykosilierende Eigenschaften aufweisen, als stereospezifische Katalysatoren auch für die Glykosylierung von Sacchariden und Saccharidalkoholen, insbesondere von Disacchariden und Disaccharidalkoholen, vorteilhaft eingesetzt werden können. Die bekannte, herkömmliche Funktion dieser Glykosidasen besteht in der hydrolytischen Spaltung von glykosidischen Bindungen. Durch die Derivatisierung der eingesetzten Substrate mit höheren Abgangsgruppen und der damit verbundenen veränderten Kinetik der Enzym/Substrat-Bindung kommt es überraschenderweise im Rahmen einer Transglykosilierung zu einer Umkehrung der enzymatischen Reaktion, das heißt zur Bildung glykosidischer Bindungen. Besonders vorteilhaft dabei ist, dass diese Glykosidasen in größerer Menge verfügbar sind und außerdem die Verwendung weniger komplexer Glykosyl-Donorsubstrate wie Di- oder Trisaccharide erlauben.

Die vorliegende Erfindung stellt daher im wesentlichen Verfahren zur Herstellung eines Galactosyl-Saccharids, insbesondere eines Galactosyl-Trisaccharids, beziehungsweise Galactosyl-Saccharidalkohols, insbesondere eines Galactosyl-Trisaccharidalkohols, bereit, welches einen Galactosylrest in β-Stellung enthält. In einem ersten Schritt wird mindestens ein Galactosyl-Donor mit einem Galactosyl-Akzeptor, welcher mindestens ein Saccharid und/oder Saccharidalkohol, insbesondere ein Disaccharid und/oder Disaccharidalkohol, oder ein mindestens eine dieser Verbindungen enthaltendes Gemisch ist, vermischt und unter Einwirkung von transglykosylierendem Enzym, nämlich mindestens einer β-Galactosidase, über eine bestimmte Reaktionszeit und vorzugsweise in wässriger Lösung so in Kontakt und Reaktion gebracht, dass Galactosylreste von dem Galactosyl-Donor auf den Galactosyl-Akzeptor übertragen werden. Als Reaktionsprodukt wird mindestens ein Galactosyl-Saccharid, mindestens ein Galactosyl-Saccharidalkohol und/oder ein Gemisch enthaltend mindestens eine dieser Verbindungen gebildet. Das erhaltene Produkt oder Produktgemisch wird erfindungsgemäß bevorzugt mittels üblicher Trennverfahren weiter nach Bedarf aufgereinigt und/oder bevorzugt einer katalytischen Hydrierung unterworfen.

Die vorliegende Erfindung betrifft in weiteren Varianten als Saccharide beziehungsweise Saccharidalkohole Oligosaccharide wie Di-, Tri-, Tetra- und Pentasaccharide sowie Monosaccharide und Mischungen davon beziehungsweise deren Alkohole und Mischungen davon.

Erfindungsgemäß bevorzugt wird als Edukt a) hydrierte Isomaltulose und/oder b) nicht-hydrierte Isomaltulose eingesetzt und als Produkt ein a) Galactosyl-Isomalt und/oder b) Galactosyl-Isomaltulose enthaltendes Gemisch erhalten.

Im Zusammenhang mit der vorliegenden Erfindung wird unter dem Begriff "Isomaltulose" beziehungsweise "nicht-hydrierte Isomaltulose" einerseits die Verbindung 6-O-α-D-Glucopyranosyl-(1→6)-β-D-fructofuranose, im Folgenden auch als 6-O-α-D-Glucopyranosylfructose bezeichnet, und andererseits ein Gemisch enthaltend im Wesentlichen 6-O-α-D-Glucopyranosyl-fructose verstanden. Bevorzugt wird Isomaltulose durch enzymatische Umsetzung aus Saccharose gewonnen. Erfindungsgemäß ist in einer bevorzugten Ausführungsform vorgesehen, dass dieses Eduktgemisch zusätzlich weitere Substanzen wie Zuckeralkohole oder Zucker, insbesondere Oligosaccharide und/oder Disaccharide, z.B. Trehalulose oder Isomaltose, und/oder Monosaccharide, z.B. Fructose, enthält. Bevorzugt sind Eduktgemische, die aus 6-O-α-D-Glucopyranosylfructose bestehen oder diese im Wesentlichen enthalten, insbesondere zu mehr als 70, 80, 85, 90, 95 oder 97 Gew.-%.

Die vorliegende Erfindung sieht also bevorzugt vor, dass unter Einwirkung einer β-Galactosidase aus 6-O-α-D-Glucopyranosyl-D-fructose oder aus einem Gemisch enthaltend 6-O-α-D-Glucopyranosyl-D-fructose das Reaktionsprodukt β-D-Galactopyranosyl-(1→3)-α-D-glucopyranosyl-(1→6)-D-fructose, β-D-Galactopyranosyl-(1→4)-α-D-glucopyranosyl-(1→6)-D-fructose und/oder β-D-Galactopyranosyl-(1→6)-α-D-glucopyranosyl-(1→6)-D-fructose, im Folgenden β-1,3-Galactosyl-Isomaltulose, β-1,4-Galactosyl-Isomaltulose beziehungsweise β-1,6-Galactosyl-Isomaltulose bezeichnet, oder ein Gemisch enthaltend mindestens eine dieser Galactosyl-Isomaltulosen erhalten wird. Je nach Verknüpfung des Galactosylrestes zu 6-O-α-D-Glucopyranosylfructose enthält das Produkt, also die Galactosyl-Isomaltulose, β-1,3-Galactosyl-Isomaltulose, β-1,4-Galactosyl-Isomaltulose und/oder β-1,6-Galactosyl-Isomaltulose; insbesondere liegt Galactosyl-Isomaltulose als Zusammensetzung dieser β-1,3-, β-1,4- und β-1,6-verknüpften Galactosyl-Isomaltulosen vor. Erfindungsgemäß ist in einer bevorzugten Ausführungsform vorgesehen, dass das erhaltene Produkt beziehungsweise Produktgemisch zusätzlich weitere galactosylierte und/oder nicht-galactosylierte Substanzen wie Zuckeralkohole oder Zucker, insbesondere Oligosaccharide, Disaccharide oder Monosaccharide oder Alkohole davon enthält. Bevorzugt sind Produktgemische, die aus β-1,3-, β-1,4- und β-1,6-verknüpften Galactosyl-Isomaltulosen bestehen oder diese im Wesentlichen, insbesondere zu mehr als 70, 80, 85, 90, 95 oder 97 Gew.-%, enthalten.

Bevorzugt stellt die erfindungsgemäß als Edukt eingesetzte hydrierte Isomaltulose Isomalt oder ein Isomalt-haltiges Gemisch dar, welches bevorzugt aus der Hydrierung von Isomaltuluse dargestellt werden kann. Im Zusammenhang mit der vorliegenden Erfindung wird unter dem Begriff "Isomalt" ein Gemisch enthaltend 6-O-α-D-Glucopyranosyl-(1→6)-D-sorbit (6-O-α-D-Glucopyranosyl-(1→6)-D-sorbit), im Folgenden 1,6-GPS, und 1-O-α-D-Glucopyranosyl-(1→1)-D-mannit, insbesondere 1-O-α-D-Glucopyranosyl-(1→1)-D-mannitdihydrat, im Folgenden 1,1-GPM, verstanden, insbesondere ein nahezu äquimolares Gemisch dieser beiden Zuckeralkohole, dabei beträgt der Anteil an 1,6-GPS im nahezu äquimolaren Gemisch von etwa 44 Gew.-% bis etwa 56 Gew.-% und der Anteil an 1,1-GPM entsprechend von etwa 56 Gew. % bis etwa 44 Gew.-%. Die Erfindung erfasst selbstverständlich auch Isomalt-Varianten, das heißt Gemische mit einem von einem nahezu äquimolaren Verhältnis der beiden Zuckeralkohole abweichenden Verhältnis von 1,6-GPS zu 1,1-GPM, zum Beispiel von 1 Gew.-% 1,6-GPS zu 99 Gew.-% 1,1-GPM bis 99 Gew.-% 1,6-GPS zu 1 Gew.-% 1,1-GPM, insbesondere 1,6-GPS-angereicherte Gemische mit einem Verhältnis von 57 Gew.-% 1,6-GPS zu 43 Gew.-% 1,1-GPM bis 99 Gew.-% 1,6-GPS zu 1 Gew.-% 1,1-GPM oder 1,1-GPM-angereicherte Gemische mit einem Verhältnis von 1 Gew.-% 1,6-GPS zu 99 Gew.-% 1,1-GPM bis 43 Gew.-% 1,6-GPS zu 57 Gew.-% 1,1-GPM, wie in der DE 195 32 396 C2 offenbart. Selbstverständlich ist in einer bevorzugten Ausführungsform vorgesehen, dass dieses Eduktgemisch zusätzlich auch weitere Substanzen wie Zuckeralkohole oder Zucker, zum Beispiel 1-O-α-D-Glucopyranosyl-D-sorbit, das heißt 1,1-GPS, Mannit, Sorbit oder/und weitere Mono-, Di- oder Oligosaccharide enthält. Bevorzugt sind dabei Eduktgemische, die aus 1,6-GPS und/oder 1,1-GPM bestehen oder diese im Wesentlichen, insbesondere zu mehr als 70, 80, 85, 90, 95 oder 97 Gew.-%, enthalten.

Erfindungsgemäß werden die dimeren Zuckeralkohole 1,6-GPS und/oder 1,1-GPM zu Trisaccharidalkoholen (DP 3) also zu galactosyliertem 1,6-GPS und/oder galactosyliertem 1,1-GPS oder einem Gemisch enthaltend galactosyliertes 1,6-GPS und/oder galactosyliertes 1,1-GPS, im Folgenden als Galactosyl-Isomalt-Zusammensetzung (A) bezeichnet, umgewandelt. Je nach Verknüpfung des Galactosylrestes zu 1,6-GPS und/oder 1,1-GPM enthält das Produkt, also die Galactosyl-Isomalt-Zusammensetzung, β-D-Galactosyl-(1→3)-α-D-glucosyl-(1→6)-D-sorbit (β-1,3-Galactosyl-1,6-GPS) und/oder β-D-Galactosyl-(1→3)-α-D-glucosyl-(1→1)-D-mannit (β-1,3-Galactosyl-1,1-GPM), β-D-Galactosyl-(1→4)-α-D-glucosyl-(1→6)-D-sorbit (β-1,4-Galactosyl-1,6-GPS) und/oder β-D-Galactosyl-(1→4)-α-D-glucosyl-(1→1)-D-mannit (β-1,4-Galactosyl-1,1-GPM), und/oder β-D-Galactosyl-(1→6)-α-D-glucosyl-(1→6)-D-sorbit (β-1,6-Galactosyl- . 1,6-GPS) und/oder β-D-Galactosyl-(1→6)-α-D-glucosyl-(1→1)-D-mannit (β-1,6-Galactosyl-1,1-GPM). Das Reaktionsprodukt weist als weitere Bestandteile bevorzugt Galactosyl-Lactose, Glucose, Isomalt, Lactose, Galactose und/oder Tetrasaccharide auf.

Bei der erfindungsgemäßen bevorzugten Galactosylierung von hydrierter Isomaltulose, insbesondere Isomalt, enthält die erhaltene Galactosyl-Isomalt-Zusammensetzung:
a) β-D-1,3-Galactosyl-Isomalt, das heißt β-D-Galactosyl-(1→3)-α-D-glucosyl-(1→6)-D-sorbit (β-1,3-Galactosyl-1,6-GPS), und β-D-Galactosyl-(1→3)-α-D-glucosyl-(1→1)-D-mannit (β-1,3-Galactosyl-1,1-GPM) wie Formel (1), 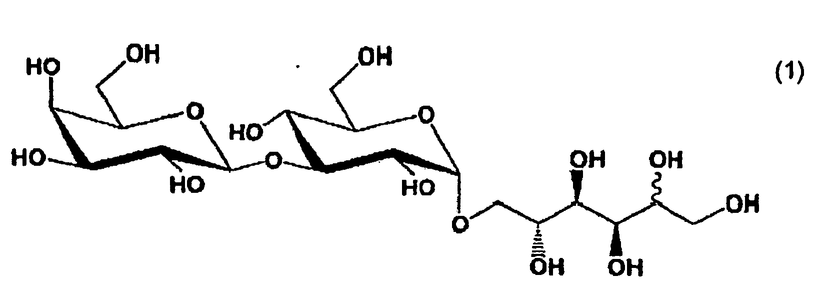
b) β-D-1,4-Galactosyl-lsomalt, das heißt β-D-Galactosyl-(1→4)-α-D-glucosyl-(1→6)-D-sorbit (β-1,4-Galactosyl-1,6-GPS) und β-D-Galactosyl-(1→4)-α-D-glucosyl-(1→1)-D-mannit (β-1,4-Galactosyl-1,1-GPM) wie Formel (2) 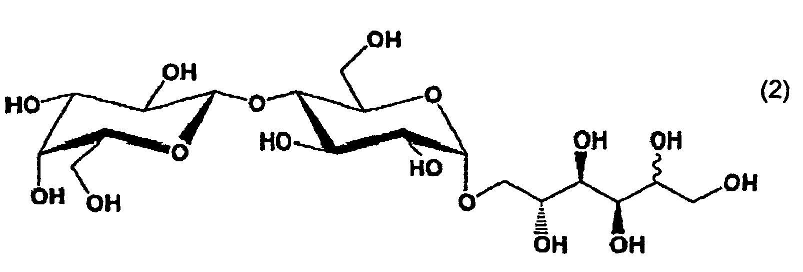 und
c) β-D-1,6-Galactosyl-Isomalt, das heißt β-D-Galactosyl-(1→6)-α-D-glucosyl-(1→6)-D-sorbit (β-1,6-Galactosyl-1,6-GPS,) und β-D-Galactosyl-(1→6)-α-D-glucosyl-(1→1)-D-mannit (β-1,6-Galactosyl-1,1-GPM) wie Formel (3). 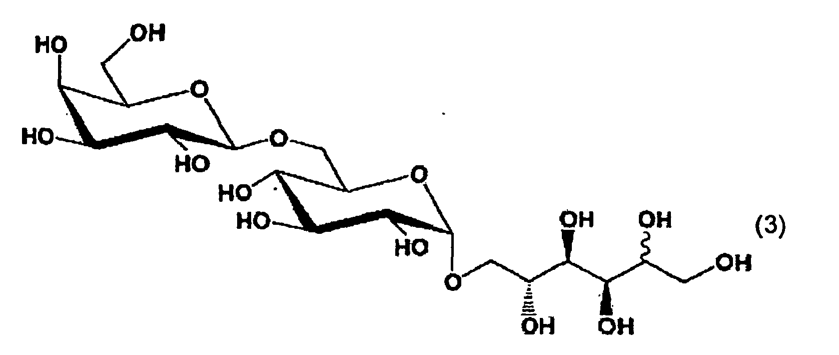 Jede der vorbezeichneten Formeln (1) bis (3) steht jeweils repräsentativ sowohl für das Sorbit- als auch für das Mannit-Epimer beziehungsweise Diastereomer des jeweiligen β-Galactosyl-Isomalt-Isomers, die auch Gegenstand der Erfindung sind. Die Erfindung erfasst also auch jedes einzelne der beiden Diastereomere (-Mannit/-Sorbit) der Galactosyl-Isomalte in isolierter Form sowie jeweils die Gemische der jeweils beiden Diastereomere in isolierter Form sowie das Gemisch aller Sorbit- und Mannit-Epimere der drei Verknüpfungsprodukte.

Bei der erfindungsgemäßen bevorzugten Galactosylierung von nichthydrierter Isomaltulose enthält die erhaltene Galactosyl-Isomaltulose-Zusammensetzung:
a) β-1,3-Galactosyl-Isomaltulose (β-D-Galactopyranosyl-(1→3)-α-D-glucopyranosyl-(1→6)-D-fructose) wie Formel (4), 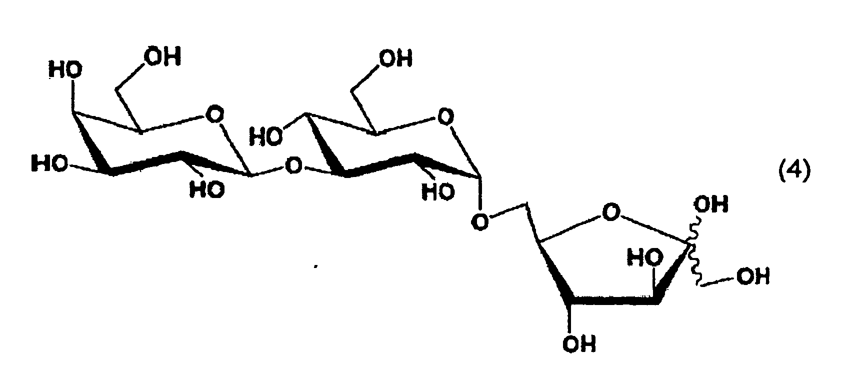
b) β-1,4-Galactosyl-Isomaltulose (β-D-Galactopyranosyl-(1→4)-α-D-glucopyranosyl-(1→6) -D-fructose) wie Formel (5) 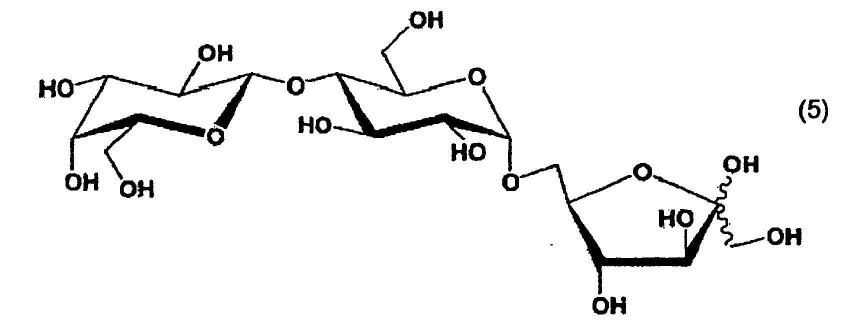 und
c) β-1,6-Galactosyl-Isomaltulose (β-D-Galactopyranosyl-(1→6)-α-D-glucopyranosyl-(1→6) -D-fructose) wie Formel (6).

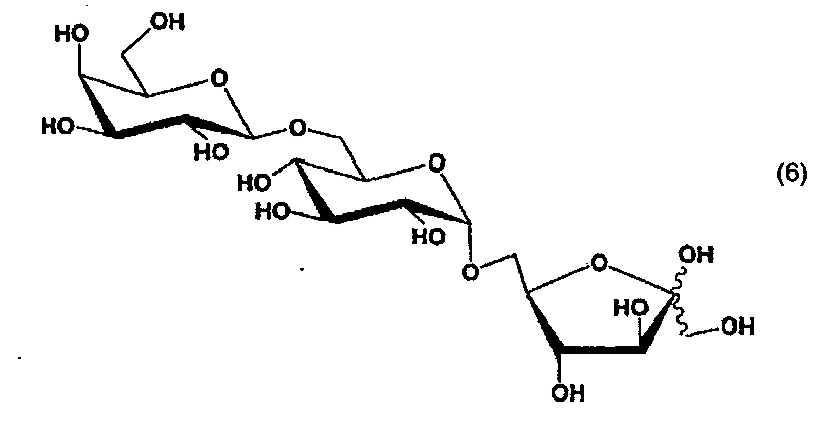

Die Erfindung betrifft also auch Gemische von zwei oder drei der vorstehenden Verbindungen (4), (5) und/oder (6) sowie die Verbindungen in isolierter Form.

In einer bevorzugten Ausführungsform beträgt das Verhältnis von Galactosyl-Donor zu Galactosyl-Akzeptor von 1 Teil Galactosyl-Donor zu 1 Teil Galactosyl-Akzeptor (1:1) bis 1 Teil Galactosyl-Donor zu 5 Teilen Galactosyl-Akzeptor (1:5) bezogen auf Gew.-%. Die Erfindung sieht in besonders bevorzugter Ausführungsform vor, dass Lactose der Galactosyl-Donor ist.

Die Erfindung sieht in einer weiteren bevorzugten Ausführungsform vor, dass das mindestens eine Enzym, nämlich die β-Galactosidase in freier, bevorzugt gelöster, und/oder in immobilisierter Form vorliegt. Es ist vorgesehen, dass das Enzym in vollständig oder partiell gereinigter Form oder auch als Rohextrakt vorliegt. Es ist auch vorgesehen, dass das Enzym natürlichen Ursprungs oder synthetisch ist, wobei es erfindungsgemäß bevorzugt mittels rekombinanter Methoden oder weiterer an sich bekannter Verfahren hergestellt wird. Es ist schließlich vorgesehen, dass das Enzym eine Abwandlung, Mutante, Mutein, Modifikation oder Derivat eines natürlichen Enzyms ist.

Erfindungsgemäß wird es - besonders vorteilhaft - ermöglicht, durch Wahl der Versuchsbedingungen, insbesondere je nach Auswahl der konkret eingesetzten β-Galactosidase, die Zusammensetzung der erfindungsgemäß erhaltenen Galactosyl-Saccharid-haltigen beziehungsweise Galactosyl-Saccharidalkohol-haltigen Gemische, vorherbestimmbar zu steuern: Das erfindungsgemäße Verfahren ermöglicht die Herstellung eines galactosylierten Produkts, wobei dieses in β-1,3-Stellung, in β-1,4-Stellung und/oder in β-1,6-Stellung verknüpftes Galactosyl-Trisaccharid beziehungsweise Galactosyl-Trisaccharidalkohol in einstellbarer Zusammensetzung enthält.

In einer bevorzugten Ausführungsform der vorliegenden Erfindung wird β-Galactosidase aus Stierhoden verwendet. Bei Verwendung von β-Galactosidase aus Stierhoden ergibt sich ein Galactosylierungsprodukt, welches einen erhöhten Anteil an β-(1,3)-verknüpftem Galactosyl-Produkt, insbesondere β-(1,3)-Galactosyl-1,6-GPS und/oder β-(1,3)-Galactosyl-1,1-GPM und/oder β-(1,3)-Galactosyl-Isomaltulose, insbesondere mehr als 50 Gew.-% β-1,3-Galactosyl-Produkt (bezogen auf DP 3-Produkte) aufweist.

In einer weiteren bevorzugten Ausführungsform wird β-Galactosidase in Form der Enzyme BgaB aus *Bacillus stearothermophilus* KVE39, BgaT aus *Thermus brockianus* ITI360 und BgIT aus *Thermus thermophilus* TH125 verwendet, wobei ebenfalls ein Galactosylierungsprodukt mit einem besonders hohen Anteil an dem vorgenannten β-(1,3)-Galactosyl-Produkt, insbesondere mehr als 50 Gew.-% an β-1,3-Galactosyl-Produkt (bezogen auf DP 3-Produkte) erhalten wird (alle drei Mikroorganismen und darin enthaltenden β-Galactosidasen werden in Fridjonsson O, Watzlawick H, Mattes R (2000), The structure of the alpha galactosidase gene loci in Thermus brockianus ITI360 and Thermus thermophilus TH125. Extremophiles 4: 23-33 und Ganter C, Böck A, Buckel P, Mattes R (1988), Production of a thermostable, recombinant alpha galactosidase suitable for raffinose elimination from sugar beet syrup. J. Biotechnol. 8: 301-310 beschrieben).

In einer weiteren bevorzugten Ausführungsform ist vorgesehen, für die Galactosylierung eine β-Galactosidase aus *Bacillus circulans* einzusetzen, wobei ein Produkt mit einem erhöhten Anteil an β-(1,4)-Galactosyl-Produkt, insbesondere β-(1,4)-Galactosyl-1,6-GPS und/oder β-(1,4)-Galactosyl-1,1-GPM und/oder β-(1,4)-Galactosyl-Isomaltulose erhalten wird; insbesondere beträgt der Anteil an β-(1,4)-Galactosyl-Produkt mehr als 50 Gew.-% (bezogen auf DP 3-Produkte).

In einer weiteren bevorzugten Ausführungsform ist schließlich vorgesehen, für die Galactosylierung eine β-Galactosidase aus *Aspergillus oryzae* zu verwenden, wobei ein Produkt mit einem erhöhten Anteil an β-(1,6)-Galactosyl-Produkt, insbesondere β-(1,6)-Galactosyl-1,6-GPS und/oder β-(1,6)-Galactosyl-1,1-GPM und/oder . β-(1,6)-Galactosyl-Isomaltulose erhalten wird; insbesondere beträgt der Anteil an β-(1,6)-Galactosyl-Produkt mehr als 50 Gew.-% (bezogen auf DP 3-Produkte).

In erfindungsgemäß besonders bevorzugten Varianten werden aus den genannten bakteriellen Organismen stammende β-Galactosidase-codierende Gene in Expressionsvektoren integriert, in Wirtsstämme eingeführt, die selbst keine β-Galactosidasen produzieren, bevorzugt *E. coli,* und Produktionsstämme erhalten, die die gewünschten β-Galactosidasen herstellen. Da die gebildeten Enzyme vorzugsweise aus thermostabilen Organismen stammen, lassen sie sich aus den Rohextrakten von bevorzugt *E. coli* durch Hitzepräzipitation der Wirtsproteine reinigen. Die erhaltenen Rohextrakte weisen insbesondere von 60 bis 90 Gew.-% des hergestellten Enzyms auf.

Erfindungsgemäß bevorzugt wird das vorliegende Verfahren bei einem sauren pH-Wert, insbesondere bei einem pH-Wert von 4,0 bis 6,5 durchgeführt, vorzugsweise in gepufferter Lösung. Es ist vorgesehen, die Galactosylierungsreaktion vorzugsweise für eine Zeitdauer von 1 bis 50 Stunden bei einer Temperatur von vorzugsweise 30°C bis 55°C durchzuführen, wobei insbesondere bei der Verwendung von β-Galactosidase aus Stierhoden eine Zeitdauer von 40 bis 50 Stunden bei einer Temperatur von 33°C bis 40°C, von β-Galactosidase aus *Bacillus circulans* eine Zeitdauer von 1 bis 3 Stunden bei einer Temperatur von 50°C bis 60°C und von β-Galactosidase aus *Aspergillus oryzae* eine Zeitdauer von 1 bis 3 Stunden bei einer Temperatur von 25°C bis 35°C vorgesehen ist.

Erfindungsgemäß ist bevorzugt vorgesehen, dass zur Abtrennung und Isolierung aller vorgenannten Reaktionsprodukte physikalische und/oder chemische Trennverfahren eingesetzt werden, die eine Abtrennung von Reaktionsprodukten mit einem gewünschten Polymerisationsgrad erlauben. Erfindungsgemäß bevorzugt werden die ausgewählten Reaktionsprodukte mittels eines chromatographischen Schrittes in an sich bekannter Weise aufgereinigt oder angereichert. Im Zusammenhang mit der vorliegenden Erfindung wird unter einem "chromatographischen Trennverfahren" jedwedes physikalische Verfahren verstanden, bei dem eine Stofftrennung durch Verteilung zwischen einer stationären und einer mobilen Phase erfolgt, wobei als Trennmechanismen bevorzugt Adsorption, ionische Wechselwirkungen, polare bzw. apolare Wechselwirkungen, Größenausschluß, Komplexbildung zugrunde liegen. In einer bevorzugter Ausführungsform der Erfindung erfolgt die Abtrennung der Reaktionsprodukte unter Verwendung von Gelpermeationsverfahren, die auch als Ausschlusschromatographie, Molekularsiebchromatographie- oder Gelfiltrationsverfahren bezeichnet werden. Unter _{"}Gelpermeation" wird der Vorgang verstanden, bei dem infolge der Wanderung von Molekülen durch eine Gelmatrix mit Porenstruktur aufgrund eines Siebeffektes eine Verteilung nach der Molekulargröße erfolgt. In einer Variante werden zur Abtrennung ausgewählter Reaktionsprodukte aus dem Reaktionsgemisch Substanzen wie Polydextrane, Polyacrylamid, Agarose usw. als Gelmatrix eingesetzt. In einer besonders bevorzugten Variante wird zum Abreichem von Begleitstoffen ein Kationenaustauscher eingesetzt, der insbesondere mit Calcium-Ionen (Ca²⁺) beladen wurde.

In einer weiteren besonders bevorzugten Ausführungsform wird die aus der erfindungsgemäßen Umsetzung von Saccharid und/oder Saccharid-haltigem Gemisch, insbesondere Isomaltulose oder einem lsomaltulose-haltigen Gemisch, in dem ersten erfindungsgemäßen Verfahrensschritt erhaltene Galactosyl-Saccharid-Zusammensetzung, insbesondere Galactosyl-Isomaltulose-Zusammensetzung, in einem weiteren nachfolgenden zweiten Verfahrensschritt mittels chemischer Hydrierung, bevorzugt als katalytische Hydrierung unter Verwendung von Hydrierkatalysatoren, in Gegenwart von Wasserstoff (H₂) im Wesentlichen zu einem Galactosyl-Saccharidalkoholhatigen Gemisch, insbesondere zu einem Galactosyl-Isomalt-haltigen Gemisch, im Folgenden als Galactosyl-Isomalt-Zusammensetzung (B) bezeichnet, umgesetzt.

Erfindungsgemäß ist bevorzugt auch vorgesehen, dass in der durch Galaktosylierung nach einem der vorstehenden Verfahren in dem ersten Schritt als Produkt erhaltenen Galactosyl-Isomaltulose-Zusammensetzung, welche für die erfindungsgemäß bevorzugte Hydrierung als Edukt vorgesehen ist, einzelne Komponenten vor der Hydrierung durch chromatographische Abtrennung an- oder abgereichert werden.

Erfindungsgemäß bevorzugt wird eine aus dem vorgenannten Verfahren erhältliche Galactosyl-Saccharid-Zusammensetzung hydriert, indem das Gemisch beziehungsweise die Einzelubsubstanz in einem wässrigen Medium, vorzugsweise in Wasser, in einer Konzentration von 20 Gew.-% bis 40 Gew.-%, vorzugsweise 30 Gew.% gelöst wird. Erfindungsgemäß ist vorgesehen, dass der pH-Wert der wässrigen Lösung unter Verwendung geeigneter Mittel, insbesondere in einer gepufferten Lösung, auf einen pH-Wert von 6 bis 8 eingestellt wird. In einer bevorzugten Ausführungsform der Erfindung wird der pH-Wert der Lösung der zu hydrierenden Verbindung(en) durch Zugabe von Natronlauge auf 7,8 eingestellt. Erfindungsgemäß ist vorgesehen, dass die Hydrierung bei einer Temperatur von 40°C bis 140°C, insbesondere 60°C bis 80°C, vorzugsweise 70°C, erfolgt. Erfindungsgemäß ist auch vorgesehen, dass die Hydrierung in Gegenwart von Wasserstoff erfolgt, wobei in einer bevorzugter Ausführungsform des erfindungsgemäßen Verfahrens der verwendete Wasserstoff einen erhöhten Druck von 50 bis 230 bar, insbesondere 100 bis 200 bar, vorzugsweise etwa 150 bar, aufweist. Vorzugsweise wird während der Hydrierung kontinuierlich gerührt. Erfindungsgemäß ist insbesondere vorgesehen, dass die Hydrierung über einen Zeitraum von mindestens 2 Stunden bis 5 Stunden, vorzugsweise mindestens 4 Stunden erfolgt. Erfindungsgemäß ist vorgesehen, dass die Hydrierung kontinuierlich durchgeführt wird. In einer Variante wird die Hydrierung semikontinuierlich oder diskontinuierlich durchgeführt. Die erfindungsgemäße Hydrierung wird bevorzugt im Festbettverfahren und/oder im Suspensionsverfahren durchgeführt.

Erfindungsgemäß besonders bevorzugt erfolgt die Hydrierung als katalytische Hydrierung unter Verwendung eines Katalysators. In einer bevorzugten Ausführungsform der Erfindung wird als Katalysator aus einem Gemisch aus einem reinen Raney-Metall und einer Raney-Metalllegierung eingesetzt, wobei das Raney-Metall vorzugsweise Nickel, Kupfer, Kobalt oder Eisen ist. Bei der Raney-Metalllegierung handelt es sich vorzugsweise um eine Legierung aus Nickel, Kupfer, Kobalt und/oder Eisen mit mindestens einer insbesondere auslaugbaren Komponente wie Aluminium, Zinn oder Silicium. In einer weiteren bevorzugten Ausführungsform der Erfindung umfasst der zur Hydrierung eingesetzte Katalysator als Aktivkomponente ein oder mehrere Metalle der VIII. Nebengruppe des Periodensystems auf einem inerten Träger. Vorzugsweise wird als Aktivkomponente Platin, Ruthenium, Palladium, Rhodium und/oder Mischungen daraus eingesetzt. Der inerte Katalysatorträger umfasst vorzugsweise Kohlenstoff (Aktivkohle), Aluminiumoxid, Zirkoniumoxid, Siliciumoxid, Titandioxid und/oder Mischungen daraus.

Erfindungsgemäß bevorzugt wird bei der Hydrierung die Galactosyl-Isomaltulose-Zusammensetzung im Wesentlichen zu galactosyliertem 1,6-GPS und galactosyliertem 1,1-GPM oder einem Gemisch enthaltend diese Verbindungen, im Folgenden als Galactosyl-Isomalt-Zusammensetzung (B) bezeichnet, umgewandelt. Je nach Verknüpfung des Galactosylrestes zu 1,6-GPS und/oder 1,1-GPM liegt das Hydrierprodukt Gälactosyl-Isomalt-Zusammensetzung (B) als β-1,3-Galactosyl-1,6-GPS und/oder β-1,3-Galactosyl-1,1-GPM, als β-1,4-Galactosyl-1,6-GPS und/oder β-1,4-Galactosyl-1,1-GPM, und/oder als β-1,6-Galactosyl-1,6-GPS und/oder β-1,6-Galactosyl-1,1-GPM vor. Als weitere Bestandteile der Galactosyl-Isomalt-Zusammensetzung (B) treten bevorzugt die Zuckeralkohole Galactosyl-Isomalt, Galactosyl-Lactit, Galactosyl-Sorbit sowie Sorbit, Isomalt, Lactit und/oder Galactit auf.

In einer bevorzugten Variante wird das erfindungsgemäße Hydrierprodukt, das heißt die Galactosyl-Isomalt-Zusammensetzung (B), wie vorbeschrieben weiter aufgereinigt oder angereichert, um insbesondere an Galactosyl-Lactit und/oder Galactosyl-Isomalt-angereicherte Produkte, insbesondere gereinigtes oder isoliertes Galactosyl-Lactit und/oder Galactosyl-Isomalt, zu erhalten.

Die Erfindung betrifft auch die erfindungsgemäß hergestellten oder erhältlichen Reaktionsprodukte und Zwischenprodukte, insbesondere Galactosyl-Isomalt-haltige Gemische, wie die Galactosyl-Isomalt-Zusammensetzung (A) und die Galactosyl-Isomalt-Zusammensetzung (B), und/oder Galactosyl-Isomaltulose-haltige Gemische wie die Galactosyl-Isomaltulose-Zusammensetzung.

Gegenstand der vorliegenden Erfindung ist eine Galactosyl-Isomaltulose-Zusammensetzung, hergestellt durch Galactosylierung eines Eduktgemischs enthaltend Isomaltulose, welche bevorzugt 4 bis 30 Gew.-%, insbesondere 5 bis 15 Gew.-% β-1,6-Galactosyl-Isomaltulose (6), bevorzugt 6 bis 60 Gew.-%, insbesondere 10 bis 40 Gew.-% β-1,4-Galactosyl-Isomaltulose (5) und bevorzugt 15 bis 90 Gew.-%, insbesondere 25 bis 60 Gew.-% β-1,3-Galactosyl-Isomaltulose (4) enthält, insbesondere daraus besteht. Erfindungsgemäß werden β-1,3-Galactosyl-Produkt angereicherte Galactosyl-Isomaltulose-Zusammensetzungen bevorzugt, die mehr als 50 Gew.-% β-1,3-Galactosyl-Isomaltulose bezogen auf die DP 3-Produkte enthalten.

Gegenstand der vorliegenden Erfindung ist weiter eine Galactosyl-Isomalt-Zusammensetzung (A), hergestellt durch Galactosylierung eines Eduktgemischs enthaltend hydrierte Isomaltulose, insbesondere Isomalt, eine Galactosyl-Isomalt-Zusammensetzung (B), hergestellt durch Hydrierung eines Eduktgemischs enthaltend galactosylierte Isomaltulose, insbesondere die vorgenannte Galactosyl-Isomaltulose-Zusammensetzung, welche bevorzugt 4 bis 30 Gew.-%, insbesondere 5 bis 15 Gew.-% β-1,6-Galactösyl-Isomalt, bevorzugt 6 bis 60 Gew.-%, insbesondere 10 bis 40 Gew.-% β-1,4-Galactosyl-Isomalt und bevorzugt 15 bis 90 Gew.-%, insbesondere 25 bis 60 Gew.-% β-1,3-Galactosyl-Isomalt enthält, insbesondere daraus besteht. Die Angaben in Gew.-% sind jeweils bezogen auf Gesamtgehalt in der Trockensubstanz an DP3-Produkten im Reaktionsprodukt, das heißt Galactosyl-Isomalt. Erfindungsgemäß werden β-1,3-Galactosyl-Produkt angereicherte Galactosyl-Isomalt-Zusammensetzungen bevorzugt, die mehr als 50 Gew.-% β-1,3-Galactosyl-Isomalt bezogen auf die DP 3-Produkte enthalten.

Die Erfindung betrifft auch die vorgenannten Produkte, welche mindestens einen weiteren Zucker und/oder Zuckeralkohol enthalten, zum Beispiel Monosaccharide (DP 1) oder deren Alkohole, Disaccharide (DP 2) oder deren Alkohole und Tetrasaccharide (DP 4) oder deren Alkohole.

In einer bevorzugten Ausführungsform enthält die Galactosyl-isomaltulose-Zusammensetzung von 1 bis 45 Gew.-%, insbesondere 2 bis 30 Gew.-% Galactosyl-Isomaltulose auch Galactose sowie Glucose, Lactose, Isomaltulose und/oder Galactosyl-Lactose.

In einer weiteren bevorzugten Ausführungsform enthält die durch Galactosylierung von lsomalt-haltigem Gemisch erhaltene Galactosyl-Isomalt-Zusammensetzung (A) neben 1 bis 45 Gew.-%, insbesondere 2 bis 30 Gew.-% Galactosyl-Isomalte auch Galactose, Glucose, Lactose, Isomalt und/oder Galactosyl-Lactose. In einer Variante enthält die Galactosyl-Isomalt-Zusammensetzung (A) auch galactosyliertes 1,1-GPS. Weitere Gegenstände der Erfindung sind die bevorzugt durch Abtrennung aus der Galactosyl-Isomalt-Zusammensetzung (A) und/oder der Galactosyl-Isomaltulose-Zusammensetzung erhaltenen isolierten Bestandteile, insbesondere Galactosyl-Lactose.

In einer weiteren bevorzugten Ausführungsform enthält die durch die Hydrierung eines Galactosyl-Isomaltulose-haltigen Gemisches erhaltene Galactosyl-Isomalt-Zusammensetzung (B) von 1 bis 45 Gew.-%, insbesondere 2 bis 30 Gew.-% Galactosyl-Isomalt sowie Galactit, Sorbit, Lactit, Isomalt, Galactosyl-Sorbit und/oder Galactosyl-Lactit. Weitere Gegenstände der Erfindung sind auch die bevorzugt durch Abtrennung aus der Galactosyl-Isomalt-Zusammensetzung (B) erhaltenen isolierten Bestandteile, insbesondere Galactosyl-Isomalt, Galactosyl-Lactit und Galactosyl-Sorbit. In einer Variante enthält die Galactosyl-Isomalt-Zusammensetzung (B) auch galactosyliertes 1,1-GPS.

Die vorgenannten erfindungsgemäßen Einzelsubstanzen, Gemische und Zusammensetzungen liegen bevorzugt in vollständig oder partiell gereinigter Form vor und werden bevorzugt in dieser Form auch verwendet. In einer alternativen Ausführungsform liegen die erfindungsgemäßen Gemische und Zusammensetzungen ohne Abtrennung von Reaktionsnebenprodukten vor und werden in dieser Form auch verwendet. Die erfindungsgemäßen Gemische und Zusammensetzungen werden bevorzugt beispielsweise in getrockneter Form, als Suspension oder in wässriger Lösung verwendet.

In den erfindungsgemäßen Gemischen und Zusammensetzungen beträgt der Anteil der Bestandteile mit dem Polymerisierungsgrad von 1 (DP 1) bevorzugt von 2 bis 30 Gew.-%, der Anteil der Produkte mit dem Polymerisierungsgrad von 2 (DP 2) bevorzugt von 30 bis 90 Gew.-% und von Produkten mit dem Polymerisierungsgrad von 4 (DP 4) bevorzugt von 0 bis 5 Gew.-%. Dabei ist erfindungsgemäß die Anreicherung einzelner Reaktionsprodukte, insbesondere von DP 3-Produkten, Galactosyl-Isomalt beziehungsweise Galactosyl-Isomaltulose und/oder Galactosyl-Lactose beziehungsweise Galactosyl-Lactit und/oder Galactosyl-Sorbit, durch chromatographische Abtrennung vorgesehen.

Die Erfindung betrifft auch isolierte und gereinigte Gemische, bestehend aus β-1,3-Galactosyl-Isomalt, β-1,4-Galactosyl-Isomalt und β-1,6-Galactosyl-lsomalt, oder diese im Wesentlichen enthaltend, zum Beispiel in einer Menge von ≥ 80, ≥ 90, ≥ 95 Gew.-% Trockensubstanz. Die Erfindung betrifft ebenso isoliertes β-1,3-Galactosyl-isomalt. Die Erfindung betrifft auch isoliertes β-1,4-Galactosyl-isomalt. Außerdem betrifft die Erfindung auch isoliertes β-1,6-Galactosyl-Isomalt.

Die Isolation der erfindungsgemäßen Verbindungen wird bevorzugt mit einem präparativen Chromatographiesystem erreicht. Die vorzugsweise mittels Gelpermeationschromatographie (siehe folgendes Beispiel 4) erhaltenen DP 3-Bereiche können in bevorzugter Ausführung mittels präparativer HPAEC z.B. unter folgenden Bedingungen in die erwünschten Einzel-Komponenten aufgetrennt werden:
Trennsäulen: 2 x Carbopac PA 1, je 22 mm x 250 mm, Anionenaustauscher, Fa. Dionex
Flussrate: 30 mL/Min
Aufgabevolumen: 0,6 mL
Detektion: Gepulster amperometrischer Detektor (Goldelektrode),
Aliquot des Eluates, Eluent, Elutionszeit, Potentiale und Pulszeiten: siehe Beispiel 5.

Eine wiederholte Aufgabe der jeweiligen Ausgangslösungen erbrachte die für NMR-Untersuchungen benötigten Substanzmengen. Die entsprechend vereinten Fraktionen wurden am Rotationsverdampfer aufkonzentriert, mikrofiltriert und gefriergetrocknet.

Die Erfindung betrifft also auch die einzelnen Stereoisomere, vorzugsweise in isolierter Form, der drei sogenannten Galactosyl-Isomalte, das heißt jeweils das Mannit- oder Sorbit-Stereoisomer von β-1,3-, von β-1,4- und von β-1,6-Galactosyl-Isomalt.

Die Erfindung betrifft also auch Verfahren zu der Herstellung der drei Galactosyl-Isomalte in voneinander getrennter Form unter Einsatz des vorgenannten Verfahrens, wobei anschließend das jeweils gewünschte konkrete Galactosyl-Isomalt aufgereinigt und von den beiden anderen Formen abgetrennt und isoliert wird und in isolierter Form oder in einem Gemisch das konkrete Galactosyl-Isomalt im Wesentlichen enthaltend, zum Beispiel in einer Menge von ≥ 80, ≥ 90, ≥ 95 Gew.-% Trockensubstanz, erhalten wird.

Des weiteren betrifft die Erfindung auch Verfahren zur Herstellung der einzelnen Stereoisomere der drei Galactosyl-Isomalte, vorzugsweise in isolierter Form, das heißt jeweils das Mannit- oder Sorbit-Stereoisomer von β-1,3-, von β-1,4- und von β-1,6-Galactosyl-Isomalt unter Einsatz des vorgenannten Verfahrens, wobei das jeweils gewünschte konkrete Galactosyl-Isomalt Stereoisomer aufgereinigt und von den anderen Formen abgetrennt und isoliert wird und in isolierter Form oder in einem Gemisch das konkrete Galactosyl-Isomalt Stereoisomer im Wesentlichen enthaltend, zum Beispiel in einer Menge von ≥ 80, ≥ 90, ≥ 95 Gew.-% Trockensubstanz, erhalten wird.

Die Erfindung betrifft darüber hinaus isolierte und gereinigte Galactosyl-Isomaltulose-Gemische, bestehend aus β-1,3-Galactosyl-Isomaltulose, β-1,4-Galactosyl-Isomaltulose und β-1,6-Galactosyl-Isomaltulose, oder diese im Wesentlichen enthaltend, zum Beispiel in einer Menge von ≥ 80, ≥ 90, ≥ 95 Gew.-% Trockensubstanz. Die Erfindung betrifft ebenso isolierte β-1,3-Galactosyl-Isomaltulose. Die Erfindung betrifft auch isolierte β-1,4-Galactosyl-Isomaltulose. Außerdem betrifft die Erfindung auch isolierte β-1,6-Galactosyl-Isomaltulose.

Die Erfindung betrifft also auch Verfahren zu der Herstellung der drei Galactosyl-Isomaltulosen in voneinander getrennter Form unter Einsatz des vorgenannten Verfahrens, wobei anschließend die jeweils gewünschte konkrete Galactosyl-Isomaltulose aufgereinigt und von den beiden anderen Formen abgetrennt und isoliert wird und in isolierter Form oder in einem Gemisch die konkrete Galactosyl-Isomaltulose im Wesentlichen enthaltend, zum Beispiel in einer Menge von ≥ 80, ≥ 90, ≥ 95 Gew.-% Trockensubstanz, erhalten wird.

In einer weiteren Ausführungsform betrifft die Erfindung auch die Verwendung der vorgenannten erfindungsgemäßen Galactosyl-Isomalt-Zusammensetzung (A) oder/und Galactosyl-Isomalt-Zusammensetzung (B) oder/und Galactosyl-Isomaltulose-Zusammensetzung und/oder der in erfindungsgemäßen Gemischen enthaltenen Reinsubstanzen, im Folgenden als erfindungsgemäße Produkte bezeichnet, hauptsächlich β-1,3-Galactosyl-Isomalt, β-1,4-Galactosyl-Isomalt, β-1,6-Galactosyl-Isomalt, β-1,3-Galactosyl-Isomaltulose, β-1,4-Galactosyl-Isomaltulose, β-1,6-Galactosyl-Isomaltulose, Galactosyl-Lactose, Galactosyl-Lactit und/oder Galactosyl-Sorbit sowie Mischungen von mindestens zwei Substanzen daraus als Nahrungs-, Lebens-, Genuss- und/oder Tierfuttermittel, insbesondere mit gesundheitsfördernder, therapeutischer oder/und prophylaktischer Wirkung. Die Erfindung betrifft aber auch die Verwendung der vorgenannten erfindungsgemäßen Produkte zur Herstellung von Nahrungs-, Lebens-, Genuss- und/oder Tierfuttermitteln sowie von Arzneimitteln.

Erfindungsgemäß wurde überraschend gefunden, dass die erfindungsgemäßen Produkte unter den Bedingungen im Magen und von den Dünndarmenzymen nicht hydrolysiert werden. In besonders vorteilhafter Weise findet die Fermentierung der erfindungsgemäßen Produkte durch Probiotika wie humane Bifidobakterien also signifikant langsamer als bei bekannten präbiotischen Substanzen statt. In besonders vorteilhafter Weise wird dadurch erreicht, dass die erfindungsgemäßen. Produkte nicht nur im vorderen Abschnitt des Dickdarms sondern über seine gesamte Länge, auch im hinteren Abschnitt, ihre positiven Effekte entfalten. Im Unterschied zu den bevorzugt eingesetzten Galactose-Akzeptor-Molekülen Isomalt und Isomaltulose gelangen die erfindungsgemäßen Produkte in den Dickdarm und wirken als unverdauliche Kohlenhydrate physiologisch als lösliche Ballaststoffe und fördern insbesondere die Darmgesundheit. Als niedermolekulare Oligosaccharide weisen die erfindungsgemäßen Produkte dabei jedoch nicht die anwendungstechnischen Nachteile anderer löslicher Ballaststoffe beziehungsweise unverdaulichen Kohlenhydrate auf.

Die erfindungsgemäßen Produkte sind im Dickdarm fermentierbar und bedingen dort mittels der dortigen Mikroflora die Bildung von nützlichen Fermentierungsprodukten, insbesondere der vorteilhaften Buttersäure beziehungsweise Butyrat. Butyrat ist die bevorzugte Energiequelle der Colonepithelzellen und daher maßgeblich für eine gesunde Dickdarmfunktion. Es fördert das Wachstum und die Differenzierung eines gesunden Epithels und den programmierten Zelltod (prä-)neoplastischer Zellen. Es reduziert zusammen mit anderen kurzkettigen Fettsäuren mutagene und andere toxische Substanzen im Dickdarmlumen und erhöht die Reduktionskraft, das heißt die Abwehrkraft, des Organismus, indem es beispielsweise über die Induktion der Genexpression protektiver Proteine wie der intestinalen Glutathion-S-Transferase und der dadurch bedingten Erhöhung detoxifizierender zellulärer Reduktionsäquivalente in Form von Glutathion. Dadurch wird oxidativer Stress verringert. Weiterhin reduzieren kurzkettige Fettsäuren einschließlich Butyrat den luminalen pH und bewirken so eine Inhibierung schädlicher bakterieller Stoffwechselaktivitäten wie β-Glucosidasen, Azoreduktasen oder Nitroreduktasen. Zudem unterdrückt ein niedriger Dickdarm-pH die schädliche Darmflora und verbessert die Wachstumsbedingungen für positive Mikroorganismen.

Weitere Effekte dieser Fermentationsprodukte der erfindungsgemäßen Produkte bestehen in ihrer Funktion als Substrate zur Stärkung der Funktion der Darmwand, in der Reduktion toxischer luminaler Substanzen, in der induzierenden Wirkung auf die intrazelluläre Synthese des Antioxidans Glutathion und der Glutathion-S-Transferase, in der antiproliferativen Wirkung auf Krebszellen, in der antineoplastischen Wirkung und in der Fähigkeit zur Erhöhung der Zelldifferenzierung. Die erfindungsgemäßen Produkte sind daher hervorragend als Mittel zur Behandlung und/oder Prophylaxe von Krankheiten, insbesondere zur Behandlung und/oder Prophylaxe von Krankheiten, die mit oxidativem Stress in Zusammenhang stehen, von Krebserkrankungen und von entzündlichen Darmerkrankungen vor allem des Dickdarmsbereichs geeignet. Weiterhin sind die erfindungsgemäßen Produkte auch in der Lage, über ihre Wechselwirkung mit Oberflächenstrukturen von insbesondere pathogenen Keimen diese zu blockieren, so dass deren Infektiösität herabgesetzt ist.

Die erfindungsgemäß bereitgestellten Produkte überwinden die Nachteile und Probleme des Standes der Technik und stellen im Sinne der Erfindung geeignete Mittel zur Gesundheitsförderung, Prophylaxe und Behandlung einer Vielzahl von Erkrankungen dar. Die erfindungsgemäßen Produkte wirken im Körper erfindungsgemäß bevorzugt als Wirkstoff direkt auf zelluläre Makromoleküle und induzieren dadurch bedingt eine Reihe von Funktionsänderungen, rufen also eine biologische Wirkung hervor. Erfindungsgemäß ist auch vorgesehen, dass die Abbau- oder Fermentationsprodukte der erfindungsgemäßen Produkte im Körper als Wirkstoffe fungieren. Die Erfindung betrifft daher auch die Verwendung der erfindungsgemäßen Produkte als Mittel oder Wirkstoff zur Behandlung und/oder Prophylaxe von Krankheiten, die durch unverdauliche Kohlenhydrate beeinflusst werden können.

Im Zusammenhang mit der vorliegenden Erfindung werden unter einer "Krankheit" oder "Erkrankung" Störungen der Lebensvorgänge und/oder Mangelzustände in einem Organismus verstanden, die mit subjektiv empfundenen und/oder objektiv feststellbaren physischen Veränderungen einhergehen. Unter "Krankheiten" werden erfindungsgemäß bevorzugt Erkrankungen, die durch unverdauliche Kohlenhydrate beeinflusst werden können, verstanden sowie Infektionskrankheiten, Krebserkrankungen, insbesondere des Dickdarmbereiches wie die Colonkarzinogenese, Obstipation, Hypertonie, Schlaganfall, durch pathogene Keime hervorgerufene Krankheiten, rheumatische Erkrankungen, Osteoporose, Koronararterien-Erkrankungen, akuter Herzinfarkt, weitere Herz-Kreislauf-Erkrankungen, chronischentzündliche Krankheiten, insbesondere des Darmbereiches und entzündliche Darmerkrankungen verstanden.

Unter "Krankheiten" werden erfindungsgemäß bevorzugt auch Erkrankungen verstanden, die im Wesentlichen durch "oxidativen Stress" verursacht werden. "Oxidativer Stress" ist ein Zustand, bei dem im Körper beziehungsweise spezifischen Organen oder Geweben ein Ungleichgewicht zwischen der Bildung und dem Abbau freier Radikalen besteht, wobei "freie Radikale" Moleküle beziehungsweise deren Bruchstücke und Atome sind, die durch ein einzelnes ungepaartes Elektron charakterisiert und daher äußerst reaktionsfähig sind.

Eine weitere bevorzugte Ausführungsform betrifft die Verwendung der erfindungsgemäßen Produkte als Mittel oder Wirkstoff zur Behandlung und/oder Vorbeugung von Verstopfung, als Wirkstoff zur Wiederherstellung und Intakterhaltung einer gesunden Mikroorganismen-Flora, als Wirkstoff zur Verbesserung der Resorbierung von Nahrungsbestandteilen, insbesondere von Mineralien wie Kalzium, insbesondere im tierischen oder menschlichen Verdauungstrakt, wobei insbesondere Nahrungsmittelmangelerscheinungen verhindert und/oder verringert werden, und als Wirkstoff zur Verhinderung und/oder Behandlung von Durchfallerkrankungen, die bei den meisten Infektionen des Darms mit Mikroorganismen (=bakterielle oder virale Enteritiden) auftritt, beispielsweise die Reisediarrhoe hervorgerufen durch enterotoxinbildende *E.coli*-Stämme sowie andere darmpathogene Bakterien, Viren, Pilze und Parasiten, auch Amöbenruhr. Schließlich betrifft eine besonders bevorzugte Ausführungsform die Verwendung der erfindungsgemäßen Produkte als Wirkstoff zur Stärkung der Immunabwehr gegen allgemeine Infekte, als Wirkstoff zur Prophylaxe von Infektionskrankheiten, zur Prophylaxe von Darmerkrankungen, zur Prophylaxe von Obstipation, zur Prophylaxe der Colonkarzinogenese, zur Prophylaxe von entzündlichen Erkrankungen und/oder zur Prophylaxe der Osteoporose.

Eine weitere bevorzugte Ausführungsform der Erfindung betrifft die Verwendung der erfindungsgemäßen Produkte als pharmazeutischer Träger in einer pharmazeutischen Zusammensetzung vorgesehen.

Selbstverständlich betrifft die Erfindung auch die Verwendungen der erfindungsgemäßen Produkte zur Herstellung von pharmazeutischen Zusammensetzungen und/oder Arzneimitteln zur Therapie und/oder Prophylaxe der vorgenannten Krankheiten. Im Zusammenhang mit der vorliegenden Erfindung wird unter einer "pharmazeutischen Zusammensetzung" oder einem "Arzneimittel" ein zu diagnostischen, therapeutischen und/oder prophylaktischen Zwecken verwendetes, also ein die Gesundheit eines menschlichen oder tierischen Körpers förderndes oder wiederherstellendes Erzeugnis oder Stoffgemisch in fester, flüssiger, gelöster oder suspendierter Form verstanden, das mindestens einen natürlichen oder synthetisch hergestellten Wirkstoff umfasst, der die therapeutische Wirkung hervorruft. Die pharmazeutische Zusammensetzung kann sowohl ein festes als auch ein flüssiges Gemisch sein. Beispielsweise kann eine den Wirkstoff umfassende pharmazeutische Zusammensetzung einen oder mehrere pharmazeutisch verträgliche Excipienten enthalten. Darüber hinaus kann die pharmazeutische Zusammensetzung üblicherweise auf dem Fachgebiet verwendete Zusatzstoffe, wie Stabilisatoren, Fertigungsmittel, Trennmittel, Sprengmittel, Gleitmittel, Farbstoffe, Geruchsstoffe, Geschmacksstoffe, Emulgatoren oder andere üblicherweise zur Herstellung pharmazeutischer Zusammensetzungen verwendete Stoffe umfassen.

Erfindungsgemäß ist vorgesehen, dass die erfindungsgemäßen Produkte in einer Dosis verabreicht werden, die ausreicht, beispielsweise den Zustand einer durch oxidativen Stress verursachten Krankheit oder den Zustand einer Infektionskrankheit vorzubeugen, die Progression einer solchen Krankheit zu stoppen, die Symptome zu lindern und/oder insbesondere die Krankheit zu heilen. Vorzugsweise werden die erfindungsgemäßen Produkte oral verabreicht, so dass sie über den Magen-Darm-Trakt in den Dickdarm gelangen kann. Die Dosierung der erfindungsgemäßen Produkte hängt dabei unter anderem von der Darreichungsform, dem Alter, dem Geschlecht und dem Körpergewicht des betroffenen Organismus, insbesondere des betroffenen Menschen oder eines zu behandelnden Tieres, des Krankheitsrisikos sowie der Schwere der Erkrankung ab. In einer bevorzugten Ausführungsform der Erfindung ist vorgesehen, dass die erfindungsgemäßen Produkte in Form einer pharmazeutischen Zusammensetzung, verabreicht werden, um beispielsweise Krankheiten oder Infekte zu behandeln und/oder diesen vorzubeugen. Insbesondere ist vorgesehen, dass die die erfindungsgemäßen Produkte enthaltende pharmazeutische Zusammensetzung die Form einer oral zu verabreichenden pharmazeutischen Zusammensetzung, insbesondere die Form einer Suspension, Tablette, Pille, Kapsel, eines Granulats, eines Pulvers oder einer ähnlich geeigneten Darreichungsform aufweist. Obwohl die erfindungsgemäß eingesetzten erfindungsgemäßen Produkte gegenüber Magensäure unempfindlich sind, können die erfindungsgemäßen Produkte auch in Arzneimittelformen enthalten sein, die eine magensaftresistente Beschichtung aufweisen. In solchen Arzneimittelformen können die in der pharmazeutischen Zusammensetzung enthaltenen Wirkstoffe den Magen ungehindert passieren und werden vorzugsweise erst in den oberen oder mittleren Darmabschnitten freigesetzt. Die Zusammensetzung von magensaftresistenten Beschichtungen und Verfahren für die Herstellung solcher magensaftresistenter Beschichtungen sind auf dem Fachgebiet bekannt. In einer besonders bevorzugten Ausführungsform der Erfindung werden Arzneimittelformen verwendet, die einen verzögerten Wirkstofffreisetzungsmechanismus aufweisen, um somit eine längerfristige Therapie von Krankheiten zu ermöglichen. Der Aufbau und die Zusammensetzung solcher Arzneimittelformen mit verzögerter Wirkstofffreisetzung sind ebenfalls auf dem Fachgebiet bekannt.

In einer besonders bevorzugten Ausführungsform der Erfindung ist vorgesehen, dass die die erfindungsgemäßen Produkte enthaltende pharmazeutische Zusammensetzung im Rahmen einer Kombinationstherapie zur Behandlung, insbesondere zur Prophylaxe von Krankheiten eingesetzt wird. Erfindungsgemäß ist also vorgesehen, dass neben den erfindungsgemäßen Produkte als Wirkstoffe gleichzeitig mindestens ein weiterer Wirkstoff beziehungsweise mindestens ein weiteres Arzneimittel für die gleiche Indikation verabreicht werden. Die kombinierte Anwendung der erfindungsgemäßen Produkte und des mindestens einen zusätzlichen Wirkstoffs beziehungsweise Arzneimittels kann auf die Verstärkung von therapeutischen oder prophylaktischen Wirkungen abzielen, kann jedoch auch auf verschiedene biologische Systeme im Organismus wirken und so die Gesamtwirkung verstärken. Die erfindungsgemäßen Produkte und das mindestens eine zusätzliche Arzneimittel können entweder getrennt oder in Form fixer Kombinationen verabreicht werden. Die Auswahl des zusätzlichen Arzneistoffes oder Wirkstoffes hängt hauptsächlich von der konkret zu behandelnden Krankheit und deren Schwere ab. Handelt es sich bei der Erkrankung zum Beispiel um eine mit oxidativem Stress im Zusammenhang stehende Erkrankung wie ein manifestiertes Colonkarcinom, so kann eine gegebenenfalls vom Arzt verordnete Basis-Chemotherapie, beispielsweise unter Anwendung von 5-Fluorouracil, durch gleichzeitige Verabreichung der erfindungsgemäßen Produkte unterstützt werden. Handelt es sich bei der Erkrankung um manifestierten Diabetes, so kann beispielsweise die medikamentöse Therapie der Makroangiopathie beim Diabetiker unter Verwendung von Plättchenaggregations-Hemmern durch gleichzeitige Verabreichung der erfindungsgemäßen Produkte unterstützt werden.

In einer weiteren bevorzugten Ausführungsform der Erfindung ist vorgesehen, dass die Verwendung der erfindungsgemäßen Produkte zur Prophylaxe und/oder Behandlung von Krankheiten von, insbesondere monogastrischen, Tieren wie Haustiere und Nutztiere dadurch erfolgt, dass die erfindungsgemäßen Produkte als Zusätze in Tierfuttermitteln oder in Trinkwasser verabreicht werden. Diese gelangen somit mit der aufgenommenen Nahrung in den Verdauungstrakt des Tieres, wo dann im Dickdarmbereich eine Fermentation durch die Darmflora erfolgt. Die Zufuhr der erfindungsgemäß verwendeten Produkte über die Nahrung ist insbesondere zur Krankheitsprophylaxe geeignet. Bei regelmäßiger Verfütterung von die erfindungsgemäßen Produkte enthaltenden Tierfuttermitteln ist eine langfristige Krankheitsprophylaxe möglich. Im Zusammenhang mit der vorliegenden Erfindung werden unter "Futtermittel" oder "Tierfuttermittel" jedwede Stoffe oder Stoffgemische verstanden, die dazu bestimmt sind, in unverändertem, zubereitetem, bearbeitetem oder verarbeitetem Zustand an Tiere verfüttert zu werden. Tierfuttermittel können sowohl in fester Form als auch in flüssiger Form vorliegen. Die Begriffe "Futtermittel" und "Tierfuttermittel" umfassen daher auch modifizierte oder supplemetierte Trinkwasser für Tiere. Bei den Futtermitteln kann es sich sowohl um Einzelfuttermittel als auch um Mischfuttermittel handeln. Die erfindungsgemäßen Wirkstoffe können dem Tierfutter sowohl in gelöster Form als auch in fester Form beigemengt werden. Zur Verabreichung an Nutztiere wie Schweine können die erfindungsgemäßen Wirkstoffe den zur tierischen Ernährung verwendeten Tierfuttermitteln oder -zusatzstoffgemischen in Form von Pulver, Sirup der Granulat beigemengt werden. Die erfindungsgemäß eingesetzten Produkte können erfindungsgemäß ebenfalls dem Trinkwasser für Tiere zugegeben werden. Der Zusatz der erfindungsgemäßen Produkte zu Trinkwasser erfolgt vorzugsweise unmittelbar vor Gebrauch, indem die erfindungsgemäßen Produkte mit Trinkwasser beispielsweise in Form von Pulver, Sirup oder Granulat zugesetzt werden, so dass die erfindungsgemäß verwendeten Substanzen vorzugsweise rasch in Lösung gehen können.

In einer weiteren bevorzugten Ausführungsform der Erfindung ist vorgesehen, dass die Verwendung der erfindungsgemäßen Produkte zur Vorbeugung und/oder Behandlung der vorgenannten Krankheiten dadurch erfolgt, dass die erfindungsgemäßen Produkte als Lebensmittel, diätetischen Lebensmittel, als Zusatz zu Lebensmitteln, diätetischen Lebensmitteln und Getränken sowie in für den menschlichen Verbrauch bestimmtem Trinkwasser eingesetzt werden. Die erfindungsgemäßen Produkte gelangen somit mit der aufgenommenen Nahrung oder Flüssigkeit in den Verdauungstrakt des Menschen, wo dann im Dickdarmbereich eine Fermentation durch die darmbesiedelnde Mikroflora erfolgt. Die Zufuhr der erfindungsgemäß verwendeten Produkte über die Nahrung ist insbesondere zur Krankheitsprophylaxe beispielsweise von Infektionskrankheiten geeignet. Bei regelmäßigem Verzehr der die erfindungsgemäßen Produkte enthaltenden Lebensmittel oder Getränke ist eine langfristige Prophylaxe und Gesundheitsvorsorge möglich.

Im Zusammenhang mit der vorliegenden Erfindung werden unter dem Begriff "Lebensmittel" vorwiegend der menschlichen Ernährung dienende Erzeugnisse oder Stoffgemische in fester, flüssiger, gelöster oder suspendierter Form verstanden, die dazu vorwiegend bestimmt sind, in unverändertem, zubereitetem oder verarbeitetem Zustand vom Menschen verzehrt zu werden. Lebensmittel können neben ihren natürlichen Bestandteilen weitere Komponenten enthalten, die natürlicher oder synthetischer Herkunft sein können. Lebensmittel können sowohl in fester Form als auch in flüssiger Form vorliegen. Der Begriff "Lebensmittel" umfasst daher auch alle Arten von Getränken einschließlich Trinkwasser, die für den menschlichen Konsum bestimmt sind. Die erfindungsgemäß eingesetzten Produkte =können dem Lebensmittel sowohl in gelöster Form als auch im festen Zustand beigemengt werden. Unter einem "Genussmittel" werden vorwiegend dem beim Verzehr auftretenden Genuss des menschlichen oder tierischen Körpers dienende Stoffe oder Stoffgemische in fester, flüssiger, gelöster oder suspendierter Form verstanden.

Im Zusammenhang mit der vorliegenden Erfindung werden unter "diätetischen Lebensmitteln" Lebensmittel verstanden, die bestimmt sind, einem bestimmten Ernährungszweck dazu zu dienen, dass sie die Zufuhr bestimmter Nährstoffe oder anderer ernährungsphysiologisch wirkender Stoffe in einem bestimmten Mengenverhältnis oder in bestimmter Beschaffenheit bewirken. Diätetische Lebensmittel unterscheiden sich maßgeblich von Lebensmitteln vergleichbarer Art durch ihre Zusammensetzung oder durch ihre Eigenschaften. Diätetische Lebensmittel können in Fällen eingesetzt werden, wo bestimmte Emährungsanforderungen aufgrund von Krankheiten, Funktionsstörungen oder allergischen Reaktionen gegen einzelne Lebensmittel beziehungsweise deren Inhaltsstoffe erfüllt werden müssen. Diätetische Lebensmittel können ebenfalls sowohl in fester Form als auch in flüssiger Form vorliegen.

In einer bevorzugten Ausführungsform der Erfindung ist vorgesehen, dass die erfindungsgemäßen Produkte als Ballaststoffe, insbesondere als lösliche Ballaststoffe oder unverdauliche Kohlenhydrate, in Lebensmitteln eingesetzt werden. Im Zusammenhang mit der vorliegenden Erfindung wird unter einem "Ballaststoff" ein für menschliche oder tierische Enzyme unverdaulicher Bestandteil der Nahrung, insbesondere deren Anteil an unverdaulichen Kohlenhydraten, verstanden. Dieser kann jedoch auch durch Dickdarmbakterien zumindest teilweise fermentiert und somit in geringem Maße für den menschlichen oder tierischen Körper energetisch verwertbar sein. "Lösliche Ballaststoffe" sind in Lösungen, insbesondere wässrigen Lösungen, löslich. Bei Verwendung als Ballaststoff regulieren die erfindungsgemäßen Produkte die Energiedichte, die aus dem Anteil der Hauptnährstoffe resultiert, und den Verdauungsvorgang hinsichtlich der Transitzeit und der Resorption im Dünndarm. Die erfindungsgemäßen Produkte sind im besonderen Maße als löslicher Ballaststoff geeignet, da sie aufgrund der sehr guten Löslichkeit in Wasser im Dickdarmbereich in gelöster Form vorliegt und dadurch von der Darmflora vollständig oder nahezu vollständig fermentiert werden kann. Gegenüber anderen, häufig verwendeten Ballaststoffen wie Weizen- oder Haferkleie weisen die erfindungsgemäßen Produkte bei Verwendung als Ballaststoffe zudem den Vorteil auf, dass sie keine Substanzen enthalten, die zu unerwünschten Nebenwirkungen führen.

Eine weitere Ausführungsform der Erfindung betrifft die Verwendung der erfindungsgemäßen Produkte als präbiotische Ballaststoffe, insbesondere als Präbiotika. Im Zusammenhang mit der vorliegenden Erfindung wird unter einem "Präbiotikum" ein Nahrungs-, Genuss-, Futter- oder Arzneimittelbestandteil verstanden, der selektiv das Wachstum und/oder die Aktivität spezifischer Bakterien im menschlichen oder tierischen Verdauungstrakt, insbesondere Bifidobakterien und/oder Lactobacillen so stimuliert, dass gesundheitsfördernde Effekte zu erwarten sind. Präbiotika sind in der Regel nicht oder nur schwer verdaulich. Infolge der Fermentation der erfindungsgemäßen Produkte zu kurzkettigen Fettsäuren, insbesondere Butyrat in hoher Menge, kommt es besonders vorteilhaft bei Verwendung der erfindungsgemäßen Produkte im Dickdarmbereich zu einer deutlichen pH-Absenkung in den sauren Bereich. Aufgrund des gesenkten pH-Wertes im Dickdarmbereich verschlechtern sich die Lebensbedingungen für pathogene Darm-Mikroorganismen und gleichzeitig verbessern sich die Lebensbedingungen für acidophile und saccharolytische Mikroorganismen.

In einer weiteren bevorzugten Ausführungsform werden die erfindungsgemäßen Produkte in Kombination mit anderen löslichen oder unlöslichen, fermentierbaren oder nicht-fermentierbaren Ballaststoffen eingesetzt. In einer bevorzugten Variante dieser Ausführungsform werden die erfindungsgemäßen Produkte in Kombination mit mindestens einem weiteren Ballaststoff ausgewählt aus der Gruppe der Ballaststoffe oder unverdaubaren Kohlenhydraten bestehend aus löslichen Ballaststoffen wie kurzkettige Fructooligosaccharide, langkettige Fructooligosaccharide, Galactooligosaccharide, hydrolysiertes Guar Gum, wie "Sunfibre" oder "Benefibre", Lactulose, Xylo-Oligosaccharide, Lactosucrose, Malto-Oligosaccharide, wie "Fibersol-2" von Matsutani, Isomalto-Oligosaccharide, Gentio-Oligosaccharide, pektische Substanzen einschließlich Hydrolasen, Kondensationsprodukte aus Zuckern, Glucosyl-Sucrose, wie "Coupling Sugar" von Hayashibara, Sojabohnen-Oligosaccharide, Chito-Oligosaccharide, Chitosan-Oligosaccharide sowie unlösliche Ballaststoffe wie resistente Stärke, Haferfasern, Weizenfasern, Gemüsefasern zum Beispiel aus Erbsen, Tomaten, Fruchtfasern zum Beispiel aus Äpfeln, Beeren und Früchten des Johannisbrotbaums, wie "Caromax" von Nutrinova, Cellulosen und Zuckerrübenfasern, wie "Fibrex" von Danisco, eingesetzt. Ein weiterer Gegenstand der Erfindung ist daher auch eine Ballaststoffzusammensetzung enthaltend eine Galactosyl-Isomalt-Zusammensetzung und/oder eine Galactosyl-Isomaltulose-Zusammensetzung und mindestens einen weiteren Ballaststoff, ausgewählt aus der Gruppe der vorgenannten Ballaststoffe.

Neben Mischungen der erfindungsgemäßen Produkte mit mindestens einem der vorgenannten Ballaststoffe sind erfindungsgemäß bevorzugt auch Mischungen der erfindungsgemäßen Produkte, allein oder in Verbindung mit mindestens einem der vorgenannten Ballaststoffe, mit Probiotika als sogenannte "Synbiotika" vorgesehen. Je nach Verwendung und Darreichungsform sind die bevorzugt zugesetzten probiotischen Biftdobakterien-, Lactobakterien- und/oder Enterobakterien-Kulturen als Lebendkulturen und/oder als Trockenkulturen oder Dauerkulturen ausgeführt. Im Zusammenhang mit der vorliegenden Erfindung werden unter einem "Synbiotikum" ein Gemisch aus mindestens einem Probiotikum und mindestens einem Präbiotikum verstanden, die durch Verbesserung der Überlebensrate und Erhöhung der Anzahl gesundheitsfördernder lebender mikrobieller Organismen im Gastrointestinaltrakt die Gesundheit des menschlichen oder tierischen Konsumenten fördern, insbesondere durch selektive Stimulierung des Wachstums und/oder der StoffwechselAktivität der mikrobiellen Organismen. Ein Gegenstand der Erfindung ist auch eine Synbiotikum enthaltend eine Galactosyl-Isomalt-Zusammensetzung und/oder eine Galactosyl-Isomaltulose-Zusammensetzung und mindestens ein Probiotikum, insbesondere Bifidobakterien.

Im Zusammenhang mit der vorliegenden Erfindung wird unter einem "Probiotikum" ein lebender mikrobieller Bestandteil eines Lebens-, Genuss-, Futter- oder Arzneimittels verstanden, der durch Stabilisierung oder Verbesserung der mikrobiellen Zusammensetzung im Verdauungstrakt des menschlichen oder tierischen Konsumenten dessen Gesundheit fördert. Derartige probiotische Mikroorganismen, die in Lebens-, Arznei- oder Futtermitteln eingesetzt werden können, sind zum Beispiel: Bifidobacterium wie die Stämme *B. adolescentis, B. animalis, B. bifidum, B. longum, B. thermophilum; Enterococcus, Lactobacillus* wie die Stämme *Lb. acidophilus, Lb. brevis, Lb. casei, Lb. cellobiosus, Lb. crispatus, Lb. delbrueckii subsp. Bulgaricus, Lb. fermentum, Lb.* GG, *Lb. johnsonii, Lb. lactis, Lb. plantarum, Lb. reuteri, Lb. rhamnosum, Lb. salivarius; Bacillus cereus toyoi; Bacillus cereus; Leuconostoc; Pediococcus acidilactici; Propionibacterium; Streptococcus* wie die Stämme *S. cremoris, S. infantarius, S. intermedius, S. lactis, S. salivarius subsp. thermophilus* (vergleiche Fuller, J. Appl. Bacteriol. (1989)). Bevorzugte Präbiotika sind Bakterien der Gattungen *Lactobacillus und Bifidobacterium.*

Die erfindungsgemäßen Produkte, allein oder als Synbiotika, dienen erfindungsgemäß als diätetische Faserquelle und/oder als Nährstoffe für eine gesunde Mikroflora der Behandlung und/oder Vorbeugung von Verstopfung, der Wiederherstellung und Intakterhaltung einer gesunden Mikroorganismenflora im Verdauungstrakt, der Verbesserung der Verfügbarkeit und der Resorbierung von Nahrungsbestandteilen, wie Mineralien, im tierischen oder menschlichen Verdauungstrakt allgemein der Unterstützung und Wiederherstellung der Gesundheit, insbesondere der Rekonvaleszenz, und verhindert, wie vorgenannt ausgeführt, die Entstehung von Dickdarmtumoren sowie von entzündlichen Darmerkrankungen. Erfindungsgemäß bevorzugt dienen die erfindungsgemäßen Produkte auch der Modulation und Unterstützung des Immunsystems des tierischen und menschlichen Körpers.

In einer weiteren bevorzugten Ausführungsform betrifft die Erfindung die Verwendung der erfindungsgemäßen Produkte zur Modulation der glykämischen Eigenschaften von Lebensmitteln oder Süßwaren, insbesondere Spezialernährung, Kinderernährung oder Ernährung für Personen mit Störungen des Glucose/Insulin-Haushalts, und der von ihnen ausgelösten glykämischen Reaktion. Unter "glykämischer Reaktion" versteht man die Änderung des Blutglucose-Spiegels nach Aufnahme eines leicht verdaulichen Kohlenhydrates. Die stärkste glykämische Reaktion verursachen solche Kohlenhydrate, aus denen nach oraler Aufnahme durch Speichel-, Pankreas- oder Dünndarmenzyme schnell Glucose freigesetzt und resorbiert werden kann. Ein Anstieg der Blutglucose bewirkt im gesunden Organismus eine Insulinausschüttung, wobei Insulin die Aufnahme von Glucose durch periphere Gewebe, zum Beispiel Skelettmuskeln, stimuliert, so dass der Blutwert wieder auf den Grundwert abfällt. Die erfindungsgemäßen Produkte können daher zur Prophylaxe und/oder Therapie von Diabetes mellitus und verwandten Stoffwechselerkrankungen wie Syndrom X, Insulin-Resistenz oder Glucose-Intoleranz, vorzugsweise als Bestandteil von diätetischen Lebens- und Genussmitteln verwendet werden. Ein weiterer Gegenstand der Erfindung ist die Verwendung der erfindungsgemäßen Produkte zur Prophylaxe und/oder Behandlung von Störungen des Glucosestoffwechsels, Diabetes I und II, Glucose-Intoleranz, Insulin-Resistenz und/oder Syndrom-X.

In einer weiteren bevorzugten Ausführungsform der Erfindung ist die Verwendung der erfindungsgemäßen Produkte als Süßungsmittel vorgesehen. Die erfindungsgemäßen Produkte besitzen eine verbesserte Süßungseigenschaften beziehungsweise Süßkraft. Sie können daher nicht nur als lösliche Ballaststoffe mit den damit verbundenen vorgenannten positiven Eigenschaften eingesetzt werden, sondern auch als Zuckeraustauschstoffe und/oder als Süßungsmittel, insbesondere in diätetischen Produkten. Da die erfindungsgemäßen Produkte auch nicht von der menschlichen Mundflora abgebaut werden, weisen sie vorteilhafte akariogene Eigenschaften auf. Die erfindungsgemäßen Produkte enthaltende Süßungsmittel zeichnen sich daher in vorteilhafter Weise durch ihre Akariogenität aus. Ein Gegenstand der Erfindung ist daher auch ein Süßungsmittel enthaltend die erfindungsgemäßen Produkte.

In einer weiteren bevorzugten Ausführungsform der Erfindung ist die Verwendung der erfindungsgemäßen Produkte zur Herstellung von Lebensmitteln, Süßwaren und Tierfuttermitteln vorgesehen. Insbesondere ist die Verwendung der erfindungsgemäßen Produkte zur Herstellung saurer Lebensmittel mit einem pH-Wert von 2 bis 5, insbesondere 2 bis 4, vorgesehen. Durch solche sauren Lebensmittel wird der präbiotische Effekt der erfindungsgemäßen Produkte unterstützt. Besonders bevorzugt werden die erfindungsgemäßen Produkte zur Herstellung von Fruchtsäften oder Fruchtsaftzubereitungen eingesetzt.

Die vorliegende Erfindung betrifft ebenfalls Nahrungs-, Lebensmittel und Genussmittel, die die erfindungsgemäßen Produkte allein oder in Verbindung mit mindestens einem weiteren Ballaststoff und/oder mit Probiotika enthalten. Weitere Gegenstände der Erfindung sind daher Nahrungs-, Lebens-, Genuss- oder Tierfuttermittel, enthaltend ein Synbiotikum, eine Ballaststoffzusammensetzung, eine Galactosyl-Isomalt-Zusammensetzung und/oder eine Galactosyl-Isomaltulose-Zusammensetzung.

Erfindungsgemäß ist vorgesehen, dass der mindestens eine weitere Ballaststoff ausgewählt ist aus der Gruppe der vorgenannten löslichen, unlöslichen Ballaststoffe oder unverdaubaren Kohlenhydrate. Da die erfindungsgemäßen Produkte unter den pH-Bedingungen des Magens und durch die Enzyme der Dünndarm-Mucosa praktisch nicht gespalten werden, handelt es sich bei den erfindungsgemäßen Nahrungs-, Lebensmittel und Genussmitteln, welche die erfindungsgemäßen Produkte in wirksamer Menge enthalten, in vorteilhafter Weise um kalorienreduzierte Nahrungs-, Lebens- oder Genussmittel. In bevorzugter Ausführungsform der Erfindung handelt es sich bei den erfindungsgemäßen Lebensmitteln um Milcherzeugnisse oder Milchprodukte, beispielsweise Käse-, Butter-, Joghurt-, Kefir-, Quark-, Sauermilch-, Buttermilch-, Sahne-, Kondensmilch-, Trockenmilch-, Molken-, Milchmisch-, Milchhalbfett-, Molkenmisch- Milchzucker-, Milcheiweiß- und Milchfett-Produkte. In einer weiteren bevorzugten Ausführungsform der Erfindung handelt es sich bei den erfindungsgemäßen Lebensmitteln um Backwaren, insbesondere Brot einschließlich Kleingebäck und feine Backwaren einschließlich Dauerbackwaren. In weiteren Ausführungsformen der Erfindung handelt es sich bei den erfindungsgemäßen Lebensmitteln um Brotaufstriche, Margarine-Erzeugnisse und Backfette sowie Instantprodukte und Brüherzeugnisse. In weiteren bevorzugten Ausführungsformen der Erfindung handelt es sich bei den erfindungsgemäßen Lebensmitteln um Obstprodukte, insbesondere Konfitüren, Marmeladen, Gelees, Obstkonserven, Fruchtpulpe, Fruchtmark, Fruchtsäfte, Fruchtsaftkonzentrate, Fruchtnektar und Fruchtpulver. Die die erfindungsgemäßen Produkte enthaltenen Lebensmittel können erfindungsgemäß auch Gemüseerzeugnisse, insbesondere Gemüsekonserven, Gemüsesäfte und Gemüsemark sein. In weiteren Ausgestaltungen der Erfindung handelt es sich bei den die erfindungsgemäßen Produkte enthaltenden Lebensmitteln um nicht-alkoholische Getränke Sportlergetränke, Getränkegrundstoffe und Getränkepulver.

Die vorliegende Erfindung betrifft in einer weiteren bevorzugten Ausführungsform die erfindungsgemäßen Produkte enthaltende Süßwaren. Die erfindungsgemäßen Produkte werden auch als Zuckeraustauschstoff und/oder Süßungsmittel auch in Süßwaren, insbesondere in diätetischen Produkten eingesetzt. Die erfindungsgemäßen Süßwaren zeichnen sich daher in vorteilhafter Weise durch ihre Akariogenität aus. Bei den erfindungsgemäßen Süßwaren handelt es sich insbesondere um Schokoladen-Erzeugnisse, Hartkaramellen, Weichkaramellen, Fondant-Erzeugnisse, Gelee-Erzeugnisse, Lakritzen, Schaumzuckerwaren, Kokosflocken, Dragees, Komprimate, kandierte Früchte, Krokant, Nougaterzeugnisse, Eiskonfekt, Marzipan, Kaugummi, Müsliriegel, sowie Speiseeis oder alkoholische oder nicht-alkoholische Süßgetränke.

Weitere vorteilhafte Ausgestaltungen der Erfindung ergeben sich aus den Unteransprüchen.

Die Erfindung wird anhand der folgenden Beispiele näher erläutert.

### Beispiel 1: Herstellung eines β-1,3-Galactosyl-Isomalt-angereichten Gemisches

1,17 mol/l Lactose als Donor werden mit β-Galactosidase aus Stierhoden (Enzymmenge: 0,75 Units) und 1,0 mol/l Isomalt (Palatinit® ) als Akzeptor für 48 Stunden bei 37°C in einem Mcllvain-Puffer (NaH₂PO₄/Zitronensäure 50 mmol/l) bei pH 4,3 umgesetzt. Es wird ein Galactosyl-Isomalt-haltiges Gemisch erhalten, welches hinsichtlich seiner DP 3-Bestandteile eine Anreicherung von β-1,3-Galactosyl-Isomalt (Formel (1)) aufweist.

### Beispiel 2: Herstellung eines β-1,4-Galactosyl-angereicherten Gemisches

### a) Herstellung eines β-1,4-Galactosyl-Isomalt angereicherten Gemisches

0,2 mol/l Lactose wird mit β-Galactosidase aus *Bacillus circulans* (Enzymmenge: 1,2 Units, Typ: E.C.3.2.1.23, "Biolacta N5", Hersteller: Daiwa Kasei Co. Ltd., Osaka) und mit 1,0 mol/l Isomalt für 2 Stunden bei 55°C in einem Natrium-Acetat-Puffer 50 mmol/l bei pH 5,0 umgesetzt. Die Reaktion wird durch Erhitzen für 10 min auf 90°C beendet. Es wird ein Galactosyl-Isomalt erhalten, welches hinsichtlich seiner DP 3-Bestandteile eine Anreicherung von β-1,4-Galactosyl-Isomalt (Formel (2)) aufweist.

### Analyse des Reaktionsprodukts:

Dazu werden 360,3 mg lsomalt (Monohydrat), entsprechend 1 mmol beziehungsweise 10 eq., und 36,0 mg Lactose, entsprechend 0,1 mmol, wie vorstehend beschrieben umgesetzt. Der Ertrag liegt bei 29,8 mg (0,059 mmol), entsprechend 59%. Ein weißer amorpher Feststoff mit C₁₈H₃₄O₁₆; MW=506,45; [α]²⁰_{D} +61 (c=0,8, H₂O); MALDI-TOF *m*/*z* 529,67 [M+Na]⁺, 545,63 [M+K]⁺ wird erhalten.

| ^{**1**}**H-NMR** (400 MHz, D₂O) | |
|---|---|
| d, 1 H, *J*_{*1,2*} = 3,6 Hz H-1 | d, 1 H, *J*_{*1",2"*} = 7,6 Hz H-1 " |
| 4,79 | 4,29 |

| ^{**12**}**C-NMR** (100,67 MHz, D₂O) | | | | | | |
|---|---|---|---|---|---|---|
| Gruppe | C-1 | C-2 | C-3 | C-4 | C-5 | C-6 |
| Gal*p*-(1→4) | 103,27 | 70,80 | 72,90 | 69,20 | 75,73 | 61,41 |
| Glc*p*-(1→4) | 98,22 | 71,54 | 71,34 | 78,72 | 72,15 | 60,21 |
| Glc*p*-ol | 62,79 | 73,25 | n.d. | n.d. | n.d. | 68,92 |
| Man*p*-ol | 63,63 | n.d. | n.d. | n.d. | n.d. | 68,92 |

### b) Herstellung eines β-1,4-Galactosyl-Isomaltulose angereicherten Gemisches

0,2 mol/l Lactose wird mit der β-Galactosidase aus *Bacillus circulans* und mit 1,0 mol/l Isomaltulose für 2 Stunden bei 55°C in einem Natrium-Acetat-Puffer 50 mmol/l bei pH 5,0 umgesetzt. Die Reaktion wird durch Erhitzen für 10 min auf 90°C beendet. Es wird ein Galactosyl-Isomalt erhalten, welches hinsichtlich seiner DP 3-Bestandteile eine Anreicherung von β-1,4-Galactosyl-Isomaltulose (Formel (5)) aufweist.

### Analyse des Reaktionsprodukts:

Dazu werden 342,3 mg Isomaltulose, entsprechend 1 mmol beziehungsweise 10 eq., und 36,0 mg Lactose, entsprechend 0,1 mmol, wie vorstehend beschrieben umgesetzt. Der Ertrag liegt bei 29,7 mg (0,059 mmol), entsprechend 59%. Ein weißer amorpher Feststoff mit C₁₈H₃₂O₁₆; MW=504,44; [α]²⁰_{D} +74 (c=0,5, H₂O); MALDI-TOF *m*/*z* 526,83 [M+Na]⁺ wird erhalten.

| ¹H-NMR (400 MHz, D₂O) | | |
|---|---|---|
| d, 1 H, *J*_{*1,2*} = 4,1 Hz H-1 | d, 1 H, *J*_{*1",2"*} = 8,1 Hz H-1" | d, 1 H, *J*_{*3',4'*} = 8,1 Hz H-3' |
| 5,12 | 4,59 | 4,26 |

| ^{**12**}**C-NMR** (100,67 MHz, D₂O) | | | | | | |
|---|---|---|---|---|---|---|
| Gruppe | C-1 | C-2 | C-3 | C-4 | C-5 | C-6 |
| Gal*p*-(1→4) | 103,25 | 70,95 | 72,93 | 68,95 | 75,64 | 61,42 |
| Glc*p*-(1→4) | 98,42 | 71,47 | 71,35 | 78,71 | 72,07 | 60,31 |
| β-Fru*f* | 63,02 | 102,10 | 75,74 | 74,92 | 79,27 | 68,36 |

### Beispiel 3: Herstellung eines β-1,6-Galactosyl-Isomalt angereicherten Gemisches

0,2 mol/l Lactose wird mit β-Galactosidase aus *Aspergillus oryzae* (Enzymmenge: 50 Units, Typ: G 5160, Hersteller: SIGMA Aldrich) und mit 1,0 mol/l Isomalt für 2 Stunden bei 30°C bei pH 4,5 (ohne Puffer) umgesetzt. Es wird ein Galactosyl-Isomalt-haltiges Gemisch erhalten, welches hinsichtlich seiner DP 3-Komponenten eine Anreicherung an β-1,6-Galactosyl-Isomalt (Formel (3)) aufweist.

Die Tabelle in Beispiel 4 zeigt die Produktzusammensetzung (DP-Verteilung) der erhaltenen Galactosyl-Isomalt-haltigen Gemische.

### Beispiel 4: Chromatographische Auftrennung der Galactosyl-Isomalt Gemische (siehe Beispiel 1 bis 3)

Die DP-Verteilung der Reaktionsprodukte aus den Beispielen 1 bis 3 wurde mittels Gelpermeationschromatographie an Fractogel® HW 40 S bei 50°C und vollentsalztem Wasser als Eluenten ermittelt, wobei Raftilose®ST als Vergleichssubstanz eingesetzt wurde.

| Umsetzung von Isomalt und Lactose mit | % -Fläche | | | |
|---|---|---|---|---|
| | DP 1 | DP 2 | DP 3 | DP 4 |
| β-1,6-Galactosidase aus *Aspergillus oryzae* | 13,9 | 83,9 | 2,2 | - |
| β-1,4-Galactosidase aus *Bacillus circulans* | 5,8 5,8 | 80,6 80,6 | 11,8 11,8 | 1,8 1,8 |
| β-1,3-Galactosidase aus Stierhoden | 8,7 8,7 | 85,0 85,0 | 6,3 6,3 | |

Unter den gewählten Inkubationsbedingungen wurden in allen Fällen Oligosaccharide (DP 3, DP 4) gebildet.

### Beispiel 5: Galactosyl-Isomalte DP 3 / DP 4-Hydrolyse

Hydrolysen der Komponenten aus den Bereichen DP 3 und DP 4 erhalten aus den Beispielen 1 bis 3 wurden in 0,5%igen Lösungen, 1 mol/l HCl bei 95°C für 3 Stunden durchgeführt. Die Analysen erfolgten mittels HPAEC unter folgenden Bedingungen:
- Trennsäulen:: 2 x Carbopac PA 1, je 4 mm x 250 mm, Vorsäule PA 1, 4 mm x 50 mm, Anionenaustauscher, Fa. Dionex.
- Eluent:: 0,16 mol/l NaOH mit 0,5 mol/l Na-Acetat-Gradienten 1 bis 54 % in
- Elutionszeit:: 30 min
- Flussrate:: 1 ml/min
- Detektion:: Gepulster amperometrischer Detektor (Goldelektrode),
- Potentiale E und Pulszeiten t:: E₁ = 0,05 V und t₁ = 400 ms,
E₂ = 0,6 V und t₂ = 300 ms,
E₃ = 0,60 V und t₃ = 240 ms

| Gehaltsanalyse HPAEC (Angaben in mg/l): | | | | | | |
|---|---|---|---|---|---|---|
| Umsetzung mit β-Galactosidase | Sorbit | Mannit | 1,6-GPS | 1,1-GPM | Glucose + Galactose | Sorbit + Mannit / Glucose+ Galactose |
| β-1,6 aus *Asp. oryzae* DP 3 | 200,2 | 174,0 | 32,3 | 29,0 | 1196,8 | 1 / 3,2 |
| β-1,4 aus *Bac. circulans* DP 4 | 489,0 | 319,6 | 60,9 | 48,3 | 3378,8 | 1 / 4,2 |
| β-1,4 aus *Bac. circulans* DP 3 | 840,5 | 496,3 | 104,7 | 75,5 | 3377,5 | 1 / 2,8 |
| β-1,3 aus Stierhoden DP 3 | 347,5 | 262,5 | 52,5 | 49,8 | 3656,4 | 1 / 3,0 |

Die Hydrolyse der isolierten DP 3 / DP 4-Produkte ergibt an Monosacchariden Sorbit, Mannit, Glucose und Galactose und daneben noch geringe Anteile an den Spaltprodukten 1,6-GPS und 1,1-GPM. Die Verhältnisse an Sorbit mit Mannit zu Glucose mit Galactose weisen einevomehmliche Bildung von Galactosyl-Isomalt auf.

### Beispiel 6: Umsetzungen mit thermophilen β-Galactosidasen

Die aus den drei thermophilen Mikroorganismen

| | |
|---|---|
| *Bacillus stearothermophilus* KVE 39 | (BgaB) |
| *Thermus brockianus* ITI360 | (BgaT) |
| *Thermus thermophilus* TH125 | (BgIT) |

abgeleiteten β-Galactosidase codierenden Gene wurden in Expressionsvektoren in *E. coli* Wirtsstämme (β-Galactosidase negativ) einkloniert und anschließend vermehrt. Die nach Zellaufschluss erhaltenen Rohextrakte von *E*. *coli* wurden mittels Hitzepräzipitation partiell gereinigt und für nachfolgende Inkubationen eingesetzt.

Ansätze: Zu 1,5 mol/l Lactose-Lösung sowie zu 1,5 mol/l Lösung bestehend aus 0,2 Teilen Lactose und jeweils 0,8 Teilen Isomaltulose, 1,6-GPS oder 1,1-GPM in 50 mmol/l Na-Phosphat-Puffer, pH 6,5, mit 0,02 % Na-Azid (NaN₃) werden 1 Unit β-Galactosidase pro 0,5 ml Ansatz gegeben und unter Schütteln bei 37°C für 16 Stunden inkubiert; Reaktionsstop: 95°C, 15 Min; HPAEC-Analysen: siehe Beispiel 4.

| **β-Galactosidase BgaT** | % Fläche | | | |
|---|---|---|---|---|
| Donor/Akzeptor | DP 1 | DP 2 | DP 3 | DP 4 |
| Lactose | 35,0 | 61,6 | 3,4 | - |
| Lactose/1,6-GPS | 10,2 | 90,3 | 2,7 | - |
| Lactose/1,1-GPM | 6,5 | 90,3 | 2,7 | 0,5 |
| Lactose/Isomaltulose | 5,9 | 91,8 | 1,5 | 0,7 |

| **β-Galactosidase BglT** | % Fläche | | | |
|---|---|---|---|---|
| Donor/Akzeptor | DP 1 | DP 2 | DP 3 | DP 4 |
| Lactose | 35,1 | 28,3 | 27,9 | 8,7 |
| Lactose/1,6-GPS | 8,5 | 80,3 | 9,1 | 2,1 |
| Lactose/1,1-GPM | 9,3 | 79,9 | 10,8 | - |
| Lactose/Isomaltulose | 8,2 | 79,1 | 11,3 | 0,8 |

| **β-Galactosidase BgaB (Wildtyp)** | % Fläche | | | |
|---|---|---|---|---|
| Donor/Akzeptor | DP1 1 | DP 2 | DP 3 | DP 4 |
| Lactose | 57,7 | 37,6 | 4,7 | - |
| Läctose/1,6-GPS | 15,0 | 85,0 | - | - |
| Lactose/1,1-GPM | 19,0 | 81,0 | - | - |
| Lactose/Isomaltulose | 12,4 | 85,4 | 2,2 | - |

Die β-Galactosidasen BgaT und BglT bilden in Gegenwart der beiden Isomalt-Komponenten 1,6-GPS / 1,1-GPM und Lactose als Donor-Molekül Oligosaccharide. Es zeigt sich, dass sowohl 1,6-GPS als auch 1,1-GPM als Akzeptoren fungieren. Umsetzungen mit Isomaltulose und Lactose ergeben bei den eingesetzten β-Galactosidasen in allen Fällen Trisaccharide und teilweise auch Tetrasaccharide. Im Falle der β-Galactosidase BgaB (Wildtyp) erfolgt keine Bildung von Tri-/Tetrasacchariden (DP 3, DP 4).

### Beispiel 7: Umsetzungen mit thermophilen β-Galactosidasen aus Mutanten von Bacillus stearothermophilus KVE 39 (BgaB)

Die β-Galactosidasen aus BgaB Mutanten pGP 17 und pGP 23 sind analog Beispiel 5 hergestellt und getestet worden.

| **β-Galactosidase pGP 17** | % Fläche | | | |
|---|---|---|---|---|
| Donor/Akzeptor | DP 1 | DP 2 | DP 3 | DP 4 |
| Lactose | 20.,7 | 70,0 | 9,3 | - |
| Lactose/1,6-GPS | 10,2 | 79,7 | 10,1 | - |
| Lactose/1,1-GPM | 5,4 | 80,0 | 14,8 | - |
| Lactose/Isomaltulose | 10,3 | 82,7 | 7,0 | - |

| **β-Galactosidase pGP 23** | % Fläche | | | |
|---|---|---|---|---|
| Donor/Akzeptor | DP 1 | DP 2 | DP 3 | DP 4 |
| Lactose | 26,3 | 58,6 | 15,1 | - |
| Lactose/1,6-GPS | 10,1 | 83,3 | 6,6 | - |
| Lactose/1,1-GPM | 14,1 | 79,5 | 6,4 | - |
| Lactose/Isomaltulose | 12,5 | 81,1 | 6,4 | - |

Es zeigt sich, dass die Mutation des β-Galactosidase Gens von BgaB zu Enzymen führt, die im Gegensatz zum Wildtyp in der Lage sind, Galactosyl-Isomalt beziehungsweise Galactosyl-Isomaltulose zu synthetisieren.

### Beispiel 8: Stabilität der Galactosyl-Isomalte in Magen und Dünndarm

### a) Stabilität im Magen

Die Stabilität einer Substanz in der Magen-Passage kann durch Bestimmung der Hydrolyserate bei pH 2,0 simuliert werden. Als Kontrolle werden Saccharose sowie 1-Kestose verwendet.

Jeweils eine 1%ige Lösung der Trisaccharide (DP 3) aus Beispiel 4 wird mit 10 mmol/l Salzsäure, pH 2,0, bei 37°C für 3 Stunden inkubiert. Aus dem Reaktionsansatz werden nach jeweils 60, 120 und 180 Minuten Proben entnommen, die mittels basischer Anionenaustausch-Chromatographie, HPAEC, analysiert werden.

| Hydrolyserate [%] | Inkubationszeit (min) | | | |
|---|---|---|---|---|
| | 0 | 60 | 120 | 180 |
| Saccharose | 0 | 2 | 5 | 8 |
| 1-Kestose | 0 | 11 | 25 | 36 |
| β-1,4-Galactosyl-Isomalt | 0 | 0 | 0 | <1 |
| β-1,3-Galactosyl-Isomalt | 0 | 0 | 0 | <1 |
| β-1,6-Galactosyl-Isomalt | 0 | 0 | 0 | <1 |
| Galactosyl-Isomaltulose | 0 | 0 | 0 | 2 |

Die isolierten Trisaccharide (DP 3) aus den β-1,3-, β-1,4- und β-1,6-angereicherten Galactosyl-Isomalt-haltigen Gemischen beziehungsweise das isolierte Galactosyl-Isomaltulose-haltige Gemisch (BglT) werden bzw. wird unter der. Magenpassage vergleichbaren Bedingungen im Vergleich zu Saccharose mit 8 % Spaltung und 1-Kestose mit 36 % Spaltung nicht hydrolysiert.

### b) Stabilität gegenüber Pankreas-Enzymen

Das Pankreas-Sekret enthält eine große Anzahl von Hydrolasen, unter anderem auch Kohlenhydrat-spaltende Enzyme wie α-Amylase, welche α-1,4-Glucane (Stärke, Glykogen) bevorzugt zu Maltose und Maltooligosacchariden spaltet. Die Prüfung der Stabilität von Sacchariden gegenüber Pankreas-Enzymen wird wie folgt durchgeführt.

Pro Ansatz werden je 3,0 ml einer Kohlenhydratlösung (Lösung 2 bis Lösung 6) mit je 0,1 ml der Enzymlösung (Lösung 7) vermischt:
- Lösung 1:: 20 mmol/l Na-Phosphat-Puffer mit pH 7,0 und mit 6 mmol/l NaCl
- Lösung 2:: 1%ige Lösung erfindungsgemäßes β-1,4-Galactosyl-Isomalt-Gemisch, hergestellt nach Beispiel 2 und Beispiel 4 in Lösung 1
- Lösung 3:: 1%ige Lösung erfindungsgemäßes β-1,3-Galactosyl-Isomalt-Gemisch, hergestellt nach Beispiel 1 und Beispiel 4 in Lösung 1
- Lösung 4:: 1%ige Lösung erfindungsgemäßes β-1,6-Galactosyl-Isomalt-Gemisch, hergestellt nach Beispiel 3 und Beispiel 4 in Lösung 1
- Lösung 5:: 1%ige Lösung erfindungsgemäßes Galactosyl-Isomaltulose-haltiges Gemisch, hergestellt nach Beispiel 6 (BgIT) und Isolierung vom Trisaccharid analog Beispiel 4 in Lösung 1
- Lösung 6:: 1%ige Stärkelösung (lösliche Stärke nach Zulkowski) in Lösung 1
- Lösung 7:: 0,2% Pankreatin-Enzym (Sigma) gelöst in Lösung 1

Nach 210 Minuten Inkubation im Thermomixer (Intervall-Schütteln) bei 37°C wird die Reaktion durch Erhitzen für 15 Minuten auf 95°C beendet und die Proben mittels HPAEC analysiert. Die stärkehaltige Probe (Lösung 6 + Lösung 7) wird vor der HPAEC-Analyse durch 3-stündiges Erhitzen in 1 mol/l Salzsäure bei 95°C vollständig hydrolysiert und die daraus entstehende Glucose ermittelt, um den Stärkegehalt der Probe zu errechnen.

| Substanz | Abbaurate [%] |
|---|---|
| β-1,4-Galactosyl-Isomalt | <1 |
| β-1,3-Galactosyl-Isomalt | <1 |
| β-1,6-Galactosyl-Isomalt | <1 |
| Galactosyl-Isomaltulose | <1 |
| Lösliche Stärke | 85 |

Die isolierten Trisaccharide (DP 3) aus den β-1,3-, β-1,4- und β-1,6-angereicherten Galactosyl-Isomalt-haltigen Gemischen sowie das Trisaccharid Galactosyl-Isomaltulose werden durch die eingesetzten Pankreas-Enzyme nicht gespalten.

### c) Stabilität gegenüber Dünndarm-α-Glucosidasen

Die im Dünndarm vorhandenen Mucosa-ständigen Enzym-Komplexe Saccharase/Isomaltase und Glucoamylase/Maltase sorgen *in vivo* dafür, dass in den Dünndarm gelangte Disaccharide wie Maltose und Saccharose und zum Teil auch Maltooligosaccharide zu Monosacchariden gespalten werden und als solche über die Darmwand in den Blutkreislauf aufgenommen werden. Die Prüfung der Stabilität der Galactosyl-Isomalte (DP 3) sowie Galactosyl-Isomaltulose gegenüber diesen Enzymen wurde wie folgt durchgeführt.

Die Enzym-Komplexe Saccharase/Isomaltase (SI-Komplex) und Glucoamylase/Maltase (GM-Komplex) werden aus Schweine Dünndarm nach der Methode von H: Heymann (Dissertation, Hannover, 1991) isoliert.
- Lösung 1:: 0,1 mol/l Triethanolamin (TEA)-Puffer mit pH 7,0
- Lösung 2:: 1 %ige Lösung DP3-Bereich von β-1,4-Galactosyl-Isomalt-Gemisch hergestellt nach Beispiel 4 in Lösung 1
- Lösung 3:: 1%ige Lösung DP3-Bereich von β-1,3-Galactosyl-Isomalt-Gemisch nach Beispiel 4 in Lösung 1
- Lösung 4:: 1%ige Lösung DP3-Bereich von β-1,6-Galactosyl-Isomalt-Gemisch hergestellt nach Beispiel 4 in Lösung 1
- Lösung 5:: 1%ige Lösung DP3-Bereich von Galactosyl-Isomaltulose-haltigem Gemisch hergestellt nach Beispiel 6 (BgIT) und
Isolierung von Trisaccharid analog Beispiel 4 in Lösung 1
- Lösung 6:: 1%ige Lösung von Maltose in Lösung 1
- Lösung 7:: 1%ige Lösung von Saccharose in Lösung 1
- Lösung 8:: 1%ige Lösung von Isomalt in Lösung 1
- Lösung 9:: 1 %ige Lösung von Isomaltulose in Lösung 1
- Lösung 10:: Saccharase/lsomaltase-Enzymkomplex in Lösung 1
- Lösung 11:: Glucoamylase/Maltase-Enzymkomplex in Lösung 1

i) Zu je 1,0 ml einer auf 37°C temperierten Kohlenhydratlösung (Lösung 2 bis Lösung 8) wurden jeweils 0,7 Units des Enzymkomplexes Saccharase/Isomaltase (Lösung 10) beziehungsweise Glucoamylase/Maltase (Lösung 11) zugegeben, gemischt und bei 37°C inkubiert.
ii) Für Inkubationen unter schärferen Bedingungen wurden 16 Units des Enzymkomplexes Saccharasellsomaltase beziehungsweise 13 Units des Enzymkomplexes Glucoamylase/Maltase eingesetzt. Die Reaktion wurde nach 2 Stunden durch Erhitzen für 15 Minuten auf 95°C gestoppt. Die dabei gebildeten Monosaccharide sowie die nicht abgebauten Saccharide der jeweiligen Ansätze wurden mittels HPAEC quantitativ bestimmt.

| Hydrolyserate [%] | 120 Minuten Inkubation mit: | | | |
|---|---|---|---|---|
| | Saccharase/ Isomaltase | | Glucoamylase/ Maltase | |
| | Standard | verschärft | Standard | verschärft |
| Saccharose (Lösung 7) | 85,6 | 91,0 | - | - |
| Maltose (Lösung 6) | - | - | 92,0 | 96,6 |
| Isomalt (Lösung 8) | 3,5 | 52,4 | 0 | 12,8 |
| Isomaltulose (Lösung 9) | 18,0 | 79,0 | 3,1 | 39,9 |
| β-1,4-Galactosyl-Isomalt | 0 | 7,5 | 0 | 0 |
| β-1,3-Galactosyl-Isomalt | 0 | 3,2 | 0 | 0 |
| β-1,6-Galactosyl-Isomalt | 6,9 | 23,7 | 0 | 0 |
| Galactosyl-Isomaltulose | 0 | 15,0 | 0 | 2 |

Unter den gewählten Standard-Bedingungen kommt es zu einer fast vollständigen Hydrolyse von Saccharose und Maltose durch den Saccharase/Isomaltase-Enzymkomplex sowie von Maltose durch den Glucoamylase/Maltase-Enzymkomplex. Isomalt wird unter diesen Bedingungen teilweise gespalten. Die Galactosyl-Isomalte bis β-1,6-Galactosyl-Isomalt (6,9%) werden überhaupt nicht gespalten.

Unter den verschärften Bedingungen erfolgt eine signifikante Spaltung bei Isomalt von 52 % für Saccharase/Isomaltase und 12,8 % für Glucoamylase/Maltase. Galactosyl-Isomalt und Galactosyl-Isomaltulose werden unter diesen Bedingungen nahezu nicht gespalten.

### d) Stabilität gegenüber Lactase

Das im Dünndarm vorhandene Mucosa-ständige Enzym Lactase sorgt *in vivo* dafür, dass in den Dünndarm gelangte Lactose gespalten wird und die entstehenden Monosaccharide über die Darmwand in den Blutkreislauf aufgenommen werden.

Die Prüfung der Stabilität der Galactosyl-Isomalte (DP 3) sowie Galactosyl-Isomaltulose gegenüber diesem Enzym werden wie folgt durchgeführt:

Das Enzym Lactase wird nach einer modifizierten Methode von Wallenfels, K. und Fischer, J. (Z. Physiologische Chemie 321 (1960) 223) isoliert.
- Lösungen 1-5:: siehe Beispiel 8c)
- Lösung 6:: 1%ige Lösung von Lactose in Lösung 1
- Lösung 7:: 1 %ige Lösung von Isomalt in Lösung 1
- Lösung 8:: Lactase in Lösung 1

Zu je 1,0 ml einer auf 37°C temperierten Kohlenhydratlösung (Lösung 2 bis 7) werden jeweils 0,9 Units des Enzyms Lactase zugegeben, gemischt und bei 37°C inkubiert. Die Reaktion wird nach 6 Stunden durch Erhitzen für 15 Minuten auf 95°C gestoppt. Die dabei gebildeten Monosaccharide sowie die nicht abgebauten Saccharide der jeweiligen Ansätze werden mittels HPAEC quantitativ bestimmt.

| Hydrolyserate [%] | Inkubation mit Lactase |
|---|---|
| Lactose | 95,6 |
| β-1,4-Galactosyl-lsomalt | 13,4 |
| β-1,3-Galactosyl-Isomalt | 4,8 |
| β-1,6-Galactosyl-lsomalt | 9,3 |
| Galactosyl-Isomaltulose | 7,0 |

Unter den gewählten Bedingungen kommt es zu einer fast vollständigen Hydrolyse der Lactose. Die Galactosyl-Isomalte sowie Galactosyl-Isomaltulose werden im Vergleich zu Lactose nur geringfügig gespalten.

### Beispiel 9: Bereitstellung von β-Galactosidasen

Für das erfindungsgemäße Verfahren besonders geeignet sind bakterielle β-Galactosidasen, die der Aktivität der β-Galactosidase aus Stierhoden ähneln. Die folgende Tabelle stellt wesentliche Eigenschaften der eingesetzten Enzyme und ihre Herkunft dar.

Die Aktivität wurde jeweils bei 65°C mit pNp-β-Gal bestimmt. Es wird allgemein ein Kalium-Phosphat-Puffer pH 6,5 für die Messungen verwendet, die Enzympräparate befinden sich in diesem Puffer. Die stabile Lagerung erfolgt bei 4°C.

| Enzym | Herkunft des Gens | Aktivität | | ml | MW | Reinheit |
|---|---|---|---|---|---|---|
| | | Units/ml | Units/mg | | | |
| BgaB | *Bacillus stearothermophilus* KVE39 | 62,6 | 44,7 | 2 | 78 | ~ 60% |
| BgaT | *Thermus brockianus* ITI360 | 6,4 | 4,6 | 1,5 | 47 | ~ 70% |
| BgIT | *Thermus thermophilus* TH125 | 6 | 12 | 2 | 73 | ~90% |

Das Temperaturoptimum ist bei den beiden Enzymen aus Thermus ssp. deutlich höher (~ 90°C).

Das Stibstratspektrum und die Regioselektivität der Enzyme wurden untersucht. Dabei zeigten sich ausgeprägte Unterschiede für die Enzyme.

Verwendet wurden diverse para-Nitrophenylglykoside bei 65°C (Substrate hier ausreichend stabil). In der folgenden Tabelle wird die Aktivität in % zu pNp-β-Gal angegeben.

| pNp-Glykosid | *Bacillus* BgaB | *Thermus* BgaT | *Thermus* BglT |
|---|---|---|---|
| -β-Gal | 100 | 100 | 100 |
| -β-Glc | 0,1 | 3 | 56,2 |
| -β-D-Fuc | 29 | 86,7 | 104 |
| -α-L-Ara | 43 | 34,3 | 6,4 |
| -α-L-Fuc | 0,5 | 0,5 | 0,2 |
| -α-Man | 0 | | |
| -α-Gal | 0,5 | | |
| -α-Glc | 0,4 | | |

Die vorgenannten bakteriellen Enzyme, deren Expressionsvektoren und die Enzyme kodierenden Gene sowie die Gene oder Expressionsvektoren aufweisende Zellen sind ebenfalls Gegenstand dieser Erfindung.

### β-Galaktosidase-Präparate:

| *A) BgaB aus Bacillus stearothermophilus* | |
|---|---|
| *E.coli* Stamm: | pHWG509 in *E. coli* JM109 |
| Anreicherung: | Hitzepräzipitation |
| Aktivität: | pNp-βGal (65°C):48 U/ml |
| Protein: | 1,15 mg/ml |

| *B) BgaT aus Thermus brockianus ITI360* | |
|---|---|
| *E.coli* Stamm: | pOF3823 in *E. coli* JM109 |
| Anreicherung: | Hitzepräzipitation |
| Aktivität: | pNp-βGal (65°C): 17 U/ml |
| Protein: | 1,9 mg/ml |

| *C) BglT aus Thermus thermophilus TH125* | |
|---|---|
| *E.coli* Stamm: | pHWG475 in *E. coli* JM109 |
| Anreicherung: | Hitzepräzipitation |
| Aktivität: | pNp-βGal (65°C): 19 U/ml |
| Protein: | 0,9 mg/ml |

β-Galactosidase-Präparate werden in 0,1 mol/l Kaliumphosphatpuffer pH 6,5, bei 4°C aufbewahrt.

### Beispiel 10: Herstellung und Charakterisierung von Enzymrohextrakten vom BgaB Wildtyp (pHWG 509) und BgaB Mutanten (pGP17 und 23)

Alle Extrakte wurden aus 100 ml Übernachtkulturen (Ampicillin, Rhamnose 0,2 %) hergestellt. Zunächst wurde 10 min für 4°C bei 6.000 U/min zentrifugiert, das erhaltene Pellet wird in 10 mmol/l Kalium-Phosphat-Puffer, pH 6,5, gewaschen. Es schließt sich eine Zentrifugation für 10 min bei 4°C und 6.000 U/min an. Das Pellet wird in Kalium-Phosphat-Puffer 10 mmol/l, pH 6,5, aufgelöst, die Zellen mittels einer French-Presse aufgeschlossen und bei Raumtemperatur eine Zentrifugation bei 13.000 U/min für 30 min durchgeführt. Der Überstand (Rohextrakt) wird 15 min bei 60°C denaturiert, bei Raumtemperatur 30 min zentrifugiert und mit 0,02 Gew.-% Na-Azid gelagert. Im folgenden ist eine Charakterisierung der Enzyme angegeben.

Zur Bestimmung der Aktivität werden 2 µl Rohextrakt mit 398 µl Kalium-Phosphat-Puffer 0,1 mol/l, pH 6,5 vermischt. Es wird 35 min bei 37°C vorinkubiert und 100 µl p-Nitrophenyl-β-D-Galactopyranosid (4 mg/ml) in Kalium-Phosphat-Puffer (0,1 mol/l, pH 6,5) hinzugegeben. Die Aktivität wird für 2 min gemessen. Die Reaktion wird mit 1 ml Natriumborat 0,4 mol/l, pH 4,0 gestoppt und die Quantität von pNP bei 405 nm gemessen.

| **Kinetische Parameter für *p*NPβGal** | | | |
|---|---|---|---|
| | pNPβGal Aktivität* (µmol/min/ml) | Proteinmenge (mg) | Spezifische Aktivität (µmol/min/mg) |
| pHWG509 (Wildtyp) | 7,1 | 4,35 | 1,63 |
| pGP17 | 8,0 | 6,90 | 1,16 |
| pGP23 | 5,0 | 7,45 | 0,67 |

| | (ρNPβGal) | | |
|---|---|---|---|
| | VMₐₚₚ (µmol/min/mg) | Kmₐₚₚ (mmolll) | VMₐₚₚ/Kmₐₚₚ |
| pHWG509 | 3,3 | 1 | 3,30 |
| pGP17 | 13,2 | 20 | 0,66 |
| pGP23 | 5,8 | 19 | 0,30 |

| **Kinetische Parameter für Oligosaccharide** (Werte erhalten mit einer 5 ml Übernachtkultur) | | | |
|---|---|---|---|
| | Vmₐₚₚ (U.E./mg) | Kmₐₚₚ (mmol/l) | Vmₐₚₚ/Kmₐₚₚ |
| | **Disaccharide (Lactose)** | | |
| pHWG509 | 1,5 | 9 | 0,25 |
| pGP17 | n.b. | 90 | n.b. |
| pGP23 | 1,5 | 100 | 0,015 |

| | **Trisaccharide (Lactosaccharose)** | | |
|---|---|---|---|
| pHWG509 | 0,84 | 5 | 0,168 |
| pGP17 | n.b. | 180 | n.b. |
| pGP23 | 0,36 | 200 | 0,0018 |

Auch die hergestellten mutanten Gene und davon codierten β-Galactosidasen sowie diese enthaltende Vektoren und Wirtszellen sind Gegenstand der Erfindung.

### Beispiel 11: Fermentation von β-Galactosyl-Isomalten in Human-Fäzes

Die Inkubation von Kohlenhydraten mit Human-Fäzes erlaubt Aussagen über die Geschwindigkeit der Verstoffwechselung durch die Bakterienpopulation sowie zur Bildung der kurzkettigen Fettsäure Buttersäure.

Zur Untersuchung der Fermentierbarkeit in einem in vitro-Fermentationsexperiment werden neben β-Galactosyl-Isomalten zum Vergleich auch Raftilose® P95 (Fructoooligosaccharide, DP4-5), Galactosyl-Lactose (Oligomate DP 3) und Lactosucrose als Kohlenhydrate ähnlicher Struktur und Polymerisationsgrad eingesetzt.

Für die in vitro-Fermentationsexperimente wird ein anaerobes Medium in folgender Zusammensetzung eingesetzt:

| *Medium 1:* | |
|---|---|
| Trypton | 1,5 g |
| Hefe-Extrakt | 1,5 g |
| KH₂PO₄ | 0,24 g |
| Na₂HPO₄ | 0,24 g |
| (NH₄)₂SO₄ | 1,24 g |
| NaCl | 0,48 g |
| MgSO₄ x 7 H₂O | 0,10 g |
| CaCl₂ x 2 H₂O | 0,06 g |
| FeSO₄ x 7 H₂O | 2,0 mg |
| Resazurin | 1,0 mg |
| Cystein/HCl | 0,5 g |
| Vitaminlösung (nach DSM 141) | 0,5 g |
| Spurenelementelösung (nach DSM 141) | 9,0 ml |
| NaHCO₃ H₂O dest. *ad* 1000 ml, pH 0,7 | 2,0 g |

### Kultivierung von Darmbakterien auf den zu testenden Oligosacchariden:

9 ml des vorstehend beschriebenen anaeroben Mediums 1 wird mit 0,5 % (w/v) der zu testenden Galactosyl-Isomalte (hergestellt nach Beispiel 1-3) und den entsprechenden Kontrollen Lactosucrose und Galactosyl-Lactose versetzt und anschließend mit 1 ml einer 10%igen Fäzes-Suspension (Mischfäzes zweier Probanden) in anaeroben 50 mmol/l Phosphatpuffer, pH 7,0, dem zuvor 0,5 g/L Cystein/HCl als Reduktionsmittel zugesetzt wird, beimpft. Anschließend werden "Hungate"-Röhrchen für maximal 28 Stunden unter Schütteln bei 37°C inkubiert. Zu unterschiedlichen Zeitpunkten werden Proben daraus entnommen und diese auf den Anteil restlicher Oligosaccharide, kurzkettige Fettsäuren, Milchsäure und pH-Wert untersucht.

### Ergebnis:

Die Fructooligosaccharide (Raftilose®P95), Galactosyl-Lactose und Lactosucrose sind bereits nach 7 Stunden nahezu vollkommen verstoffwechselt. Die drei verschiedenen β-Galactosyl-Isomalte, hergestellt nach den Beispielen 1 bis 3, werden hingegen signifikant langsamer fermentiert, so dass die Substanzen nach Eintritt in den Dickdarm weiter im distalen Bereich positive Effekte hervorrufen.

| Abbaurate [%] | Inkubationszeit [h] | | | | Butyrat- Gehalt (Endprobe) |
|---|---|---|---|---|---|
| | 7 | 14 | 22 | 28 | |
| Raftilose®P95 | 100 | - | - | - | 2,5 mmol/l |
| Galactosyl-Lactose (Oligomate DP 3) | 96,6 | 99,5 | 100 | - | 6,3 mmol/l |
| Lactosucrose | 98,9 | 100 | - | - | 6,5 mmo/l |
| β-1,3-Galactosyl-Isomalt | 62 | ***n.d.*** | 93,6 | 97 | 13,3 mmol/l |
| β-1,4-Galactosyl-Isomalt | 61 | ***n.d.*** | 92,7 | 96 | 17,0 mmol/l |
| β-1,6-Galactosyl-Isomalt | 64,2 | n.d. | 96,1 | 99,2 | 15,3 mmol/l |

Der Gehalt an gebildetem Butyrat am Ende der Fermentationen liegt für die Kontrollen Lactosucrose beziehungsweise Galactosyl-Lactose bei ca. 6 mmol/l, bei Fermentation von Raftilose entsteht noch weniger Butyrat (2,5 mmol/l). Werden hingegen β-1,3-, β-1,4- oder β-1,6-Galactosyl-Isomalte eingesetzt, erfolgt eine Verstoffwechselung zu deutlich höheren Konzentrationen an Butyrat (13,3 bis 17 mmol/l).

### Beispiel 12: Fermentation von β-1,3-Galactosyl-Isomalt durch humane Bifidobakterien

Zur Untersuchung präbiotischer und bifidogener Eigenschaften von Galactosyl-Isomalt wurde das Wachstum diverser humaner Bifidobakterien auf diesem Substrat *in vitro* untersucht. Dazu wurde ein anaerobes Medium folgender Zusammensetzung eingesetzt:

| *Medium 2:* | |
|---|---|
| Caseinpepton | 10,0 g |
| Fleisch-Extrakt | 5,0 g |
| Hefe-Extrakt | 5,0 g |
| Na₂HPO₄ | 1,44 g |
| NaH₂PO₄ | 0,24 g |
| K₂HPO₄ | 6,0 g |
| Tween 80 | 1,0 g |
| Spurenelementelösung nach DSM 141 | 9,0 ml |
| Vitaminlösung nach DSM 141 | 1,0 ml |
| Resazurin | 1,0 mg |
| Cystein/HCl | 0,5 g |
| Galactosyl-Isomalt H₂O dest. *ad* 1000 ml, pH 7,0 | 1,0 g |

22 Stämme humaner Bifidobakterien wurden zunächst in "Hungate"-Röhrchen auf dem oben genannten Medium 1 vorkultiviert, dessen Kohlenhydratquelle jedoch zuvor durch Glucose (10 g/l) ersetzt worden war. Im Anschluss daran wurden die Stämme auf Medium 2 überimpft, für 48 Stunden bebrütet und danach die optische Dichte (E₅₇₈), der pH-Wert, die Bildung von Acetat und Lactat sowie die Abnahme der β-1,3-Galactosyl-Isomaltkonzentration bestimmt.

### Ergebnisse:

Hinsichtlich der Verstoffwechselung von Galactosyl-Isomalt werden verschiedene Abbauwege unterschieden. Einige Stämme spalten mit einer β-Galactosidase Galactose vom Substrat ab und verstoffinrechseln dieses Monosaccharid (+).

Andere Stämme spalten neben Galactose zusätzlich Glucose vom entstandenen Isomalt ab, verstoffwechseln beide Monosaccharide, während dieses für das entstandene Mannit oder Sorbit nicht gilt (++). Die dritte Gruppe ist in der Lage, Galactosyl-Isomalt als Substrat zu nutzen, auch Mannit und Sorbit werden mit verstoffwechselt (+++).

Aus der nachfolgenden Tabelle wird ersichtlich, inwieweit die getesteten Bifidobakterien Galactosyl-Isomalt als Substrat nutzen können:

| Stamm der Gattung *Bifidobacterium* | Wachstum auf β-1,3-Galactosyl-Isomalt |
|---|---|
| *B. adolescentis* DSM 20083 | +++ |
| *B. adolescentis* DSM 20086 | +++ |
| *B. adolescentis* DSM 20087 | +++ |
| *B. angulatum* DSM 20098 | ++ |
| *B. angulatum* DSM 20225 | ++ |
| *B. bifidum* DSM 20082 | + |
| *B. bifidum* DSM 20215 | + |
| *B. bifidum* DSM 20239 | + |
| *B. bifidum* DSM 20456 | - |
| *B. breve* DSM 20091 | + |
| *B. breve* DSM 20213 | + |
| *B. catenulatum* DSM 20103 | ++ |
| *B. catenulatum* DSM 20224 | ++ |
| *B. gallicum* DSM 20093 | - |
| *B. infantis* DSM 20088 | ++ |
| *B. infantis* DSM 20090 | ++ |
| *B. infantis* DSM 20218 | - |
| *B. infantis* DSM 20223 | +++ |
| *B. longum* DSM 20219 | +++ |
| *B. longum* DSM 20097 | ++ |
| *B. pseudocatenulatum* DSM 20438 | ++ |
| *B. pseudocatenulatum* DSM 20439 | ++ |

| | |
|---|---|
| -: kein Abbau, +: Abspaltung und Verstoffwechselung von Galactose aus β-1,3-Galactosyl-Isomalt, ++: Abspaltung und Verstoffwechselung von Galactose und Glucose aus β-1,3-Glactosyl-Isomalt, +++: kompletter Abbau von β-1,3-Galactosyl-Isomalt. | |

### Beispiel 13: Hydrierung eines Galactosyl-Isomaltulose-haltigen Gemisches

500 ml einer durch die Umsetzung von Isomaltulose (analog Beispiel 6) mit β-Galactosidase BglT erhaltenen Galactosyl-Isomaltulose-haltigen Reaktionslösung, verdünnt auf 30% TS, wurde durch Zugabe von 1 N NaOH unter Rühren auf einen pH-Wert von 7,8 eingestellt. Die Hydrierung erfolgte mittels eines Nickel-Katalysators (200 g Feuchtmasse) in Gegenwart von Wasserstoff (150 bar) bei 70°C unter Rühren.

Proben wurden nach 0, 1, 2, 3 und 4 Stunden genommen und auf ihren Gehalt an Galactosyl-Isomaltulose sowie Galactosyl-Isomalt überprüft. Die Hydrierung wurde nach quantitativer Umsetzung der freien Isomaltulose zu Galactosyl-Isomalt beendet.

| g/l | Hydrierverlauf [Stunden] | | | | |
|---|---|---|---|---|---|
| | 0 | 1 | 2 | 3 | 4 |
| Galactosyl-Isomaltulose | 0 | 14,7 | 6,9 | 2,4 | 0 |
| Galactosyl-Isomalte | 26,8 | 11,5 | 19,3 | 23,6 | 25,3 |
| Summe | 26,8 | 26,2 | 26,2 | 26,0 | 25,3 |

Nach 4-stündiger Reaktionszeit war die in der Galactosyl-Isomaltulose-haltigen Reaktionslösung enthaltene Galactosyl-Isomaltulose vollständig zu Galactosyl-Isomalt hydriert. Die nach Abtrennung des Katalysators erhaltene Lösung des hydrierten Gemischs wurde durch lonenaustausch an einem H⁺-beladenen Kationenaustauscher und einem OH⁻-Anionenaustauscher gereinigt.

### Beispiel 14: Isolierung von Galactosyl-Isomalt sowie Galactosyl-Lactit aus dem hydrierten Gemisch

200 ml der hydrierten Lösung des gemäß Beispiel 13 hydrierten, verdünnt auf 20 % TS, wurden mittels Gelpermeationschromatographie (Fractogel HW40S, 3 Trennsäulen je 120 cm Länge, 10 cm Durchmesser) bei 55 °C und einer Durchflussrate von 600 ml/Stunde chromatographiert. Die Fraktionen des Bereichs DP3 wurden vereint, aufkonzentriert und gefriergetrocknet.

Das erhaltene Lyophilisat (2,9 g) wurde in *in* vitro-Analysen wie Verdaubarkeit, Fermentierbarkeit usw. eingesetzt.

### Anwendungsbeispiele:

Die folgenden Ausführungsbeispiele beziehen sich auf die Verwendung von Galactosyl-Isomalt-haltigen Zusammensetzungen, enthaltend mindestens ein Galactosyl-Isomalt ausgewählt aus β-1,3-Isomalt, β-1,4-Galactosyl-Isomalt und β-1,6-Galactosyl-Isomalt, in Nahrungsmitteln. Im Folgenden werden diese Gemische oder Einzelsubstanzen allgemein als "Galactosyl-Isomalt" bezeichnet.

### Anwendungsbeispiel 1: Süßwaren

**Weingummi**

| **Rezeptur [kg]** | **1** | **2** | **3** | **4** | **5** | **6** | **7** |
|---|---|---|---|---|---|---|---|
| Gelatine | 10 | 14 | 11 | 0 | 0 | 20 | 15 |
| Wasser | 20 | 26 | 22 | 80 | 90 | 35 | 30 |
| Zucker | 40 | 35 | 35 | 40 | 50 | 40 | 40 |
| Glukosesirup | 10 | 10 | 40 | 15 | 10 | 40 | 20 |
| **Galactosyl-Isomalt** | 25 | 40 | 55 | 20 | 45 | 40 | 20 |
| Fruchtsäure | 1,3 | 1,6 | 1,4 | 1,0 | 0,6 | 0,5 | 0,7 |
| Glycerin | 1,2 | 4 | 0 | 0 | 4,6 | 0 | 0 |
| Gummi Arabicum | 0 | 0 | 0 | 80 | 84 | 0 | 0 |
| Kochtemperatur [°C] | 136 | 136 | 123 | 123 | 121 | 123 | 130 |

Gelatine mit Wasser einweichen beziehungsweise lösen; Zucker, Glukosesirup und Galactosyl-Isomalt auf die vorgeschriebene Temperatur kochen, etwas abkühlen lassen; Gelatine, Fruchtsäure und Glycerin zugeben; Masse gießen, in Wärmekammer geben, auspudern und ölen.

Gummi Arabicum über Nacht in Wasser lösen, über ein Haarsieb geben; Zucker, Glukosesirup und Galactosyl-Isomalt auf die vorgeschriebene Temperatur kochen, etwas abkühlen lassen; die Gummilösung, Glycerin und Fruchtsäure zugeben; Masse gießen, in Wärmekammer geben, auspudem und ölen.

**Geleefrüchfe**

| | |
|---|---|
| 25 kg | Zucker |
| 25 kg | Galactosyl-Isomalt |
| 0,8 kg | Agar-Agar |
| 30 kg | Wasser |
| 11 kg | Apfelmark |
| 0,5 kg | Weinsäure |
| 0,06 kg | Aroma, Essenzen oder Farbe |

Agar in Wasser einweichen, auflösen, Zucker und weitere Zutaten zugeben und auf 105°C kochen. Die Masse in die entsprechenden Formen gießen.

**Hartkaramellen**

| | **Rezeptur [g]** | |
|---|---|---|
| | **1** | **2** |
| **Galactosyl-Isomalt** | 3250 | 1500 |
| Saccharose | - | 1500 |
| Glucosesirup | - | 1500 |
| Wasser | 968,5 | 200 |
| DL-Äpfelsäure | 30 | 30 |
| Aroma | 6 | 6 |
| Farbe | 3 | 3 |

### Rezeptur 1:

Galactosyl-Isomalt und Wasser werden auf 160°C gekocht und dann evakuiert (-0,9 bar). Nach Abkühlen auf 120°C werden die vorgelöste DL-Äpfelsäure, Aroma und Farbe eingerührt. Die Schmelze wird geprägt oder gegossen.

### Rezeptur 2:

Saccharose, Glukosesirup, Galactosyl-Isomalt und Wasser werden auf 135°C gekocht und dann evakuiert. Nach Abkühlen auf 120°C werden die vorgelöste DL-Äpfelsäure, Aroma und Farbe eingerührt. Die Schmelze wird geprägt oder gegossen.

**Weichkaramellen**

| | **Rezeptur [g]** |
|---|---|
| **Galactosyl-Isomalt** | 164,50 |
| Lycasin 80/55 | 325,00 |
| Wasser | 32,50 |
| Toffix P | 52,50 |
| Gelatine | 19,50 |
| Monomuls 90-35 | 3,25 |
| Lecithin [g}] | 1,30 |
| Calciumcarbonat | 50,00 |
| Acesulfam K | 0,33 |
| Aspartam | 0,33 |
| Aroma | 1,3 |

Galactosyl-Isomalt, Lycasin, Süßstoff und Wasser lösen; bei 120°C Toffix, Lecithin und Monomuls einrühren; bei 125°C Gelatine, Calciumcarbonat und Aroma einrühren; formen.

### Anwendungsbeispiel 2: Hundenahrung

**Hundekuchen**

| | |
|---|---|
| 150 g | Quark |
| 90 g | Milch |
| 90 g | Speiseöl |
| 1 | Eigelb |
| 75 g | **Galactosyl-Isomalt** |
| 200 g | Hundeflocken |

Die Zutaten mischen, kleine Kugeln formen und 200°C 20 Minuten backen.

**Cookies**

| | |
|---|---|
| 150 g | Weizenvollkornmehl |
| 200 g | Vollkomhaferflocken |
| 30 g | Honig |
| 50 g | **Galactosyl-Isomalt** |
| 5 g | gekörnte Brühe |
| 100 g | Vollei |
| 150 g | Milch |

Die Zutaten mischen, Kugeln formen und bei 220°C 15 Minuten backen.

### Anwendungsbeispiel 3: Müsli

**Müsliriegel**

| | |
|---|---|
| 200 g | Haferflocken |
| 100 g | . Cornflakes |
| 100 g | Haselnüsse |
| 50 g | Sonnenblumenkerne |
| 30 g | Kokosraspel |
| 75 g | brauner Zucker |
| 75 g | Honig |
| 100 g | **Galactosyl-Isomalt** |
| 50g | Butter |
| ½ | Zitrone |

Zucker, Honig, Galactosyl-Isomalt, Butter und den Saft der halben Zitrone karamellisieren. Haferflocken, Cornflakes, Nüsse, Sonnenblumenkerne und Kokosraspel mischen und dazugeben. Masse gut durchmischen und auf ein Backblech geben. Riegel ausschneiden und trocken lagern.

**Winter-Birchermüsli**

| | |
|---|---|
| 4 EL | Haferflocken |
| 2 EL | Hirseflocken |
| 1 EL | Weizenkeimflocken |
| Saft von 1 | Zitrone |
| 150 g | Joghurt |
| 1 EL | Sanddorn |
| 50 g | gehackte Nüsse |
| 10 g | Rosinen |
| 400 g | Äpfel |
| 200 g | Birnen |
| 300 g | Orangen |
| 150 g | Banane |
| 80 g | **Galactosyl-Isomalt** |
| (EL = schwach gehäufter Esslöffel) | |

Flocken, Joghurt und Sanddorn vermischen, die Nüsse zugeben. Den Apfel grob reiben und die übrigen Früchte fein würfeln, Zitronensaft über den Apfel geben und Galactosyl-Isomalt zugeben.

**Sommermüsli**

| | |
|---|---|
| 150 g | Aprikosen, gewürfelt |
| 150 g | fettarmer Joghurt |
| 40 g | **Galactosyl-Isomalt** |
| 30 g | Cornflakes |

**Frühstücksceralien**

| | |
|---|---|
| 69,3 g | Weizenmehl Typ 405 |
| 15 g | Hafermehl |
| 1 g | Malz, hell |
| 2,1 g | Malz, dunkel |
| 0,6 g | Salz |
| 10 g | Wasser |
| 12 g | **Galactosyl-Isomalt** |

Weizenmehl, Hafermehl, helles und dunkles Malz, Galactosyl-Isomalt und Salz mischen. Die Zugabe des Wassers erfolgt im Extruder. Der Teig wird dort gemischt, geschert, gekocht, plastifiziert und durch Ringdüsen extrudiert. Anschließend werden die Ringe getrocknet und gekühlt.

### Anwendungsbeispiel 4: Getränke

**Power-Drink**

| | |
|---|---|
| 3 | Orangen |
| 2 EL | Weizenkeime |
| 35 g | **Galactosyl-Isomalt** |
| 200 g | Joghurt |
| (EL = schwach gehäufter Esslöffel) | |

Orangen auspressen, mit Weizenkeimen und Galactosyl-Isomalt verquirlen und Joghurt unterrühren.

**Hobbythek-Drink**

| | |
|---|---|
| 150 ml | Orangensaft |
| 50 ml | Mineralwasser |
| 1 Prise | Multivitaminpulver HT |
| 1 TL | Multimineralpulver HT |
| 5 g | Apfel-Weizen-Ballast HT |
| 7,5 g | **Galactosyl-Isomalt** |
| (TL = schwach gehäufter Teelöffel) | |

**Driver 1**

| | |
|---|---|
| 200 ml | Hagebuttentee |
| 100 ml | Traubensaft |
| 5 g | Apfel-Weizen-Ballast HT |
| 1 TL | Honig |
| 5 g | **Galactosyl-Isomalt** |
| (TL = schwach gehäufter Teelöffel) | |

**Driver 2**

| | |
|---|---|
| 300 ml | Hagebuttentee |
| 5 g | Apfel-Weizen-Ballast HT |
| 1 EL | Quark |
| 100 ml | Traubensaft |
| 10 g | **Galactosyl-Isomalt** |
| (EL = schwach gehäufter Esslöffel) | |

**Ballastgetränk Aronia-Apfel**

| | |
|---|---|
| 200 ml | Mineralwasser |
| 1 ½ TL | Fruchtsirup Aronia |
| 1 TL | Fruchtsirup Apfel |
| 2 TL | Apfelfaser HT |
| 10 g | Galactosyl-Isomalt |
| (TL = schwach gehäufter Teelöffel) | |

**Sportlercocktail**

| | |
|---|---|
| 2 | Tomaten |
| ½ | Salatgurke |
| 250 g | Möhren |
| 250 g | Äpfel |
| 4 EL | Sahne Petersilie |
| 50 g | **Galactosyl-Isomalt** |
| (EL = schwach gehäufter Esslöffel) | |

Tomaten, Gurke, Möhre und Äpfel entsaften, Sahne, Petersilie und Galactosyl-Isomalt hinzufügen.

**Tomatencocktail**

| | |
|---|---|
| 6 | Tomaten |
| 4 EL | Sahne |
| Saft von 1 | Orange |
| 1 Prise | Salz |
| 7,5 g | **Galactosyl-Isomalt** |
| 1 Prise | Paprika |
| 2 Spritzer | Tabasco |
| (EL = ca. 12 ml) | |

Tomaten pürieren und mit restlichen Zutaten verrühren.

**Orangennektar mit 50% Fruchtgehalt:**

| | |
|---|---|
| 120 kg | Orangennektar-Grundstoff 50:11; Saftgehalt 400%; Extraktgehalt 50 % |
| 48 kg | Zuckersirup 65 % TS |
| 60 kg | **Galactosyl-lsomalt** |
| 820 kg | Trinkwasser |

**Zitronenlimonade**

| | |
|---|---|
| 4,5 kg | Limonaden-Grundstoff 3:100; Extraktgehalt 40 % |
| 60 kg | Zuckersirup 65 % TS |
| 75 kg | **Galactosyl-Isomalt** |
| 888,5 kg | Trinkwasser |
| 8 kg | CO₂ |

### Anwendungsbeispiel 5: Fruchtzubereitungen

**Rote Grütze**

| | |
|---|---|
| 330 g | Sauerkirschen |
| 150 g | Heidelbeeren |
| 300 g | Himbeeren |
| 300 g | Erdbeeren |
| 60 g | Stärke |
| 1 l | Fruchtsaft |
| 60 g | Zucker |
| 50 g | **Galactosyl-Isomalt** |

Die Stärke mit etwas kaltem Fruchtsaft anrühren und in den kochenden Fruchtsaft einrühren. 5 Minuten kochen lassen. Die Früchte, den Zucker und die Galactosyl-Isomalt zugeben.

**Rhabarberkaltschale**

| | |
|---|---|
| 750 g | Rhabarber |
| 0,51 l | Wasser |
| Saft von ½ | Zitrone |
| 120 g | Zucker |
| 75 g | **Galactosyl-Isomalt** |
| 0,2 l | Weißwein |

Rhabarber waschen, schneiden mit Wasser und dem Zitronensaft weich dünsten. Noch warm mit Zucker und Galactosyl-Isomalt verrühren, abkühlen lassen und Weißwein einrühren.

**Fruchtpüree**

| | |
|---|---|
| 750 g | Früchte |
| 30 g | Fruchtsaft |
| 50 g | **Galactosyl-Isomalt** |
| 3 ml | Rum |

Die Zutaten im Mixer pürieren.

**Erdbeercreme**

| | |
|---|---|
| 375 g | Erdbeeren |
| 50 g | **Galactosyl-Isomalt** |
| 1 Päckchen | Vanillezucker |
| 2 Blatt | Gelatine weiß |
| 2 Blatt | Gelatine rot |
| 250 ml | Sahne |

Beeren pürieren, Galactosyl-Isomalt und Vanillezucker zugeben, aufgelöste Gelatine zugeben und kaltstellen. Die Sahne steif schlagen und unterheben.

**Aprikosencreme**

| | |
|---|---|
| 100 g | Aprikosen |
| 375 ml | Wasser |
| 30 g | Zucker |
| 50 g | **Galactosyl-Isomalt** |
| 1 Päckchen | Vanillezucker |
| 4 Blatt | weiße Gelatine |
| 1 Blatt | rote Gelatine |
| 250 ml | Sahne |

Aprikosen, Wasser, Zucker, Galactosyl-Isomalt und Vanillezucker 30 Minuten kochen. Gelatine in Aprikosenkompott auflösen, Masse pürieren und kalt stellen. Sahne steif schlagen und unterheben.

### Anwendungsbeispiel 6: Joghurt

**Joghurt-Zitronenshake**

| | |
|---|---|
| 600 g | Magerjoghurt |
| Saft von 4 | Zitronen |
| 4 TL | Honig |
| 30 g | **Galactosyl-Isomalt** |
| 4 | Eigelb |
| Zutaten mischen. | |

**Zitronenjoghurtcreme**

| | |
|---|---|
| 4 | Eier |
| 40 g | Zucker |
| **40 g** | **Galactosyl-Isomalt** |
| 25 ml | Zitronensaft |
| 300 g | Joghurt |
| 6 g | Gelatinepulver |

Die Gelatine einweichen. Eigelb vom Eiklar trennen. Joghurt, Eigelb, Zucker, Galactosyl-Isomalt und Zitronensaft mischen. Die Gelatine auflösen und zugeben. Das Eiklar zu Schnee schlagen und unterheben.

### Anwendungsbeispiel 7: Konfitüre

**Südzucker-Gelierzucker-Rezepturen**

| **Rezeptur [g]** | **GZ 1 plus 1** | | **GZ 1 plus 1 Fructose** |
|---|---|---|---|
| Pektin | 7,370 | | 7,370 |
| Citronensäure | 10,700 | | 10,700 |
| Galactosyl-Isomalt | 490,965 | | 490,965 |
| Zucker | 490,965 | | 0,000 |
| Fruktose | 0,000 | | 490,965 |
| Fruchtmenge | 970,000 | | 970,000 |

| **Rezeptur [g]** | **GZ 2 plus 1** | **GZmZ** | **GZ 3 plus 1** |
|---|---|---|---|
| amidiertes Pektin | 6,41 | 8,00 | 11,55 |
| Citronensäure | 3,80 | 3,80 | 3,80 |
| Sorbinsäure | 0,63 | 0,63 | 0,63 |
| **Galactosyl-Isomalt** | 489,17 | 110,00 | 484,02 |
| Zucker | 0,00 | 377,57 | 0,00 |
| Fruchtmenge | 970,00 | 1000,00 | 1455,00 |

Kochzeit jeweils 4 Minuten (außer GZmZ), GZmZ: Kochzeit 5 Minuten

**Sauerkirschkonfitüre mit Amaretto und Vanille**

| | |
|---|---|
| 1 kg | Sauerkirschen |
| 3 | Vanillestangen |
| 500 g | Gelierzucker 2:1 |
| 40 ml | Amaretto (Mandellikör) |

Die Hälfte der Sauerkirschen im Mixer gut zerkleinem. Das Fruchtmus mit den restlichen Kirschen, dem Mark der Vanillestangen und Gelierzucker vermischen und unter Rühren zum Kochen bringen. 4 Minuten sprudelnd kochen lassen. Den Amaretto zufügen. Die Konfitüre heiß in Gläser füllen und sofort verschließen.

**Rhabarber-Erdbeer-Konfitüre**

| | |
|---|---|
| 750 g | Rhabarber |
| 250 g | Erdbeeren |
| 1000 g | Gelierzucker 1:1 |
| 3 Päckchen | Vanillezucker |
| 1 EL | feingehackte Zitronenmelisse |

Rhabarber und Erdbeeren in Stücke schneiden. Die Früchte mit Gelier- und Vanillezucker mischen und zugedeckt 3 bis 4 Stunden durchziehen lassen. Dann unter Rühren zum Kochen bringen, 4 Minuten sprudelnd kochen lassen. Die Zitronenmelisse unterrühren. Die Konfitüre heiß in Gläser füllen und sofort verschließen.

**Kürbisgelee**

| | |
|---|---|
| 1,5 kg | Kürbis |
| 1,2 l | Wasser |
| 1 kg | Gelierzucker 1:1 |
| Saft von 2 | Zitronen |
| 1 TL | gehackte Minze |

Den Kürbis in Würfel schneiden und mit dem Wasser 20 bis 30 Minuten weich kochen. Den Saft durch ein Tuch ablaufen lassen. 750 ml kalten Saft mit Gelierzucker und Zitronensaft mischen und unter Rühren zum Kochen bringen. 4 Minuten sprudelnd kochen lassen. Die Minze unterrühren. Das Gelee heiß in Gläser füllen und sofort verschließen.

**Erdbeerkonfitüre mit Grand Marnier**

| | |
|---|---|
| 1 kg | Erdbeeren |
| 1 kg | Gelierzucker |
| 1 | unbehandelte Orange |
| 65 g | Grand Marnier (Orangenlikör) |

Die Erdbeeren zerdrücken, Gelierzucker und die abgeriebene Schale der Orange hinzufügen und alles gut vermischen. Unter Rühren zum Kochen bringen, 4 Minuten sprudelnd kochen lassen. Grand Marnier unterrühren. Heiß in Gläser füllen und sofort verschließen.

### Anwendungsbeispiel 8: Backwaren

In den aufgeführten Rezepturen wird Hefe als Backtriebmittel eingesetzt. Die erfindungsgemäße Galactosyl-Isomalt kann von Backhefe nur bedingt als Substrat genutzt werden. Daher wird nur ein Teil des Zuckers gegen Galactosyl-Isomalt ausgetauscht.

**Frühstückshörnchen**

| Komponente [g] | |
|---|---|
| Hefe | 25 |
| Sahne | 250 |
| Zucker | 25 |
| **Galactosyl-Isomalt** | 35 |
| Weizenmehl Typ 550 | 400 |
| Salz | 0,15 |
| Margarine | 200 |
| Eigelb | 50 |

Hefe, laufwarme Sahne, 1 Prise Salz und 1 Prise Mehl verrühren. 10 Minuten gehen lassen. Mit weiteren Zutaten verkneten und 20 Minuten gehen lassen. Teig durchkneten, ausrollen, 15 Dreiecke ausschneiden und zu Hörnchen aufrollen. Kurz aufgehen lassen und 10 Min. bei 200°C backen.

**Weißbrot**

| **Komponente [g]** | |
|---|---|
| Hefe | 40 |
| Zucker | 15 |
| **Galactosyl-Isomalt** | 20 |
| Weizenmehl Typ 550 | 1000 |
| Milch | 500 |
| Margarine | 250 |
| abgeriebene Zitronenschale | 2,5 |
| Vollei | 50 |

Hefe mit Zucker in lauwarme Milch einrühren und 10 Minuten gehen lassen. Mit den weiteren Zutaten kneten und 20 Minuten gehen lassen. In einer Brotbackform 45 Minuten bei 175°C backen.

**Sesambrot**

| **Komponente [g]** | |
|---|---|
| Hefe | 60 |
| Milch | 500 |
| Zucker | 30 |
| **Galactosyl-Isomalt** | 45 |
| Weizenmehl Typ 550 | 300 |
| Roggenmehl Typ 1150 | 250 |
| Weizenschrot Typ 1700 | 200 |
| Salz | 0,15 |
| Margarine | 100 |
| Sesamsaat | 100 |

Herstellung siehe Weißbrot

**Grundrezept Mürbteig**

| **Komponente [g]** | **Mürbeteig** | **Mürbeteig ohne Zucker** |
|---|---|---|
| Mehl | 250 | 250 |
| Zucker | 35 | 0 |
| Galactosyl-Isomalt | 45 | 90 |
| Salz | 0,15 | 0,15 |
| gekühlte Margarine | 125 | 125 |
| Vollei | 50 | 50 |

Alle Zutaten mit Knethaken auf niedrigster Stufe kurz vermischen und dann auf höherer Stufe gut verkneten. Teig vor dem Abbacken kaltstellen.

**Grundrezept Rührmasse**

| **Komponente [g]** | **Rührmasse** | **Rührmasse ohne Zucker** |
|---|---|---|
| Margarine | 125 | 125 |
| Zucker | 65 | 0 |
| **Galactosyl-Isomalt** | 90 | 180 |
| Salz | 0,15 | 0,15 |
| Vollei | 100 | 100 |
| Mehl | 250 | 250 |
| Backpulver | 8 | 8 |
| Milch | 125 | 125 |

Alle Zutaten mit dem Schneebesen zunächst auf kleiner Stufe, dann auf höchster Stufe rühren. Die beiden so hergestellten Rührmassen zeigen eine stärkere Bräunung als eine Rührmasse mit Zucker und sind weniger süß. Daher wird empfohlen, die beiden oben aufgeführten Rührmassen bei Bedarf mit einem Süßstoff aufzusüßen.

**Grundrezept Biskuit**

| **Komponente [g]** | **Biskuit** | **Biskuit ohne Zucker** |
|---|---|---|
| Vollei | 200 | 200 |
| Wasser | 60 | 60 |
| Zucker | 65 | 0 |
| **Galactosyl-Isomalt** | 90 | 180 |
| Mehl | 75 | 75 |
| Speisestärke | 75 | 75 |
| Backpulver | 0,5 | 0,5 |

Eigelb, Wasser, Zucker, Galactosyl-Isomalt und Salz mit dem Schneebesen schaumig schlagen. Sehr steif geschlagenes Eiweiß auf die Eigelbmasse geben. Mehl, Speisestärke und Backpulver mischen, auf den Schnee sieben und vorsichtig unterziehen.

### Anwendungsbeispiel 9: Rezepturen mit Galactosyl-Isomaltulose

In sämtlichen in den Anwendungsbeispielen 1 bis 8 aufgeführten Rezepturen wurde die Komponente Galactosyl-Isomalt in der jeweils angegebenen Menge durch eine identische Menge die erfindungsgemäße Galactosyl-Isomaltulose ersetzt, wobei ebenfalls vorteilhafte Nahrungs-, Lebens- oder Genussmittel beziehungsweise Tierfuttermittel erhalten wurden.

## Patentansprüche

1. Verfahren zur Herstellung einer galactosylierte hydrierte Isomaltulose oder galactosylierte nicht-hydrierte Isomaltulose enthaltenden Zusammensetzung, wobei eine wässrige Lösung mindestens eines Galactosyl-Donors mit mindestens einer β-Galactosidase und einer wässrigen Lösung eines hydrierte Isomaltulose oder nicht-hydrierte Isomaltulose als Galactosyl-Akzeptor enthaltenden Gemisches in Kontakt gebracht wird, und eine Galactosyl-Isomalt-Zusammensetzung (A) oder eine Galactosyl-Isomaltulose-Zusammensetzung erhalten wird.

2. Verfahren nach Anspruch 1, wobei der Galactosyl-Donor Lactose ist.

3. Verfahren nach Anspruch 1 oder 2, wobei eine Galactosyl-Isomalt-Zusammensetzung (A) erhalten wird, die Galactosyl-1,6-GPS, Galactosyl-1,1-GPM, Galactosyl-Lactose sowie Glucose, Galactose, Lactose und/oder Isomalt enthält.

4. Verfahren nach einem der Ansprüche 1 bis 3, wobei eine Galactosyl-Isomaltulose-Zusammensetzung erhalten wird, die Galactosyl-Isomaltulose, Galactosyl-Lactose, sowie Glucose, Galactose, Lactose und/oder Isomaltulose enthält.

5. Verfahren nach einem der vorstehenden Ansprüche, wobei die β-Galactosidase aus *Bacillus circulans,* aus *Aspergillus oryzae,* aus Stierhoden, aus *Bacillus stearothermophilus* KVE39, *Thermus brockianus* ITI360 oder *Thermus thermophilus* TH125 stammt.

6. Verfahren nach einem der vorstehenden Ansprüche, wobei das Gewichtsverhältnis des mindestens einen Galactosyl-Donors zu dem hydrierte Isomaltulose oder nicht-hydrierte Isomaltulose enthaltenden Gemisch von 1:1 bis 1:5 beträgt.

7. Verfahren nach einem der vorstehenden Ansprüche, wobei der erste Schritt über einen Reaktionszeitraum von 1 bis 48 Stunden durchgeführt wird.

8. Verfahren nach einem der vorstehenden Ansprüche, wobei der erste Schritt bei einer Temperatur von 30 °C bis 55 °C durchgeführt wird.

9. Verfahren nach einem der vorstehenden Ansprüche, wobei die nach dem ersten Schritt erhaltene Galactosyl-Isomaltulose-Zusammensetzung in einem nachfolgenden zweiten Schritt hydriert und anschließend eine Galactosyl-Isomalt-Zusammensetzung (B) erhalten wird.

10. Verfahren nach einem der vorstehenden Ansprüche, wobei in einem nachfolgenden Schritt aus der erhaltenen Galactosyl-Isomalt-Zusammensetzung (A, B) Galactosyl-1,1-GPM, Galactosyl-1,6-GPS abgetrennt wird.

11. Verfahren nach einem der vorstehenden Ansprüche, wobei die Hydrierung als katalytische Hydrierung und bei erhöhter Temperatur und/oder erhöhtem Druck sowie in Gegenwart von Wasserstoff und unter Verwendung eines Hydrierkatalysators erfolgt.

12. Verfahren nach einem der vorstehenden Ansprüche, wobei eine die Galactosyl-Isomaltulose-Zusammensetzung enthaltende wässrige Lösung vor der Hydrierung auf einen pH-Wert von 6 bis 8 eingestellt wird.

13. Verfahren nach einem der vorstehenden Ansprüche, wobei die Hydrierung bei einer erhöhten Temperatur von 40 bis 140 °C, bevorzugt von 60 bis 80 °C erfolgt.

14. Verfahren nach einem der vorstehenden Ansprüche, wobei die Hydrierung bei einem erhöhten Druck von 50 bis 230 bar, bevorzugt von 100 bis 200 bar erfolgt.

15. Verfahren nach einem der vorstehenden Ansprüche, wobei der Hydrierkatalysator ein Gemisch aus einem reinen Raney-Metall und einer Raney-Metalllegierung umfasst.

16. Verfahren nach einem der vorstehenden Ansprüche, wobei in dem zweiten Schritt die Hydrierung über einen Zeitraum von 2 bis 5 Stunden erfolgt.

17. Verfahren nach einem der vorstehenden Ansprüche, wobei die Hydrierung kontinuierlich, semi-kontinuierlich oder diskontinuierlich erfolgt.

18. Verfahren nach einem der vorstehenden Ansprüche, wobei die Hydrierung im Festbett- oder Suspensionsverfahren durchgeführt wird.

19. Galactosyl-Isomalt-Zusammensetzung (A, B), hergestellt nach einem Verfahren gemäß einem der vorstehenden Ansprüche.

20. β-1,3-Galactosyl-Isomalt der Formel: 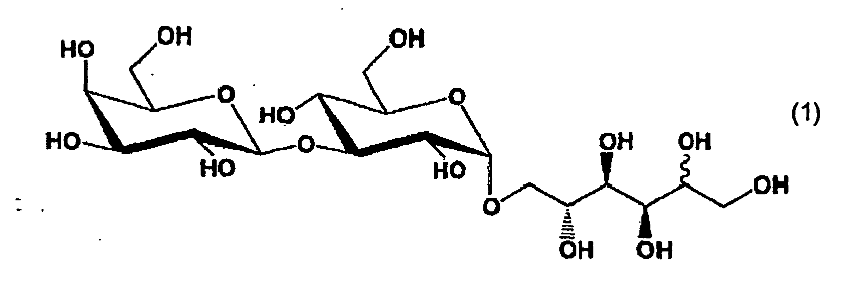 oder dessen Sorbit-Diastereomer oder dessen Mannit-Diastereomer.

21. β-1,4-Galactosyl-lsomalt der Formel: 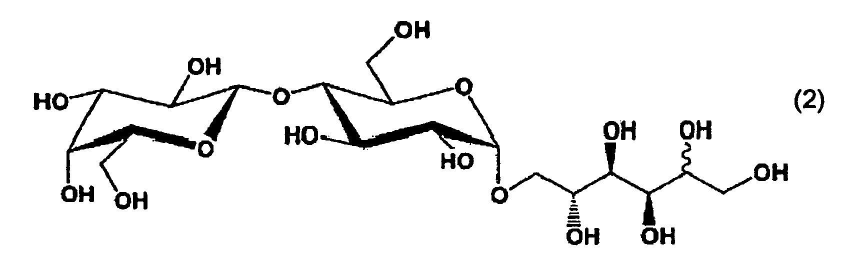 oder dessen Sorbit-Diastereomer oder dessen Mannit-Diastereomer.

22. β-1,6-Galactosyl-Isomalt der Formel: 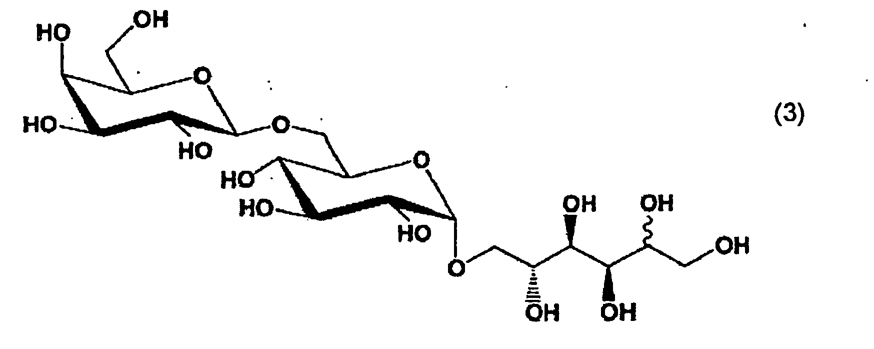 oder dessen Sorbit-Diastereomer oder dessen Mannit-Diastereomer.

23. Galactosyl-Isomalt-Zusammensetzung enthaltend
a) 4 bis 30 Gewichtsprozent β-1,6-Galactosyl-Isomalt, enthaltend 1,6-Galactosyl-1,1-GPM und 1,6-Galactosyl-1,6-GPS,
b) 6 bis 60 Gewichtsprozent β-1,4-Galactosyl-Isomalt, enthaltend 1,4-Galactosyl-1,1-GPM und 1,4-Galactosyl-1,6-GPS und
c) 15 bis 90 Gewichtsprozent β-1,3-Galactosyl-Isomalt, enthaltend 1,3-Galactosyl-1,1-GPM und 1,3-Galactosyl-1,6-GPS.

24. Galactosyl-Isomalt-Zusammensetzung nach einem der vorstehenden Ansprüche, zusätzlich enthaltend mindestens ein Monosaccharid (DP 1), mindestens ein Disaccharid (DP 2), mindestens ein Tetrasaccharid (DP 4) und/oder mindestens ein Alkohol davon.

25. Galactosyl-Isomalt-Zusammensetzung nach vorstehendem Anspruch, wobei das Gemisch 2 bis 30 Gewichtsprozent Monosaccharide (DP 1), 60 bis 90 Gewichtsprozent Disaccharide (DP 2), 1 bis 45 Gewichtsprozent, vorzugsweise 2 bis 30 Gewichtsprozent, Galactosyl-Isomalt der Formeln (1), (2) und (3) (DP 3) sowie bis 5 Gewichtsprozent Tetrasaccharide (DP 4) aufweist.

26. Galactosyl-Isomalt-Zusammensetzung (A) nach einem der vorstehenden Ansprüche enthaltend Galactose, Glucose, Lactose, Isomalt und/oder Galactosyl-Lactose.

27. Galactosyl-Isomalt-Zusammensetzung (B) nach einem der vorstehenden Ansprüche enthaltend Galactit, Sorbit, Lactit, Isomalt und/oder Galactosyl-Lactit.

28. β-1,3-Galactosyl-Isomaltulose der Formel:

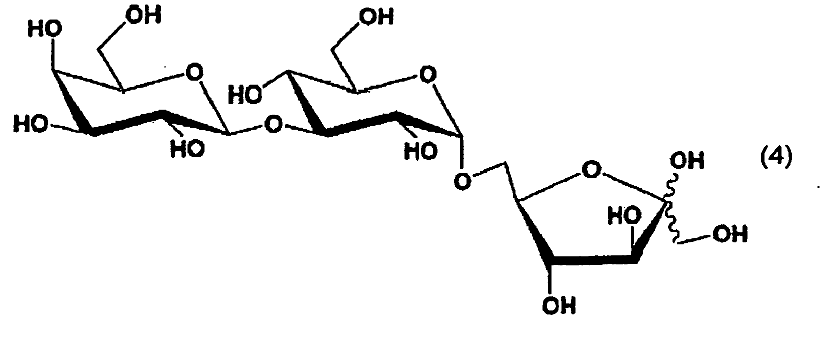

29. β-1,4-Galactosyl-Isomaltulose der Formel:

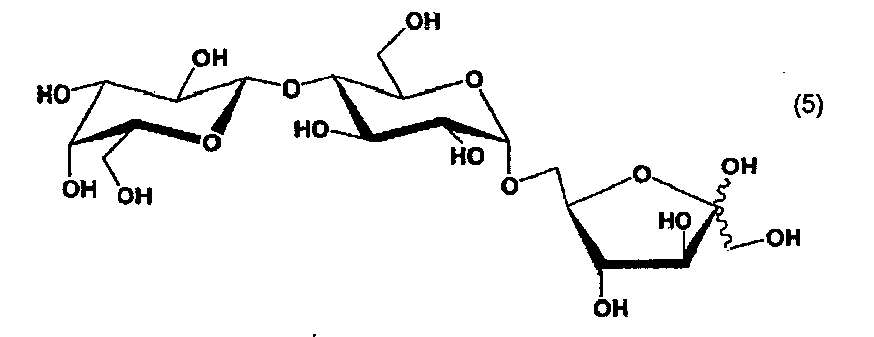

30. β-1,6-Galactosyl-Isomaltulose der Formel:

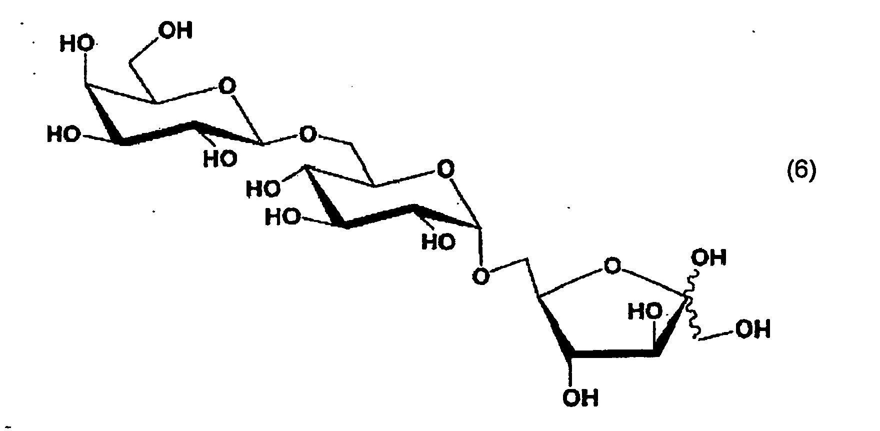

31. Galactosyl-Isomaltulose-Zusammensetzung enthaltend
a) 4 bis 30 Gewichtsprozent β-1,6-Galactosyl-Isomaltulose,
b) 6 bis 60 Gewichtsprozent β-1,4-Galactosyl-Isomaltulose, und
c) 15 bis 90 Gewichtsprozent β-1,3-Galactosyl-Isomaltulose.

32. Galactosyl-Isomaltulose-Zusammensetzung nach vorstehendem Anspruch zusätzlich enthaltend mindestens ein Monosaccharid (DP 1), mindestens ein Disaccharid (DP 2), mindestens ein Tetrasaccharid (DP 4) und/oder mindestens einen Alkohol davon.

33. Galactosyl-Isomaltulose-Zusammensetzung nach vorstehendem Anspruch, wobei das Gemisch 2 bis 30 Gewichtsprozent Monosaccharide (DP 1), 60 bis 90 Gewichtsprozent Disaccharide (DP 2), 1 bis 45 Gewichtsprozent, vorzugsweise 2 bis 30 Gewichtsprozent, Galactosyl-Isomaltulose der Formeln (4), (5) und (6) (DP 3) sowie bis 5 Gewichtsprozent Tetrasaccharide (DP 4) aufweist.

34. Galactosyl-Isomaltulose-Zusammensetzung nach einem der vorstehenden Ansprüche enthaltend Galactose, Glucose, Lactose, Isomaltulose und/oder Galactosyl-Lactose.

35. Synbiotikum enthaltend eine Galactosyl-Isomalt-Zusammensetzung und/oder eine Galactosyl-Isomaltulose-Zusammensetzung nach einem der vorstehenden Ansprüche und mindestens ein Probiotikum, insbesondere Bifidobakterien.

36. Ballaststoffzusammensetzung enthaltend eine Galactosyl-Isomalt-Zusammensetzung und/oder eine Galactosyl-Isomaltulose-Zusammensetzung nach einem der vorstehenden Ansprüche und mindestens einen weiteren Ballaststoff, ausgewählt aus der Gruppe der Ballaststoffe bestehend aus kurzkettigen Fructooligosacchariden, langkettigen Fructooligosacchariden, Galactooligosacchariden, hydrolysiertem Guar Gum, Lactulose, Xylo-Oligosacchariden, Lactosucrose, Malto-Oligosacchariden, Isomalto-Oligosacchariden, Gentio-Oligosacchariden, pektischen Substanzen, Hydrolasen, Kondensationsprodukten aus Zuckern, Glucosyl-Sucrose, wie "Coupling Sugar" von Hayashibara, Glucosyl-Sucrose, Sojabohnen-Oligosacchariden, Chito-Oligosacchariden, Chitosan-Oligosacchariden, resistenter Stärke; Haferfasem, Weizenfasern, Gemüsefasem, Fruchtfasem, Cellulosen und Zuckerrübenfasern.

37. Nahrungs-, Lebens-, Genuss- oder Tierfuttermittel, enthaltend ein Synbiotikum, eine Ballaststoffzusammensetzung, eine Galactosyl-Isomalt-Zusammensetzung und/oder eine Galactosyl-Isomaltulose-Zusammensetzung nach einem der vorstehenden Ansprüche.

38. Lebensmittel nach vorstehendem Anspruch, wobei es sich um Milcherzeugnisse und Milchprodukte, insbesondere Käse-, Butter-, Joghurt-, Kefir-, Quark-, Sauermilch-, Buttermilch-, Sahne-, Kondensmilch-, Trockenmilch-, Molken-, Milchzucker-, Milcheiweiß-, Milchmisch-, Milchhalbfett-, Molkenmisch- oder Milchfett-Produkte oder -Zubereitungen; um Backwaren, insbesondere um Brot einschließlich Kleingebäck und feine Backwaren einschließlich Dauerbackwaren, Keksprodukte und Waffeln; um Brotaufstriche; um Margarine-Erzeugnisse und Backfette; um Instantprodukte und Brüherzeugnisse; um Obstprodukte oder -zubereitungen, insbesondere um Konfitüren, Marmeladen, Gelees, Obstkonserven, Fruchtpulpe, Fruchtmark, Fruchtsäfte, Fruchtsaftkonzentrate, Fruchtnektar und Fruchtpulver; um Gemüseerzeugnisse oder deren Zubereitungen, insbesondere um Gemüsekonserven, Gemüsesäfte und Gemüsemark; um Gewürzmischungen, um Müsli und Müsli-Mischungen sowie fertig zubereitete Müsli enthaltende Produkte oder um nicht-alkoholische Getränke, Getränkegrundstoffe und Getränkepulver handelt..

39. Lebensmittel nach einem der vorstehenden Ansprüche, wobei es sich um ein kalorienreduziertes Lebensmittel handelt.

40. Süßwaren, enthaltend ein Synbiotikum, eine Ballaststoffzusammensetzung, eine Galactosyl-Isomalt-Zusammensetzung und/oder eine Galactosyl-Isomaltulose-Zusammensetzung nach einem der vorstehenden Ansprüche.

41. Süßwaren nach vorstehendem Anspruch, wobei es sich um kalorienreduzierte Süßwaren handelt.

42. Lebensmittel oder Süßwaren nach einem der vorstehenden Ansprüche, wobei es sich um Schokolade, Hartkaramellen, Weichkaramellen, Fondant-Erzeugnisse, Gelee-Erzeugnisse, Lakritzen, Schaumzuckerwaren, Kokosflocken, Dragees, Komprimate, kandierte Früchte, Krokant, Nougat-Erzeugnisse, Eiskonfekt, Marzipan, Kaugummi, Müsliriegel, sowie Speiseeis oder alkoholische und nicht-alkoholische Süßgetränke handelt.

43. Diätetische Spezialnahrungsmittel, insbesondere zur Ernährung von Personen mit Glucose-Intoleranz, Insulin-Resistenz, Syndrom-X, enthaltend ein Synbiotikum, eine Ballaststoffzusammensetzung, eine Galactosyl-Isomalt-Zusammensetzung und/oder eine Galactosyl-Isomaltulose-Zusammensetzung nach einem der vorstehenden Ansprüche.

44. Kindernährungsmittel, enthaltend ein Synbiotikum, eine Ballaststoffzusammensetzung, eine Galactosyl-Isomalt-Zusammensetzung und/oder eine Galactosyl-Isomaltulose-Zusammensetzung nach einem der vorstehenden Ansprüche.

45. Süßungsmittel, enthaltend ein Synbiotikum, eine Ballaststoffzusammensetzung, eine Galactosyl-Isomalt-Zusammensetzung und/oder eine Galactosyl-Isomaltulose-Zusammensetzung nach einem der vorstehenden Ansprüche.

46. Pharmazeutische Zusammensetzung, enthaltend ein Synbiotikum, eine Ballaststoffzusammensetzung, eine Galactosyl-Isomalt-Zusammensetzung und/oder eine Galactosyl-Isomaltulose-Zusammensetzung nach einem der vorstehenden Ansprüche.

47. Pharmazeutische Zusammensetzung nach vorstehendem Anspruch als Wirkstoff.

48. Pharmazeutische Zusammensetzung nach einem der vorstehenden Ansprüch als pharmazeutischer Träger.

49. Verwendung der Galactosyl-Isomalt-Zusammensetzung und/oder der Galactosyl-Isomaltulose-Zusammensetzung nach einem der vorstehenden Ansprüche als diätetische Faserquelle und/oder als Nährstoff für eine gesunde Mikroflora.

50. Verwendung der Galactosyl-Isomalt-Zusammensetzung und/oder der Galactosyl-Isomaltulose-Zusammensetzung nach einem der vorstehenden Ansprüche als gegen einen Abbau im Säugetier-Magen und/oder gegen die Enzyme des Säugetier-Verdauungstraktes resistentes Mittel.

51. Verwendung der Galactosyl-Isomalt-Zusammensetzung und/oder der Galactosyl-Isomaltulose-Zusammensetzung nach einem der vorstehenden Ansprüche als Zusatz in Lebensmitteln, Tierfuttermitteln oder Getränken, insbesondere als löslicher Ballaststoff, bevorzugt als präbiotischer Ballaststoff.

52. Verwendung der Galactosyl-Isomalt-Zusammensetzung und/oder der Galactosyl-Isomaltulose-Zusammensetzung nach einem der vorstehenden Ansprüche zur Modulation der glykämischen Eigenschaften von Lebensmitteln oder Süßwaren, insbesondere von Spezialernährung, Kinderemährung oder Ernährung von Personen mit Störungen des Glucose/Insulin-Haushalts.

53. Verwendung der Galactosyl-Isomalt-Zusammensetzung und/oder der Galactosyl-Isomaltulose-Zusammensetzung nach einem der vorstehenden Ansprüche als Süßungsmittel.

54. Verwendung der Galactosyl-Isomalt-Zusammensetzung und/oder der Galactosyl-Isomaltulose-Zusammensetzung nach einem der vorstehenden Ansprüche zur Herstellung von Nahrungs-, Lebens- Genuss- oder Tierfuttermitteln.

55. Verwendung der Galactosyl-Isomalt-Zusammensetzung und/oder der Galactosyl-Isomaltulose-Zusammensetzung nach einem der vorstehenden Ansprüche zur Prophylaxe und/oder Behandlung von Störungen des Glucosestoffwechsels, Diabetes I und II, Glucose-Intoleranz, Insulin-Resistenz und/oder Syndrom-X.

56. Verwendung der Galactosyl-Isomalt-Zusammensetzung und/oder der Galactosyl-Isomaltulose-Zusammensetzung nach einem der vorstehenden Ansprüche zur Herstellung eines Arzneimittels zur Prophylaxe und/oder Behandlung von Störungen des Glucosestoffwechsels, Diabetes I und II, Glucose-Intoleranz, Insulin-Resistenz und/oder Syndrom-X.

57. Verwendung der Galactosyl-Isomalt-Zusammensetzung und/oder der Galactosyl-Isomaltulose-Zusammensetzung nach einem der vorstehenden Ansprüche zur Verbesserung der Resorbierung von Nahrungsbestandteilen im tierischen oder menschlichen Verdauungstrakt.

58. Verwendung der Galactosyl-Isomalt-Zusammensetzung und/oder der Galactosyl-Isomaltulose-Zusammensetzung nach einem der vorstehenden Ansprüche zur Herstellung eines Arzneimittels zur Verbesserung der Resorbierung von Nahrungsbestandteilen im tierischen oder menschlichen Verdauungstrakt.

59. Verwendung der Galactosyl-Isomalt-Zusammensetzung und/oder der Galactosyl-Isomaltulose-Zusammensetzung nach einem der vorstehenden Ansprüche als Wirkstoff zur Prophylaxe und/oder Behandlung von Durchfallerkrankungen, insbesondere solche die durch Infektion mit Mikroorganismen hervorgerufen werden; von entzündlichen Darmerkrankungen; von Infektionskrankheiten; der Colonkarzinogenese; der Osteoporose; von Erkrankungen, die durch unverdauliche Kohlenhydrate beeinflusst werden können; von Krebserkrankungen; der Obstipation; der Hypertonie; von Schlaganfall; von durch pathogene Keime hervorgerufenen Krankheiten; von rheumatischen Erkrankungen, von Koronararterien-Erkrankungen; von akutem Herzinfarkt; von Herz-Kreislauf-Erkrankungen; von chronisch-entzündlichen Krankheiten; zur Wiederherstellung und Intakterhaftung einer gesunden Mikroorganismenflora im Verdauungstrakt und/oder eines gesunden Darmepithels und/oder zur Stärkung der Immunabwehr gegen allgemeine Infekte.

60. Verwendung der Galactosyl-Isomalt-Zusammensetzung und/oder der Galactosyl-Isomaltulose-Zusammensetzung nach einem der vorstehenden Ansprüche zur Herstellung eines Arzneimittels zur Prophylaxe und/oder Behandlung von Durchfallerkrankungen, insbesondere solche die durch Infektion mit Mikroorganismen hervorgerufen werden; von entzündlichen Darmerkrankungen; von Infektionskrankheiten; der Colonkarzinogenese; der Osteoporose; von Erkrankungen, die durch unverdauliche Kohlenhydrate beeinflusst werden können; von Krebserkrankungen; der Obstipation; der Hypertonie; von Schlaganfall; von durch pathogene Keime hervorgerufenen Krankheiten; von rheumatischen Erkrankungen, von Koronararterien-Erkrankungen; von akutem Herzinfarkt; von Herz-Kreislauf-Erkrankungen; von chronisch-entzündlichen Krankheiten; zur Wiederherstellung und Intakterhaltung einer gesunden Mikroorganismenflora im Verdauungstrakt und/oder eines gesunden Darmepithels und/oder zur Stärkung der Immunabwehr gegen allgemeine Infekte.

61. Verwendung nach einem der vorstehenden Ansprüche, wobei die Galactosyl-Isomalt-Zusammensetzung und/oder die Galactosyl-Isomaltulose-Zusammensetzung nach einem der vorstehenden Ansprüche in einer Dosis verabreicht wird, die ausreicht, den Zustand einer durch oxidativen Stress verursachten Krankheit oder eines Infektes zu heilen oder ihm vorzubeugen, die Progression der Krankheit zu stoppen und/oder die Symptome der Krankheit zu lindem.

62. Verwendung nach einem der vorstehenden Ansprüche, wobei die Galactosyl-Isomalt-Zusammensetzung und/oder die Galactosyl-lsomaltulose-Zusammensetzung nach einem der vorstehenden Ansprüche als pharmazeutische Zusammensetzung, insbesondere als Suspension, Sirup, Tablette, Pille, Kapsel, Granulat oder Pulver verabreicht wird.

## Claims

1. A method for producing a composition containing galactosylated hydrogenated isomaltulose or galactosylated nonhydrogenated isomaltulose, where an aqueous solution of at least one galactosyl donor is brought into contact with at least one β-galactosidase and an aqueous solution of a hydrogenated isomaltulose or nonhydrogenated isomaltulose as galactosyl acceptor, and a galactosyl isomalt composition (A) or a galactosyl isomaltulose composition is obtained.

2. A method as in Claim 1, where the galactosyl donor is lactose.

3. A method as in Claim 1 or 2, where a galactosyl isomalt composition (A) that contains galactosyl-1,6-GPS, galactosyl-1,1-GPM, galactosyl lactose and glucose, galactose, lactose and/or isomalt is obtained.

4. A method as in one of Claims 1-3, where a galactosyl isomaltulose composition that contains galactosyl isomaltulose, galactosyl lactose, and glucose, galactose, lactose and/or isomaltulose is obtained.

5. A method as in one of the preceding claims, where the β-galactosidase derives from Bacillus circulans, from Aspergillus oryzae, from bull testicle, from Bacillus stearothermophilus KVE39, Thermus brockianus ITI360 or Thermus thermophilus TH125.

6. A method as in one of the preceding claims, where the weight ratio of the minimum of one galactosyl donor to the mixture containing hydrogenated isomaltulose or nonhydrogenated isomaltulose is from 1:1-1:5.

7. A method as in one of the preceding claims, where the first step is carried out over a reaction time of 1-48 h.

8. A method as in one of the preceding claims, where the first step is carried out at a temperature from 30-55°C.

9. A method as in one of the preceding claims, where the galactosyl isomaltulose composition obtained after the first step is hydrogenated in a subsequent second step and then a galactosyl isomalt composition (B) is obtained.

10. A method as in one of the preceding claims, where in a subsequent step galactosyl-1,1-GPM, galactosyl-1,6-GPS is separated from the resulting galactosyl isomalt composition (A,B).

11. A method as in one of the preceding claims, where the hydrogenation takes place as a catalytic hydrogenation and at elevated temperature and/or elevated pressure as well as in the presence of hydrogen and using a hydrogenation catalyst.

12. A method as in one of the preceding claims, where an aqueous solution containing the galactosyl isomalt composition is adjusted to a pH of 6-8 before the hydrogenation.

13. A method as in one of the preceding claims, where the hydrogenation takes place at an elevated temperature of 40-140°C, preferably 60-80°C.

14. A method as in one of the preceding claims, where the hydrogenation takes place at an elevated pressure of 50-230 bar, preferably 100-200 bar.

15. A method as in one of the preceding claims, where the hydrogenation catalyst consists of a mixture of a pure Raney metal and a Raney metal alloy.

16. A method as in one of the preceding claims, where in the second step the hydrogenation takes place over a period of time of 2-5 h.

17. A method as in one of the preceding claims, where the hydrogenation takes place continuously, semicontinuously or discontinuously.

18. A method as in one of the preceding claims, where the hydrogenation is carried out in a fixed bed or suspension process.

19. A galactosyl isomalt composition (A,B) prepared by a method as in one of the preceding claims.

20. β-1,3-Galactosyl isomalt of the formula: 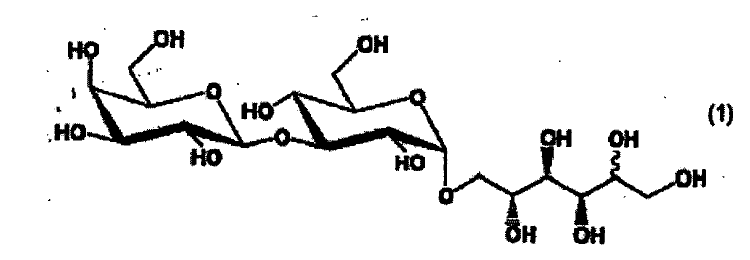 or its sorbitol diastereomer or its mannitol diastereomer.

21. β-1,4-Galactosyl isomalt of the formula: 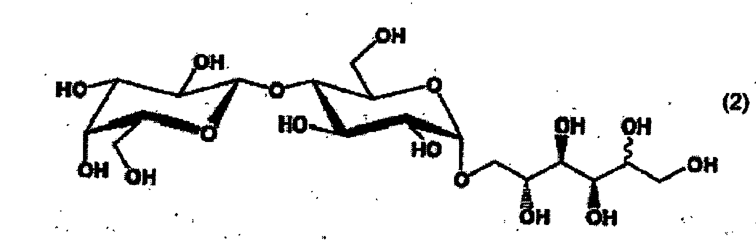 or its sorbitol diastereomer or its mannitol diastereomer.

22. β-1,6-Galactosyl isomalt of the formula: 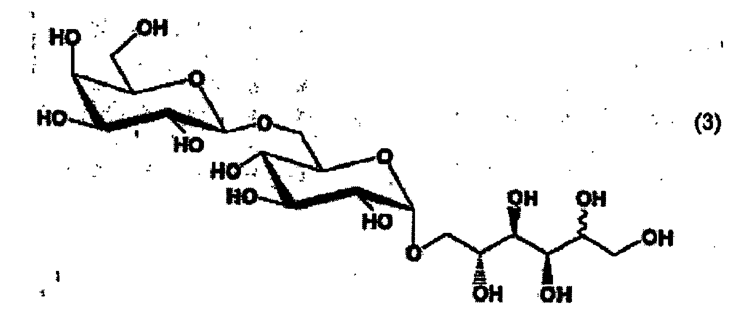 or its sorbitol diastereomer or its mannitol diastereomer.

23. A galactosyl isomalt composition containing
a) 4-30% by weight β-1,6-galactosyl isomalt, containing 1,6 galactosyl-1,1-GPM and 1,6-galcatosyl-1,6-GPS,
b) 6-60% by weight β-1,4-galactosyl isomalt, containing 1,4-galactosyl-1,1-GPM and 1,4-galactosyl-1,6-GPS and
c) 15-90% by weight β-1,3-galactosyl isomalt, containing 1,3-galactosyl-1,1-GPM and 1,3-galactosyl-1,6-GPS.

24. A galactosyl isomalt composition as in one of the preceding claims, additionally containing at least one monosaccharide (DP 1), at least one disaccharide (DP 2), at least one tetrasaccharide (DP 4) and/or at least one alcohol thereof.

25. A galactosyl isomalt composition as in the preceding claim, where the mixture contains 2-30% by weight monosaccharides (DP 1), 60-90% by weight disaccharides (DP 2), 1 45% by weight, preferably 2-30% by weight, galactosyl isomalt of formulas (1), (2) and (3) (DP 3) and up to 5% by weight tetrasaccharides (DP 4).

26. A galactosyl composition (A) as in one of the preceding claims containing galactose, glucose, lactose, isomalt and/or galactosyl lactose.

27. Galactosyl isomalt composition (B) as in one of the preceding claims containing galactitol, sorbitol, lactitol, isomalt and/or galactosyl lactitol.

28. β-1,3-Galactosyl isomaltulose of the formula:

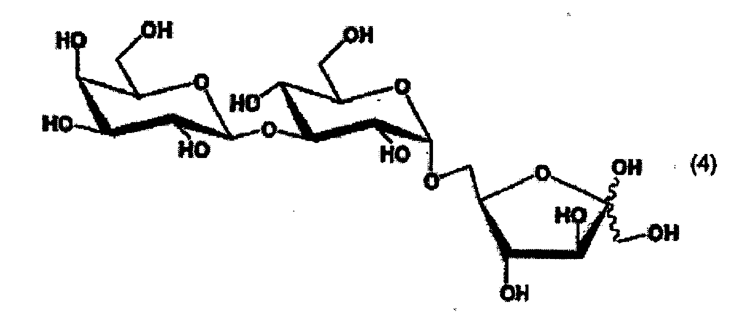

29. β-1,4-Galactosyl isomaltulose of the formula:

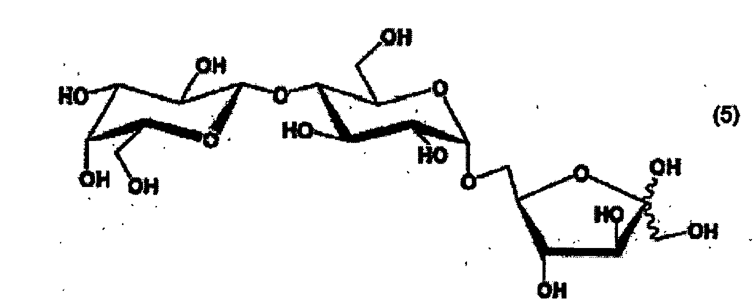

30. β-1,6-Galactosyl isomaltulose of the formula:

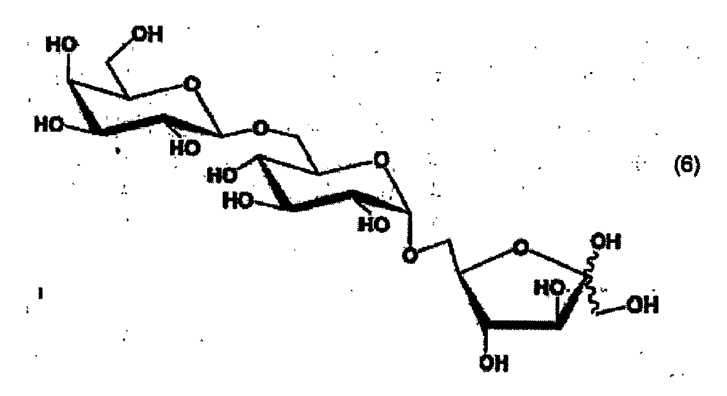

31. A galactosyl isomaltulose composition containing
a) 4-30% by weight β-1,6-galactosyl isomaltulose,
b) 6-60% by weight β-1,4-galactosyl isomaltulose, and
c) 15-90% by weight β-1,3-galactosyl isomaltulose.

32. A galactosyl isomaltulose composition as in the preceding claim, additionally containing at least one monosaccharide (DP 1), at least one disaccharide (DP 2), at least one tetrasaccharide (DP 4) and/or at least one alcohol thereof.

33. A galactosyl isomaltulose composition as in the preceding claim, where the mixture contains 2-30% by weight monosaccharides (DP 1), 60-90% by weight disaccharides (DP 2), 1 45% by weight, preferably 2-30% by weight, galactosyl isomaltulose of formulas (4), (5) and (6) (DP 3) and up to 5% by weight tetrasaccharides (DP 4).

34. A galactosyl isomaltulose composition as in one of the preceding claims containing galactose, glucose, lactose, isomaltulose and/or galactosyl lactose.

35. A synbiotic containing a galactosyl isomalt composition and/or a galactosyl isomaltulose composition as in one of the preceding claims and at least one probiotic, in particular bifidobacteria.

36. A roughage composition containing a galactosyl isomalt composition and/or a galactosyl isomaltulose composition as in one of the preceding claims, and at least one other type of roughage chosen from the group consisting of short-chain fructooligosaccharides, long-chain fructooligosaccharides, galactooligosaccharides, hydrolyzed guar gum, lactulose, xylooligosaccharides, lactosucrose, maltooligosaccharides, isomaltooligosaccharides, gentiooligoscaccharides, pectin, hydrolases, condensation products of sugars, glucosyl sucrose, such as "Coupling Sugar" from Hayashibara, glucosyl sucrose, soybean oligosaccharides, chitooligosaccharides, chitosan oligosaccharides, resistant starch, oat fiber, wheat fiber, vegetable fiber, fruit fiber, celluloses and beet fiber.

37. Foodstuffs, foods, semi-luxury foods or animal feeds containing a synbiotic, a roughage composition, a galactosyl isomalt composition and/or a galactosyl isomaltulose composition as in one of the preceding claims.

38. Foods as in the preceding claim, where these are milk items and milk products, in particular cheese, butter, yogurt, kefir, fresh cheese, sour milk, buttermilk, cream, condensed milk, dry milk, whey, milk sugar, milk protein, mixed milk, low fat milk, mixed whey or milk fat products or preparations; baked goods, in particular bread, including cookies and fine baked goods including durable baked goods, crisp cake products and waffles; sandwich spreads; margarine products and cooking oils; instant products and concentrated broth products; fruit products or preparations, especially jams, marmalades, jellies, fruit preserves, fruit pulp, fruit paste, fruit juices, fruit juice concentrates, fruit nectar and fruit powder; vegetable products or their preparations, especially vegetable preserves, vegetable juices and vegetable paste; flavoring mixtures, muesli and muesli mixtures as well as products containing prepared muesli, or nonalcoholic beverages, beverage bases and beverage powders.

39. Foods as in one of the preceding claims, where these are low calorie foods.

40. Sweets containing a synbiotic, a roughage composition, a galactosyl isomalt composition and/or a galactosyl isomaltulose composition as in one of the preceding claims.

41. Sweets as in the preceding claim, where these are low calorie sweets.

42. Foods or sweets as in one of the preceding claims, where these are chocolates, hard caramels, soft caramels, fondant products, jelly products, licorices, marshmallow cream products, coconut flakes, dragees, compressed goods, candied fruits, crocant, nougat products, ice chocolate, marzipan, chewing gum, cereal bars as well as ice cream or alcoholic or nonalcoholic sweetened beverages.

43. Dietetic special foodstuffs, in particular for the diets of persons with glucose intolerance, insulin resistance, syndrome X, containing a synbiotic, a roughage composition, a galactosyl isomalt composition and/or a galactosyl isomaltulose composition as in one of the preceding claims.

44. Children's foodstuffs containing a synbiotic, a roughage composition, a galactosyl isomalt composition and/or a galactosyl isomaltulose composition as in one of the preceding claims.

45. A sweetener containing a synbiotic, a roughage composition, a galactosyl isomalt composition and/or a galactosyl isomaltulose composition as in one of the preceding claims.

46. A pharmaceutical composition containing a synbiotic, a roughage composition, a galactosyl isomalt composition and/or a galactosyl isomaltulose composition as in one of the preceding claims.

47. A pharmaceutical composition as in the preceding claim as active agents.

48. A pharmaceutical composition as in one of the preceding claims as pharmaceutical vehicle.

49. The use of the galactosyl isomalt composition and/or the galactosyl isomaltulose composition as in one of the preceding claims as dietetic sources of fiber and/or as nutrients for healthy microflora.

50. The use of the galactosyl isomalt composition and/or the galactosyl isomaltulose composition as in one of the preceding claims as an agent resistant to digestion in the stomachs of mammals and/or to the enzymes of the digestive tracts of mammals.

51. The use of the galactosyl isomalt composition and/or the galactosyl isomaltulose composition as in one of the preceding claims as additives in foods, animal feeds or beverages, especially as soluble roughage, preferably as prebiotic roughage.

52. The use of the galactosyl isomalt composition and/or the galactosyl isomaltulose composition as in one of the preceding claims for modulation of glycemic properties of foods or sweets, especially special foods, children's foods or foods for persons with disorders of the glucose/insulin balance.

53. The use of the galactosyl isomalt composition and/or the galactosyl isomaltulose composition as in one of the preceding claims as sweeteners.

54. The use of the galactosyl isomalt composition and/or the galactosyl isomaltulose composition as in one of the preceding claims for preparation of foodstuffs, foods, semi-luxury foods or animal feeds.

55. The use of the galactosyl isomalt composition and/or the galactosyl isomaltulose composition as in one of the preceding claims for prophylaxis and/or treatment of disorders of glucose metabolism, diabetes I and II, glucose intolerance, insulin resistance and/or syndrome X.

56. The use of the galactosyl isomalt composition and/or the galactosyl isomaltulose composition as in one of the preceding claims for preparation of a drug for prophylaxis and/or treatment of disorders of glucose metabolism, diabetes I and II, glucose intolerance, insulin resistance and/or syndrome X.

57. The use of the galactosyl isomalt composition and/or the galactosyl isomaltulose composition as in one of the preceding claims to improve the absorption of nutrient components in the animal or human digestive tract.

58. The use of the galactosyl isomalt composition and/or the galactosyl isomaltulose composition as in one of the preceding claims for preparation of a drug for improving the absorption of nutrient components in the animal or human digestive tract.

59. The use of the galactosyl isomalt composition and/or the galactosyl isomaltulose composition as in one of the preceding claims as active agents for prophylaxis and/or treatment of diarrheal diseases, especially ones caused by infection by microorganisms; inflammatory intestinal diseases; infectious diseases; of colon carcinogenesis; osteoporosis; diseases that can be affected by indigestible carbohydrates; cancer diseases; constipation; hypertension; stroke; diseases caused by pathogenic microbes; rheumatic diseases, coronary artery diseases; acute cardiac infarct; cardiovascular diseases; chronic inflammatory diseases; restoration and maintenance of healthy microflora in the digestive tract and/or healthy intestinal epithelium and/or to enhance the immune response to general infections.

60. The use of the galactosyl isomalt composition and/or the galactosyl isomaltulose composition as in one of the preceding claims for preparation of a drug for prophylaxis and/or treatment of diarrheal diseases, especially ones caused by infection by microorganisms; inflammatory intestinal diseases; infectious diseases; colon carcinogenesis; osteoporosis; diseases that can be affected by indigestible carbohydrates; cancer diseases; constipation; hypertension; stroke; diseases caused by pathogenic microbes; rheumatic diseases, coronary artery diseases; acute cardiac infarct; cardiovascular diseases; chronic inflammatory diseases; restoration and maintenance of healthy microflora in the digestive tract and/or healthy intestinal epithelium and/or to enhance the immune response to general infections.

61. A use as in one of the preceding claims, where the galactosyl isomalt composition and/or the galactosyl isomaltulose composition as in one of the preceding claims is administered in a dosage that is sufficient to cure or prevent the condition of a disease or infection caused by oxidative stress, to stop the progression of the disease, and/or to relieve the symptoms of the disease.

62. A use as in one of the preceding claims, where the galactosyl isomalt composition and/or the galactosyl isomaltulose composition as in one of the preceding claims is administered as a pharmaceutical composition, in particular as a suspension, syrup, tablet, pill, capsule, granulate or powder.

## Revendications

1. Procédé de production d'une composition contenant de l'isomaltulose hydrogéné galactosylé ou de l'isomaltulose non hydrogéné galactosylé, dans lequel une solution aqueuse d'au moins un donneur de galactosyle est mise en contact avec au moins une β-galactosidase et une solution aqueuse d'un mélange contenant de l'isomaltulose hydrogéné ou de l'isomaltulose non hydrogéné comme accepteur de galactosyle, et une composition galactosyl-isomalt (A) ou une composition galactosyl-isomaltulose est obtenue.

2. Procédé selon la revendication 1, dans lequel ledit donneur de galactosyle est du lactose.

3. Procédé selon la revendication 1 ou la revendication 2, dans lequel une composition galactosyl-isomalt (A) est obtenue, laquelle contient du galactosyl-1,6-GPS, du galactosyl-1,1-GPM, du galactosyl-lactose ainsi que du glucose, du galactose, du lactose et/ou de l'isomalt.

4. Procédé selon l'une des revendications 1 à 3, dans lequel une composition galactosyl-isomaltulose est obtenue, laquelle contient du galactosyl-isomaltulose, du galactosyl-lactose ainsi que du glucose, du galactose, du lactose et/ou de l'isomaltulose.

5. Procédé selon l'une des revendications précédentes, dans lequel ladite β-galactosidase est dérivée de *Bacillus circulans, d'Aspergillus oryzae,* de testicule de taureau, de *Bacillus stearothermophilus* KVE39, *Thermus brockianus* ITI360 ou *Thermus thermophilus* TH125.

6. Procédé selon l'une des revendications précédentes, dans lequel le rapport de poids dudit, au moins un, donneur de galactosyle par rapport audit mélange contenant de l'isomaltulose hydrogéné ou de l'isomaltulose non hydrogéné est de 1:1 à 1:5.

7. Procédé selon l'une des revendications précédentes, dans lequel la première étape s'effectue sur un temps de réaction allant de 1 à 48 heures.

8. Procédé selon l'une des revendications précédentes, dans lequel ladite première étape s'effectue à une température allant de 30 °C à 55 °C.

9. Procédé selon l'une des revendications précédentes, dans lequel la composition galactosyl-isomaltulose obtenue après ladite première étape est hydrogénée au cours d'une deuxième étape ultérieure et une composition galactosyl-isomalt (B) est ensuite obtenue.

10. Procédé selon l'une des revendications précédentes, dans lequel du galactosyl-1,1-GPM, galactosyl-1,6-GPS est séparé de ladite composition galactosyl-isomalt (A, B) obtenue au cours d'une étape subséquente.

11. Procédé selon l'une des revendications précédentes, dans lequel l'hydrogénation se produit sous forme d'hydrogénation catalytique et à température accrue et/ou pression accrue, ainsi qu'en présence d'hydrogène et avec l'utilisation d'un catalyseur d'hydrogénation.

12. Procédé selon l'une des revendications précédentes, dans lequel une solution aqueuse contenant ladite composition galactosyl-isomaltulose est ajustée à un pH de 6 à 8 avant l'hydrogénation.

13. Procédé selon l'une des revendications précédentes, dans lequel ladite hydrogénation se produit à une température accrue allant de 40 à 140°C, de préférence 60 à 80 °C.

14. Procédé selon l'une des revendications précédentes, dans lequel ladite hydrogénation se produit à une pression accrue allant de 50 à 230 bars, de préférence 100 à 200 bars.

15. Procédé selon l'une des revendications précédentes, dans lequel ledit catalyseur d'hydrogénation comprend un mélange composé d'un métal de Raney pur et d'un alliage métallique de Raney.

16. Procédé selon l'une des revendications précédentes, dans lequel ladite hydrogénation se produit sur une période de 2 à 5 heures au cours de la deuxième étape.

17. Procédé selon l'une des revendications précédentes, dans lequel ladite hydrogénation se produit de façon continue, semi-continue ou discontinue.

18. Procédé selon l'une des revendications précédentes, dans lequel ladite hydrogénation s'effectue au cours du procédé à lit fixe ou de suspension.

19. Composition galactosyl-isomalt (A, B), laquelle est produite à partir d'un procédé suivant l'une des revendications précédentes.

20. β-1,3-galactosyl-isomalt de formule : 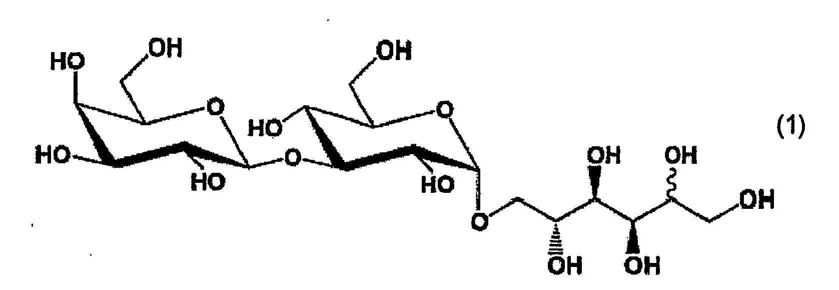 ou son diastéréomère de sorbitol ou son diastéréomère de mannitol.

21. β-1,4-galactosyl-isomalt de formule : 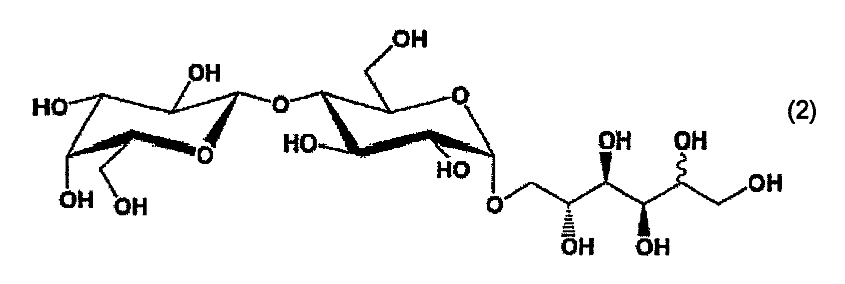 ou son diastéréomère de sorbitol ou son diastéréomère de mannitol.

22. β-1,6-galactosyl-isomalt de formule : 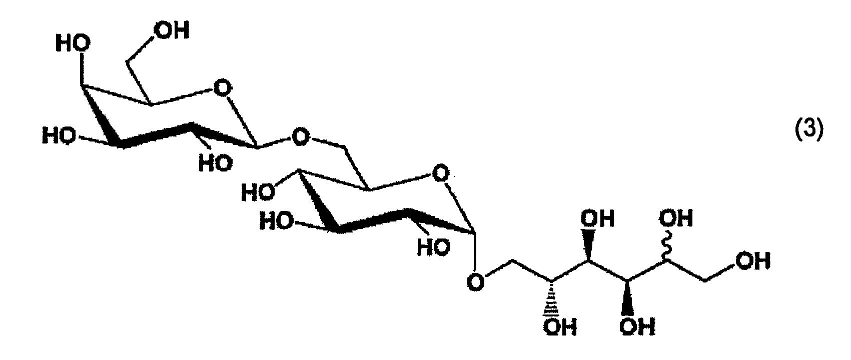 ou son diastéréomère de sorbitol ou son diastéréomère de mannitol.

23. Composition galactosyl-isomalt contenant
a) 4 à 30 % en poids de β-1,6-galactosyl-isomalt, contenant du 1,6-galactosyl-1,1-GPM et 1,6-galactosyl-1,6-GPS,
b) 6 à 60 % en poids de β-1,4-galactosyl-isomalt, contenant du 1,4-galactosyl-1,1-GPM et 1,4-galactosyl-1,6-GPS et
c) 15 à 90 % en poids de β-1,3-galactosyl-isomalt, contenant du 1,3-galactosyl-1,1-GPM et 1,3-galactosyl-1,6-GPS.

24. Composition galactosyl-isomalt selon l'une des revendications précédentes, contenant de plus au moins un monosaccharide (DP 1), au moins un disaccharide (DP 2), au moins un tétrasaccharide (DP 4) et/ou au moins un alcool de ceux-ci.

25. Composition galactosyl-isomalt selon la revendication précédente, dans laquelle le mélange présente 2 à 30 % en poids de monosaccharide (DP 1), 60 à 90 % en poids de disaccharide (DP 2), 1 à 45 % en poids, de préférence 2 à 30 % en poids, de galactosyl-isomalt de formules (1), (2) et (3) (DP 3) et jusqu'à 5 % en poids de tétrasaccharide (DP 4).

26. Composition galactosyl-isomalt (A) selon l'une des revendications précédentes, contenant du galactose, du glucose, du lactose, de l'isomalt et/ou du galactosyl-lactose.

27. Composition galactosyl-isomalt (B) selon l'une des revendications précédentes, contenant de la galactite, du sorbitol, de la lactite, de l'isomalt et/ou du galactosyl-lactite.

28. β-1,3-galactosyl-isomaltulose de formule :

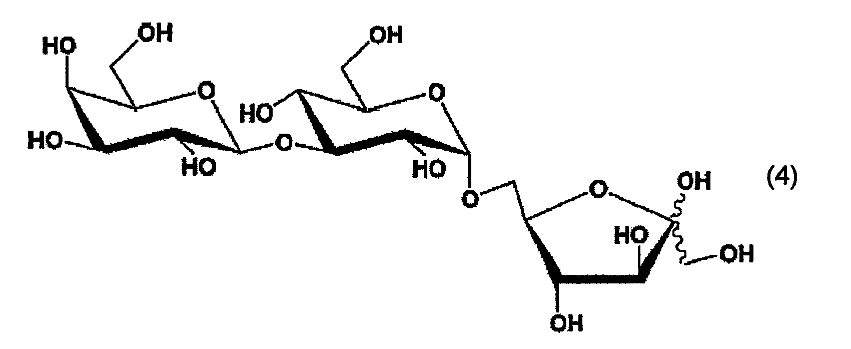

29. β-1,4-galactosyl-isomaltulose de formule :

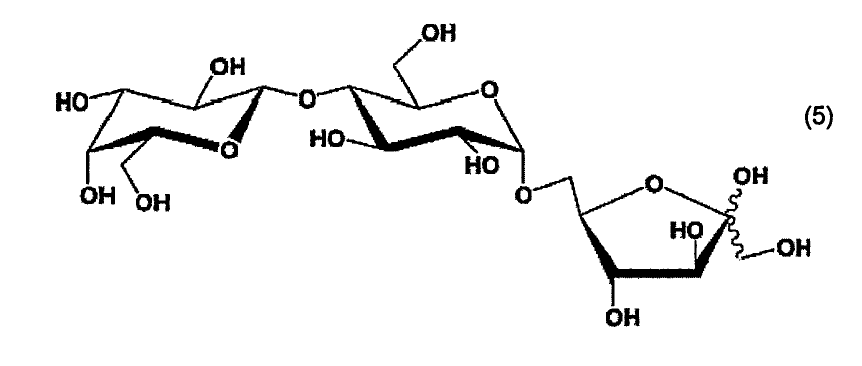

30. β-1,6-galactosyl-isomaltulose de formule :

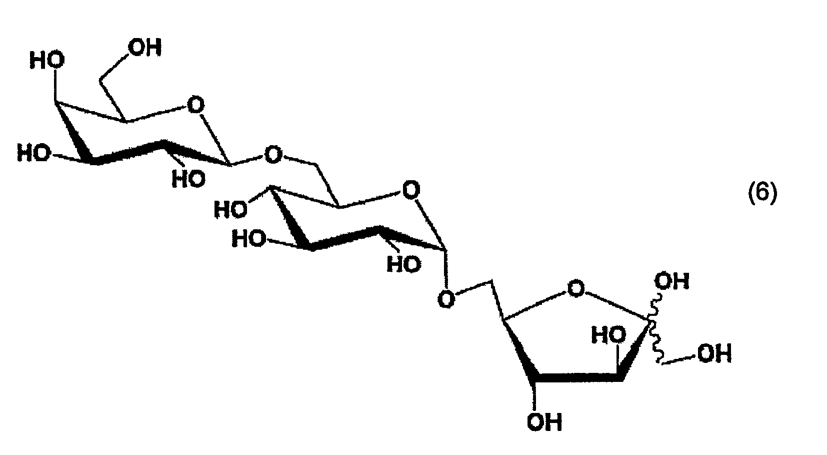

31. Composition galactosyl-isomaltulose contenant
a) 4 à 30 % en poids de β-1,6-galactosyl-isomaltulose,
b) 6 à 60 % en poids de β-1,4-galactosyl-isomaltulose, et
c) 15 à 90 % en poids de β-1,3-galactosyl-isomaltulose.

32. Composition galactosyl-isomaltulose selon la revendication précédente, contenant de plus au moins un monosaccharide (DP 1), au moins un disaccharide (DP 2), au moins un tétrasaccharide (DP 4) et/ou au moins un alcool de ceux-ci.

33. Composition galactosyl-isomaltulose selon la revendication précédente, dans laquelle le mélange présente 2 à 30 % en poids de monosaccharide (DP 1), 60 à 90 % en poids de disaccharide (DP 2), 1 à 45 % en poids, de préférence 2 à 30 % en poids, de galactosyl-isomaltulose de formules (4), (5) et (6) (DP 3) et jusqu'à 5 % en poids de tétrasaccharide (DP 4).

34. Composition galactosyl-isomaltulose selon l'une des revendications précédentes, contenant du galactose, du glucose, du lactose, de l'isomaltulose et/ou du galactosyl-lactose.

35. Synbiotique contenant une composition galactosyl-isomalt et/ou une composition galactosyl-isomaltulose selon l'une des revendications précédentes et au moins un probiotique, en particulier des bifidobactéries.

36. Composition d'aliments de lest contenant une composition galactosyl-isomalt et/ou une composition galactosyl-isomaltulose selon l'une des revendications précédentes et au moins un aliment de lest supplémentaire, sélectionné dans le groupe d'aliments de lest composé de fructooligosaccharides à chaîne courte, fructooligosaccharides à longue chaîne, galactooligosaccharides, gomme de guar hydrolysée, lactulose, xylooligosaccharides, lactosucrose, maltooligosaccharides, isomaltooligosaccharides, gentiooligosaccharides, substances pectiques, hydrolases, produits de condensation de sucres, glucosyl-sucrose, comme le « coupling sugar » de Hayashibara, glucosyl-sucrose, oligosaccharides de soja, chitooligosaccharides, oligosaccharides de chitosane, amidon résistant, fibre d'avoine, fibre de blé, fibre végétale, fibre de fruit, celluloses et fibre de betterave.

37. Aliment, produit alimentaire, denrée de luxe ou nourriture pour animaux, contenant un synbiotique, une composition d'aliments de lest, une composition galactosyl-isomalt et/ou une composition galactosyl-isomaltulose selon l'une des revendications précédentes.

38. Produits alimentaires selon la revendication précédente, lesquels consistent en produits du lait et produits laitiers, en particulier des produits ou des préparations à base de fromage, beurre, yaourt, kéfir, fromage blanc, lait caillé, babeurre, crème, lait condensé, lait en poudre, lactosérum (petit-lait), lactose, lactalbumine, mélange de lait, lait demi-écrémé, mélange de petit-lait ou matière grasse du lait ; produits boulangers et pâtissiers, en particulier le pain, y compris viennoiseries, gâteaux et pâtisseries fines dont produits boulangers et pâtissiers longue conservation, produits à base de biscuits et gaufres ; pâtes à tartiner ; produits type margarine et matières grasses ; produits instantanés et bouillons ; produits ou préparations à base de fruits, en particulier des confitures, marmelades, gelées, conserves de fruits, pulpes de fruits, purées de fruits, jus de fruit, jus de fruit concentré, nectar de fruit et poudre de fruits ; produits ou préparations à base de légumes, en particulier des conserves de légumes, jus de légumes et purées de légumes ; mélanges d'épices ; muesli et mélanges à base de muesli ainsi que des produits contenant du muesli « prêts à manger » ou des boissons non alcoolisées, composants de boissons et préparations pour boissons en poudre.

39. Produit alimentaire selon l'une des revendications précédentes, lequel consiste en un produit alimentaire à teneur réduite en calories.

40. Confiseries, contenant un synbiotique, une composition d'aliments de lest, une composition galactosyl-isomalt et/ou une composition galactosyl-isomaltulose selon l'une des revendications précédentes.

41. Confiseries selon la revendication précédente, lesquelles consistent en des confiseries à teneur réduite en calories.

42. Produits alimentaires ou confiseries selon l'une des revendications précédentes, lesquels consistent en chocolats, caramels durs, caramels mous, confections à base de fondant, confections à base de gelée, réglisse, guimauve, flocons de noix de coco, dragées, comprimés, fruits confits, nougatine, produits à base de nougat, bonbons chocolats glacés, pâte d'amandes, chewing-gum, barres au muesli et glace ou boissons sucrées alcoolisées et non alcoolisées.

43. Aliment diététique spécial, particulièrement destiné à l'alimentation des personnes souffrant d'une intolérance au glucose, d'une insulinorésistance et du syndrome X, contenant un synbiotique, une composition d'aliments de lest, une composition galactosyl-isomalt et/ou une composition galactosyl-isomaltulose selon l'une des revendications précédentes.

44. Aliment pour enfants, contenant un synbiotique, une composition d'aliments de lest, une composition galactosyl-isomalt et/ou une composition galactosyl-isomaltulose selon l'une des revendications précédentes.

45. Édulcorant, contenant un synbiotique, une composition d'aliments de lest, une composition galactosyl-isomalt et/ou une composition galactosyl-isomaltulose selon l'une des revendications précédentes.

46. Composition pharmaceutique, contenant un synbiotique, une composition d'aliments de lest, une composition galactosyl-isomalt et/ou une composition galactosyl-isomaltulose selon l'une des revendications précédentes.

47. Composition pharmaceutique selon la revendication précédente, laquelle est sous forme de substance active.

48. Composition pharmaceutique selon l'une des revendications précédentes, laquelle est sous forme de support pharmaceutique.

49. Utilisation de ladite composition galactosyl-isomalt et/ou de ladite composition galactosyl-isomaltulose selon l'une des revendications précédentes comme source diététique de fibres et/ou comme nutriment pour une microflore saine.

50. Utilisation de ladite composition galactosyl-isomalt et/ou de ladite composition galactosyl-isomaltulose selon l'une des revendications précédentes comme moyen de résistance contre une dégradation dans l'estomac des mammifères et/ou contre les enzymes du tube digestif des mammifères.

51. Utilisation de ladite composition galactosyl-isomalt et/ou de ladite composition galactosyl-isomaltulose selon l'une des revendications précédentes comme complément dans des produits alimentaires, de la nourriture pour animaux ou des boissons, en particulier comme aliment de lest soluble, de préférence comme aliment de lest probiotique.

52. Utilisation de ladite composition galactosyl-isomalt et/ou de ladite composition galactosyl-isomaltulose selon l'une des revendications précédentes pour la modulation des propriétés glycémiques de produits alimentaires ou de confiseries, en particulier liés aux régimes alimentaires spéciaux, à l'alimentation des enfants ou à l'alimentation des personnes souffrant de dysfonctionnements au niveau du site de contrôle du glucose/de l'insuline.

53. Utilisation de ladite composition galactosyl-isomalt et/ou de ladite composition galactosyl-isomaltulose selon l'une des revendications précédentes comme édulcorant.

54. Utilisation de ladite composition galactosyl-isomalt et/ou de ladite composition galactosyl-isomaltulose selon l'une des revendications précédentes pour la production d'aliments, de produits alimentaires, de denrées de luxe ou de nourriture pour animaux.

55. Utilisation de ladite composition galactosyl-isomalt et/ou de ladite composition galactosyl-isomaltulose selon l'une des revendications précédentes pour la prophylaxie et/ou le traitement de dysfonctionnements liés au métabolisme de glucose, du diabète de type et de type II, de l'intolérance au glucose, de l'insulinorésistance et/ou du syndrome X.

56. Utilisation de ladite composition galactosyl-isomalt et/ou de ladite composition galactosyl-isomaltulose selon l'une des revendications précédentes pour la production d'un médicament destiné à la prophylaxie et/ou au traitement de dysfonctionnements liés au métabolisme de glucose, du diabète de type I et de type II, de l'intolérance au glucose, de l'insulinorésistance et/ou du syndrome X.

57. Utilisation de ladite composition galactosyl-isomalt et/ou de ladite composition galactosyl-isomaltulose selon l'une des revendications précédentes pour l'amélioration de la résorption des composants alimentaires dans le tube digestif de l'homme ou de l'animal.

58. Utilisation de ladite composition galactosyl-isomalt et/ou de ladite composition galactosyl-isomaltulose selon l'une des revendications précédentes pour la production d'un médicament destiné à l'amélioration de la résorption des composants alimentaires dans le tube digestif de l'homme ou de l'animal.

59. Utilisation de ladite composition galactosyl-isomalt et/ou de ladite composition galactosyl-isomaltulose selon l'une des revendications précédentes comme substance active pour la prophylaxie et/ou le traitement de maladies diarrhéiques, en particulier celles déclenchées par une infection due à des micro-organismes ; de maladies intestinales inflammatoires ; de maladies infectieuses ; de la carcinogenèse du colon ; de l'ostéoporose ; de maladies pouvant être conditionnées par des glucides indigestibles ; de maladies cancéreuses ; de la constipation ; de l'hypertension ; de l'apoplexie ; de maladies provoquées par des d'un infarctus du myocarde aigu ; de maladies liées à la circulation cardiaque ; de maladies inflammatoires chroniques ; pour la restauration et le maintien d'une flore microbienne saine dans le tube digestif et/ou d'un épithélium intestinal sain et/ou pour le renforcement des défenses immunitaires contre les infections communes.

60. Utilisation de ladite composition galactosyl-isomalt et/ou de ladite composition galactosyl-isomaltulose selon l'une des revendications précédentes pour la production d'un médicament destiné à la prophylaxie et/ou au traitement de maladies diarrhéiques, en particulier celles déclenchées par une infection due à des micro-organismes ; de maladies intestinales inflammatoires ; de maladies infectieuses ; de la carcinogenèse du colon ; de l'ostéoporose ; de maladies pouvant être conditionnées par des glucides indigestibles ; de maladies cancéreuses ; de la constipation ; de l'hypertension ; de l'apoplexie ; de maladies provoquées par des germes pathogènes ; de maladies rhumatismales ; de maladies coronariennes ; d'un infarctus du myocarde aigu ; de maladies liées à la circulation cardiaque ; de maladies inflammatoires chroniques ; pour la restauration et le maintien d'une flore microbienne saine dans le tube digestif et/ou d'un épithélium intestinal sain et/ou pour le renforcement des défenses immunitaires contre les infections communes.

61. Utilisation selon l'une des revendications précédentes, dans laquelle ladite composition galactosyl-isomalt et/ou ladite composition galactosyl-isomaltulose selon l'une des revendications précédentes est administrée en dose suffisante pour guérir une maladie provoquée par un état de stress oxydatif ou une infection ou la prévenir, pour arrêter la progression de la maladie et/ou soulager les symptômes de la maladie.

62. Utilisation selon l'une des revendications précédentes, dans laquelle ladite composition galactosyl-isomalt et/ou ladite composition galactosyl-isomaltulose selon l'une des revendications précédentes est administrée sous forme de composition pharmaceutique, en particulier sous forme de suspension, sirop, comprimé, pilule, gélule, granulé ou poudre.
